# FASCICULE DE BREVET EUROPEEN

(11) **EP 0 874 049 B1**
(45) Date de publication et mention de la délivrance du brevet: **17.10.2007**
(21) Numéro de dépôt: 98400968.8
(22) Date de dépôt: 21.04.1998
(51) Int. Cl.: C07K 14/47, C12N 15/85, C12N 15/86, A61K 38/18, C12N 5/10, C07K 16/18, C12N 15/11, C12Q 1/68

(54) **Séquences d'acide nucléique de genes CIITA**
Nukleinsäuren von CIITA Genen
Nucleic acid sequences of CIITA genes

(30) Priorité: 22.04.1997 FR 9704954
(43) Date de publication de la demande: 28.10.1998
(73) Titulaire: Novimmune SA, 1211 Genève (CH)
(72) Inventeur: Mach, Bernard, 1292 Chambesy, Geneve (CH)
(74) Mandataire: Gutmann, Ernest

(56) Documents cités:
- EP-A- 0 648 836
- STEIMLE V ET AL: "Complementation cloning of an MHC class II transactivator mutated in hereditary MHC class II deficiency (or bare lymphocyte syndrome)." CELL, (1993 OCT 8) 75 (1) 135-46., XP002051559
- RILEY J L ET AL: "Activation of class II MHC genes requires both the X box region and the class II transactivator (CIITA)." IMMUNITY, (1995 MAY) 2 (5) 533-43., XP002051560
- MUHLETHALER-MOTTET A ET AL: "Expression of MHC class II molecules in different cellular and functional compartments is controlled by differential usage of multiple promoters of the transactivator CIITA." EMBO JOURNAL, (1997 MAY 15) 16 (10) 2851-60., XP002051561
- LENNON A M ET AL: "Isolation of a B-cell-specific promoter for the human class II transactivator." IMMUNOGENETICS, (1997) 45 (4) 266-73., XP002051562
- STEIMLE V ET AL: "Regulation of MHC class II expression by interferon-gamma mediated by the transactivator gene CIITA." SCIENCE, (1994 JUL 1) 265 (5168) 106-9., XP002051563
- REITH, W. ET AL.: "Molecular defects in the bare lymphocyte syndrome and regulation of MHC class II genes" IMMUNOLOGY TODAY., vol. 16, 1995, pages 539-45, XP002051564 CAMBRIDGE GB
- STEIMLE V ET AL: "Major histocompatibility complex class II deficiency: a disease of gene regulation." ADVANCES IN IMMUNOLOGY, (1996) 61 327-40., XP002051565
- PISKURICH, J. & AL.: "Identification of distinct regions of 5' flanking DNA that mediate constitutive, IFN-gamma, and TGF-beta-regulated expression of the class II transactivator gene" JOURNAL OF IMMUNOLOGY., vol. 160, janvier 1998, pages 233-240, XP002073465 BALTIMORE US

### LISTE DE SEQUENCES

(1) INFORMATIONS GENERALES:
   (i) DEPOSANT:
      (A) NOM: TRANSGENE SA
      (B) RUE: 11 RUE MOLSHEIM
      (C) VILLE: STRASBOURG
      (E) PAYS: FRANCE
      (F) CODE POSTAL: 67082
      (G) TELEPHONE: (33)388 27 91 00
      (H) TELECOPIE: (33)388 22 58 07
   (ii) TITRE DE L' INVENTION: SEQUENCES D'ACIDE NUCLEIQUE DE GENES CIITA, SUSCEPTIBLES D'ETRE IMPLIQUEES DANS LE CONTROLE ET LA REGULATION DE L'EXPRESSION DE GENES CODANT POUR DES MOLECULES MHC DE TYPE II ET LEUR UTILISATION, NOTAMMENT COMME MEDICAMENT
   (iii) NOMBRE DE SEQUENCES: 25
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: Patent In Release #1.0, Version #1.30 (OEB)
   (vi) DONNEES DE LA DEMANDE ANTERIEURE:
      (A) NUMERO DE LA DEMANDE: FR 9704954
      (B) DATE DE DEPOT: 22-APR-1997
(2) INFORMATIONS POUR LA SEQ ID NO: 1:
   (i) CARACTERISTIQUES DE LA SEQUENCE:
      (A) LONGUEUR: 5463 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: gène cIIta de type I
   (xi) DESCRIPTION DE LA SEQUENCE: SEQ ID NO: 1:
   GTAAGTACTG TAACAGAGAC TAAATGCTAA GTAAGGCAGG CGTGGTGGCT CACACTTGTA 60
   ATCCCAGTAC TTTGGAGGAC TGAGGCAAGA GGATCACTTG AGCCCAGAAT TCAAGACCAG 120
   CCTGGGAAAC AGAGTGAGAC CCTGTCTCTA CTAAAAATAA AAAAATAAAA AATTAGCGGG 180
   GCATGATGGT GTGAGTCTGT AGTCCCAGCT ACTGGGAAAC AGAGATGGGA GGTTTGTTTG 240
   AGCCTGGGAA GTTGAGGCTG CAGTGAGTTA TGATCACGCC ACTGCACACC AGCCTGGGCA 300
   ACAGAGCAAG ACCCTGTCAA AAAAAAAAAA AAGGCTATGT AACACCCCCA AGTCACATAA 360
   TTGGCAAGGA GCAGCAGATC TGGGACTTGA ACATAGGCAG ATTAGCTCCA AGCCTATTTG 420
   CTTAACCTCT ATACCACAAT GCCTTCTTGC TATGGTAAAA CATCTGAAAA GACCTATTAC 480
   CCTATAGGTC CTCTAAGGAG GCATGTCGCC TTCCTCTTAG CAATACTAGA TTGGCTCCAA 540
   CAGAAGGCTG TGGGCTTCTC TGGCACATGC ACCTGGGTAG GACCCAGAGA ATATCTGTGG 600
   AGTCTGAATC AACCCAAAAG CCAATATCCA TCCGTTCATC AGGAACCCCA GCCTACAACG 660
   CAAAAGAGGA AATCTTCCTA AGTAGAAATA AACTGTAATA AATTGCAGAG GTTCCCTCGT 720
   CCTGGTTTTC ACTTCATGTT TTGGATGCTG CATGCTGGGT GAGCGGAGAT TCCAGGCACT 780
   GGCCAGGGCA GCTGCCCTGA CTCCAAGGGC TGCCATGAAC AACTTCCAGG CCATCCTGAC 840
   TCAGGTGAGA ATGCTGCTCT CCAGCCATCA GCCCAGCCTG GTGCAGGCCC TCTTGGACAA 900
   CCTGCTGAAG GAGGACCTCC TCTCCAGGGA ATACCACTGC ACTCTGCTCC ATGAGCCTGA 960
   TAGTGAGGCT CTGGCCAGGA AGATCTCTTT GACCCTACTA GAGAAAGGAG ACCTGGATTT 1020
   GGCCCTCCTG GGGTGGGCCC GGAGTGGGCT GCAGCCCCCA GCAGCCGAGA GGGGCCCCGG 1080
   CCACAGTGAC CATGGTGGCA GCTCACAGTG TGCCACCATG GAGTTGGGGC CCCTAGAAGG 1140
   TGGCTACCTG GAGCTTCTTA ACAGCGATGC TGACCCCCTG TGCCTCTACC ACTTCTATGA 1200
   CCAGATGGAC CTGGCTGGAG AAGAAGAGAT TGAGCTCTAC TCAGAACCCG ACACAGACAC 1260
   CATCAACTGC GACCAGTTCA GCAGGCTGTT GTGTGACATG GAAGGTGATG AAGAGACCAG 1320
   GGAGGCTTAT GCCAATATCG CGGAACTGGA CCAGTATGTC TTCCAGGACT CCCAGCTGGA 1380
   GGGCCTGAGC AAGGACATTT TCAAGCACAT AGGACCAGAT GAAGTGATCG GTGAGAGTAT 1440
   GGAGATGCCA GCAGAAGTTG GGCAGAAAAG TCAGAAAAGA CCCTTCCCAG AGGAGCTTCC 1500
   GGCAGACCTG AAGCACTGGA AGCCAGCTGA GCCCCCCACT GTGGTGACTG GCAGTCTCCT 1560
   AGTGGGACCA GTGAGCGACT GCTCCACCCT GCCCTGCCTG CCACTGCCTG CGCTGTTCAA 1620
   CCAGGAGCCA GCCTCCGGCC AGATGCGCCT GGAGAAAACC GACCAGATTC CCATGCCTTT 1680
   CTCCAGTTCC TCGTTGAGCT GCCTGAATCT CCCTGAGGGA CCCATCCAGT TTGTCCCCAC 1740
   CATCTCCACT CTGCCCCATG GGCTCTGGCA AATCTCTGAG GCTGGAACAG GGGTCTCCAG 1800
   TATATTCATC TACCATGGTG AGGTGCCCCA GGCCAGCCAA GTACCCCCTC CCAGTGGATT 1860
   CACTGTCCAC GGCCTCCCAA CATCTCCAGA CCGGCCAGGC TCCACCAGCC CCTTCGCTCC 1920
   ATCAGCCACT GACCTGCCCA GCATGCCTGA ACCTGCCCTG ACCTCCCGAG CAAACATGAC 1980
   AGAGCACAAG ACGTCCCCCA CCCAATGCCC GGCAGCTGGA GAGGTCTCCA ACAAGCTTCC 2040
   AAAATGGCCT GAGCCGGTGG AGCAGTTCTA CCGCTCACTG CAGGACACGT ATGGTGCCGA 2100
   GCCCGCAGGC CCGGATGGCA TCCTAGTGGA GGTGGATCTG GTGCAGGCCA GGCTGGAGAG 2160
   GAGCAGCAGC AAGAGCCTGG AGCGGGAACT GGCCACCCCG GACTGGGCAG AACGGCAGCT 2220
   GGCCCAAGGA GGCCTGGCTG AGGTGCTGTT GGCTGCCAAG GAGCACCGGC GGCCGCGTGA 2280
   GACACGAGTG ATTGCTGTGC TGGGCAAAGC TGGTCAGGGC AAGAGCTATT GGGCTGGGGC 2340
   AGTGAGCCGG GCCTGGGCTT GTGGCCGGCT TCCCCAGTAC GACTTTGTCT TCTCTGTCCC 2400
   CTGCCATTGC TTGAACCGTC CGGGGGATGC CTATGGCCTG CAGGATCTGC TCTTCTCCCT 2460
   GGGCCCACAG CCACTCGTGG CGGCCGATGA GGTTTTCAGC CACATCTTGA AGAGACCTGA 2520
   CCGCGTTCTG CTCATCCTAG ACGCCTTCGA GGAGCTGGAA GCGCAAGATG GCTTCCTGCA 2580
   CAGCACGTGC GGACCGGCAC CGGCGGAGCC CTGCTCCCTC CGGGGGCTGC TGGCCGGCCT 2640
   TTTCCAGAAG AAGCTGCTCC GAGGTTGCAC CCTCCTCCTC ACAGCCCGGC CCCGGGGCCG 2700
   CCTGGTCCAG AGCCTGAGCA AGGCCGACGC CCTATTTGAG CTGTCCGGCT TCTCCATGGA 2760
   GCAGGCCCAG GCATACGTGA TGCGCTACTT TGAGAGCTCA GGGATGACAG AGCACCAAGA 2820
   CAGAGCCCTG ACGCTCCTCC GGGACCGGCC ACTTCTTCTC AGTCACAGCC ACAGCCCTAC 2880
   TTTGTGCCGG GCAGTGTGCC AGCTCTCAGA GGCCCTGCTG GAGCTTGGGG AGGACGCCAA 2940
   GCTGCCCTCC ACGCTCACGG GACTCTATGT CGGCCTGCTG GGCCGTGCAG CCCTCGACAG 3000
   CCCCCCCGGG GCCCTGGCAG AGCTGGCCAA GCTGGCCTGG GAGCTGGGCC GCAGACATCA 3060
   AAGTACCCTA CAGGAGGACC AGTTCCCATC CGCAGACGTG AGGACCTGGG CGATGGCCAA 3120
   AGGCTTAGTC CAACACCCAC CGCGGGCCGC AGAGTCCGAG CTGGCCTTCC CCAGCTTCCT 3180
   CCTGCAATGC TTCCTGGGGG CCCTGTGGCT GGCTCTGAGT GGCGAAATCA AGGACAAGGA 3240
   GCTCCCGCAG TACCTAGCAT TGACCCCAAG GAAGAAGAGG CCCTATGACA ACTGGCTGGA 3300
   GGGCGTGCCA CGCTTTCTGG CTGGGCTGAT CTTCCAGCCT CCCGCCCGCT GCCTGGGAGC 3360
   CCTACTCGGG CCATCGGCGG CTGCCTCGGT GGACAGGAAG CAGAAGGTGC TTGCGAGGTA 3420
   CCTGAAGCGG CTGCAGCCGG GGACACTGCG GGCGCGGCAG CTGCTTGAGC TGCTGCACTG 3480
   CGCCCACGAG GCCGAGGAGG CTGGAATTTG GCAGCACGTG GTACAGGAGC TCCCCGGCCG 3540
   CCTCTCTTTT CTGGGCACCC GCCTCACGCC TCCTGATGCA CATGTACTGG GCAAGGCCTT 3600
   GGAGGCGGCG GGCCAAGACT TCTCCCTGGA CCTCCGCAGC ACTGGCATTT GCCCCTCTGG 3660
   ATTGGGGAGC CTCGTGGGAC TCAGCTGTGT CACCCGTTTC AGGGCTGCCT TGAGCGACAC 3720
   GGTGGCGCTG TGGGAGTCCC TGCGGCAGCA TGGGGAGACC AAGCTACTTC AGGCAGCAGA 3780
   GGAGAAGTTC ACCATCGAGC CTTTCAAAGC CAAGTCCCTG AAGGATGTGG AAGACCTGGG 3840
   AAAGCTTGTG CAGACTCAGA GGACGAGAAG TTCCTCGGAA GACACAGCTG GGGAGCTCCC 3900
   TGCTGTTCGG GACCTAAAGA AACTGGAGTT TGCGCTGGGC CCTGTCTCAG GCCCCCAGGC 3960
   TTTCCCCAAA CTGGTGCGGA TCCTCACGGC CTTTTCCTCC CTGCAGCATC TGGACCTGGA 4020
   TGCGCTGAGT GAGAACAAGA TCGGGGACGA GGGTGTCTCG CAGCTCTCAG CCACCTTCCC 4080
   CCAGCTGAAG TCCTTGGAAA CCCTCAATCT GTCCCAGAAC AACATCACTG ACCTGGGTGC 4140
   CTACAAACTC GCCGAGGCCC TGCCTTCGCT CGCTGCATCC CTGCTCAGGC TAAGCTTGTA 4200
   CAATAACTGC ATCTGCGACG TGGGAGCCGA GAGCTTGGCT CGTGTGCTTC CGGACATGGT 4260
   GTCCCTCCGG GTGATGGACG CAAGTTCACG GCTGCCGGGG CCCAGCAGCT CGCTGCCAGC 4320
   CTTCGGAGGT GTCCTCATGT GGAGACGCTG GCGATGTGGA CGCCCACCAT CCCATTCAGT 4380
   GTCCAGGAAC ACCTGCAACA ACAGGATTCA CGGATCAGCC TGAGATGATC CCAGCTGTGC 4440
   TCTGGACAGG CATGTTCTCT GAGGACACTA ACCACGCTGG ACCTTGAACT GGGTACTTGT 4500
   GGACACAGCT CTTCTCCAGG CTGTATCCCA TGAGGCCTCA GCATCCTGGC ACCCGGCCCC 4560
   TGCTGGTTCA GGGTTGGCCC CTGCCCGGCT GCGGAATGAA CCACATCTTG CTCTGCTGAC 4620
   AGACACAGGC CCGGCTCCAG GCTCCTTTAG CGCCCAGTTG GGTGGATGCC TGGTGGCAGC 4680
   TGCGGTCCAC CCAGGAGCCC CGAGGCCTTC TCTGAAGGAC ATTGCGGACA GCCACGGCCA 4740
   GGCCAGAGGG AGTGACAGAG GCAGCCCCAT TCTGCCTGCC CAGGCCCCTG CCACCCTGGG 4800
   GAGAAAGTAC TTCTTTTTTT TTATTTTTAG ACAGAGTCTC ACTGTTGCCC AGGCTGGCGT 4860
   GCAGTGGTGC GATCTGGGTT CACTGCAACC TCCGCCTCTT GGGTTCAAGC GATTCTTCTG 4920
   CTTCAGCCTC CCGAGTAGCT GGGACTACAG GCACCCACCA TCATGTCTGG CTAATTTTTC 4980
   ATTTTTAGTA GAGACAGGGT TTTGCCATGT TGGCCAGGCT GGTCTCAAAC TCTTGACCTC 5040
   AGGTGATCCA CCCACCTCAG CCTCCCAAAG TGCTGGGGAT TACAAGCGTG AGCCACTGCA 5100
   CCGGGCCACA GAGAAAGTAC TTCTCCACCC TGCTCTCCGA CCAGACACCT TGACAGGGCA 5160
   CACCGGGCAC TCAGAAGACA CTGATGGGCA ACCCCCAGCC TGCTAATTCC CCAGATTGCA 5220
   ACAGGCTGGG CTTCAGTGGC AGGCTGCTTT TGTCTATGGG ACTCAATGCA CTGACATTGT 5280
   TGGCCAAAGC CAAAGCTAGG CCTGGCCAGA TGCACCAGGC CCTTAGCAGG GAAACAGCTA 5340
   ATGGGACACT AATGGGGCGG TGAGAGGGGA ACAGACTGGA AGCACAGCTT CATTTCCTGT 5400
   GTCTTTTTTC ACTACATTAT AAATGTCTCT TTAATGTCAC AAAAAAAAAA AAAAAAAAAA 5460
   AAA 5463
(2) INFORMATIONS POUR LA SEQ ID NO: 2:
   (i) CARACTERISTIQUES DE LA SEQUENCE:
      (A) LONGUEUR: 4564 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: gène cIIta de type II
   (xi) DESCRIPTION DE LA SEQUENCE: SEQ ID NO: 2:
   CCCGGGCGCC CCGCCTCAGT TTCCCCATCT ATAAAGTGGA GATGATAATA GCATTCAGAG 60
   TCACTGATCT AAGGGCTCAG GGACACCATT CAGTGTAAGC CCCATACACT CCCTGCAAGA 120
   GGAAGCTGGT TCTGACTCAG CCTTGAGGCT GGCGTCTGAG GCAACCACAA GCCCAACGTG 180
   CATGGTGGAA AGATGACTGC AGCTCACAGT GTGCCACCAT GGAGTTGGGG CCCCTAGAAG 240
   GTGGCTACCT GGAGCTTCTT AACAGCGATG CTGACCCCCT GTGCCTCTAC CACTTCTATG 300
   ACCAGATGGA CCTGGCTGGA GAAGAAGAGA TTGAGCTCTA CTCAGAACCC GACACAGACA 360
   CCATCAACTG CGACCAGTTC AGCAGGCTGT TGTGTGACAT GGAAGGTGAT GAAGAGACCA 420
   GGGAGGCTTA TGCCAATATC GCGGAACTGG ACCAGTATGT CTTCCAGGAC TCCCAGCTGG 480
   AGGGCCTGAG CAAGGACATT TTCAAGCACA TAGGACCAGA TGAAGTGATC GGTGAGAGTA 540
   TGGAGATGCC AGCAGAAGTT GGGCAGAAAA GTCAGAAAAG ACCCTTCCCA GAGGAGCTTC 600
   CGGCAGACCT GAAGCACTGG AAGCCAGCTG AGCCCCCCAC TGTGGTGACT GGCAGTCTCC 660
   TAGTGGGACC AGTGAGCGAC TGCTCCACCC TGCCCTGCCT GCCACTGCCT GCGCTGTTCA 720
   ACCAGGAGCC AGCCTCCGGC CAGATGCGCC TGGAGAAAAC CGACCAGATT CCCATGCCTT 780
   TCTCCAGTTC CTCGTTGAGC TGCCTGAATC TCCCTGAGGG ACCCATCCAG TTTGTCCCCA 840
   CCATCTCCAC TCTGCCCCAT GGGCTCTGGC AAATCTCTGA GGCTGGAACA GGGGTCTCCA 900
   GTATATTCAT CTACCATGGT GAGGTGCCCC AGGCCAGCCA AGTACCCCCT CCCAGTGGAT 960
   TCACTGTCCA CGGCCTCCCA ACATCTCCAG ACCGGCCAGG CTCCACCAGC CCCTTCGCTC 1020
   CATCAGCCAC TGACCTGCCC AGCATGCCTG AACCTGCCCT GACCTCCCGA GCAAACATGA 1080
   CAGAGCACAA GACGTCCCCC ACCCAATGCC CGGCAGCTGG AGAGGTCTCC AACAAGCTTC 1140
   CAAAATGGCC TGAGCCGGTG GAGCAGTTCT ACCGCTCACT GCAGGACACG TATGGTGCCG 1200
   AGCCCGCAGG CCCGGATGGC ATCCTAGTGG AGGTGGATCT GGTGCAGGCC AGGCTGGAGA 1260
   GGAGCAGCAG CAAGAGCCTG GAGCGGGAAC TGGCCACCCC GGACTGGGCA GAACGGCAGC 1320
   TGGCCCAAGG AGGCCTGGCT GAGGTGCTGT TGGCTGCCAA GGAGCACCGG CGGCCGCGTG 1380
   AGACACGAGT GATTGCTGTG CTGGGCAAAG CTGGTCAGGG CAAGAGCTAT TGGGCTGGGG 1440
   CAGTGAGCCG GGCCTGGGCT TGTGGCCGGC TTCCCCAGTA CGACTTTGTC TTCTCTGTCC 1500
   CCTGCCATTG CTTGAACCGT CCGGGGGATG CCTATGGCCT GCAGGATCTG CTCTTCTCCC 1560
   TGGGCCCACA GCCACTCGTG GCGGCCGATG AGGTTTTCAG CCACATCTTG AAGAGACCTG 1620
   ACCGCGTTCT GCTCATCCTA GACGCCTTCG AGGAGCTGGA AGCGCAAGAT GGCTTCCTGC 1680
   ACAGCACGTG CGGACCGGCA CCGGCGGAGC CCTGCTCCCT CCGGGGGCTG CTGGCCGGCC 1740
   TTTTCCAGAA GAAGCTGCTC CGAGGTTGCA CCCTCCTCCT CACAGCCCGG CCCCGGGGCC 1800
   GCCTGGTCCA GAGCCTGAGC AAGGCCGACG CCCTATTTGA GCTGTCCGGC TTCTCCATGG 1860
   AGCAGGCCCA GGCATACGTG ATGCGCTACT TTGAGAGCTC AGGGATGACA GAGCACCAAG 1920
   ACAGAGCCCT GACGCTCCTC CGGGACCGGC CACTTCTTCT CAGTCACAGC CACAGCCCTA 1980
   CTTTGTGCCG GGCAGTGTGC CAGCTCTCAG AGGCCCTGCT GGAGCTTGGG GAGGACGCCA 2040
   AGCTGCCCTC CACGCTCACG GGACTCTATG TCGGCCTGCT GGGCCGTGCA GCCCTCGACA 2100
   GCCCCCCCGG GGCCCTGGCA GAGCTGGCCA AGCTGGCCTG GGAGCTGGGC CGCAGACATC 2160
   AAAGTACCCT ACAGGAGGAC CAGTTCCCAT CCGCAGACGT GAGGACCTGG GCGATGGCCA 2220
   AAGGCTTAGT CCAACACCCA CCGCGGGCCG CAGAGTCCGA GCTGGCCTTC CCCAGCTTCC 2280
   TCCTGCAATG CTTCCTGGGG GCCCTGTGGC TGGCTCTGAG TGGCGAAATC AAGGACAAGG 2340
   AGCTCCCGCA GTACCTAGCA TTGACCCCAA GGAAGAAGAG GCCCTATGAC AACTGGCTGG 2400
   AGGGCGTGCC ACGCTTTCTG GCTGGGCTGA TCTTCCAGCC TCCCGCCCGC TGCCTGGGAG 2460
   CCCTACTCGG GCCATCGGCG GCTGCCTCGG TGGACAGGAA GCAGAAGGTG CTTGCGAGGT 2520
   ACCTGAAGCG GCTGCAGCCG GGGACACTGC GGGCGCGGCA GCTGCTTGAG CTGCTGCACT 2580
   GCGCCCACGA GGCCGAGGAG GCTGGAATTT GGCAGCACGT GGTACAGGAG CTCCCCGGCC 2640
   GCCTCTCTTT TCTGGGCACC CGCCTCACGC CTCCTGATGC ACATGTACTG GGCAAGGCCT 2700
   TGGAGGCGGC GGGCCAAGAC TTCTCCCTGG ACCTCCGCAG CACTGGCATT TGCCCCTCTG 2760
   GATTGGGGAG CCTCGTGGGA CTCAGCTGTG TCACCCGTTT CAGGGCTGCC TTGAGCGACA 2820
   CGGTGGCGCT GTGGGAGTCC CTGCGGCAGC ATGGGGAGAC CAAGCTACTT CAGGCAGCAG 2880
   AGGAGAAGTT CACCATCGAG CCTTTCAAAG CCAAGTCCCT GAAGGATGTG GAAGACCTGG 2940
   GAAAGCTTGT GCAGACTCAG AGGACGAGAA GTTCCTCGGA AGACACAGCT GGGGAGCTCC 3000
   CTGCTGTTCG GGACCTAAAG AAACTGGAGT TTGCGCTGGG CCCTGTCTCA GGCCCCCAGG 3060
   CTTTCCCCAA ACTGGTGCGG ATCCTCACGG CCTTTTCCTC CCTGCAGCAT CTGGACCTGG 3120
   ATGCGCTGAG TGAGAACAAG ATCGGGGACG AGGGTGTCTC GCAGCTCTCA GCCACCTTCC 3180
   CCCAGCTGAA GTCCTTGGAA ACCCTCAATC TGTCCCAGAA CAACATCACT GACCTGGGTG 3240
   CCTACAAACT CGCCGAGGCC CTGCCTTCGC TCGCTGCATC CCTGCTCAGG CTAAGCTTGT 3300
   ACAATAACTG CATCTGCGAC GTGGGAGCCG AGAGCTTGGC TCGTGTGCTT CCGGACATGG 3360
   TGTCCCTCCG GGTGATGGAC GCAAGTTCAC GGCTGCCGGG GCCCAGCAGC TCGCTGCCAG 3420
   CCTTCGGAGG TGTCCTCATG TGGAGACGCT GGCGATGTGG ACGCCCACCA TCCCATTCAG 3480
   TGTCCAGGAA CACCTGCAAC AACAGGATTC ACGGATCAGC CTGAGATGAT CCCAGCTGTG 3540
   CTCTGGACAG GCATGTTCTC TGAGGACACT AACCACGCTG GACCTTGAAC TGGGTACTTG 3600
   TGGACACAGC TCTTCTCCAG GCTGTATCCC ATGAGGCCTC AGCATCCTGG CACCCGGCCC 3660
   CTGCTGGTTC AGGGTTGGCC CCTGCCCGGC TGCGGAATGA ACCACATCTT GCTCTGCTGA 3720
   CAGACACAGG CCCGGCTCCA GGCTCCTTTA GCGCCCAGTT GGGTGGATGC CTGGTGGCAG 3780
   CTGCGGTCCA CCCAGGAGCC CCGAGGCCTT CTCTGAAGGA CATTGCGGAC AGCCACGGCC 3840
   AGGCCAGAGG GAGTGACAGA GGCAGCCCCA TTCTGCCTGC CCAGGCCCCT GCCACCCTGG 3900
   GGAGAAAGTA CTTCTTTTTT TTTATTTTTA GACAGAGTCT CACTGTTGCC CAGGCTGGCG 3960
   TGCAGTGGTG CGATCTGGGT TCACTGCAAC CTCCGCCTCT TGGGTTCAAG CGATTCTTCT 4020
   GCTTCAGCCT CCCGAGTAGC TGGGACTACA GGCACCCACC ATCATGTCTG GCTAATTTTT 4080
   CATTTTTAGT AGAGACAGGG TTTTGCCATG TTGGCCAGGC TGGTCTCAAA CTCTTGACCT 4140
   CAGGTGATCC ACCCACCTCA GCCTCCCAAA GTGCTGGGGA TTACAAGCGT GAGCCACTGC 4200
   ACCGGGCCAC AGAGAAAGTA CTTCTCCACC CTGCTCTCCG ACCAGACACC TTGACAGGGC 4260
   ACACCGGGCA CTCAGAAGAC ACTGATGGGC AACCCCCAGC CTGCTAATTC CCCAGATTGC 4320
   AACAGGCTGG GCTTCAGTGG CAGGCTGCTT TTGTCTATGG GACTCAATGC ACTGACATTG 4380
   TTGGCCAAAG CCAAAGCTAG GCCTGGCCAG ATGCACCAGG CCCTTAGCAG GGAAACAGCT 4440
   AATGGGACAC TAATGGGGCG GTGAGAGGGG AACAGACTGG AAGCACAGCT TCATTTCCTG 4500
   TGTCTTTTTT CACTACATTA TAAATGTCTC TTTAATGTCA CAAAAAAAAA AAAAAAAAAA 4560
   AAAA 4564
(2) INFORMATIONS POUR LA SEQ ID NO: 3:
   (i) CARACTERISTIQUES DE LA SEQUENCE:
      (A) LONGUEUR: 5105 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: gene cIIta de type IV
   (xi) DESCRIPTION DE LA SEQUENCE: SEQ ID NO: 3:
   GGGGAGAAGT CAGAGGTAAC CTTGCCCCCT CCCTCAATTC CAGATGAGGA AATTCAGGCC 60
   TGAAAAGGGA AAGTGACCAC CTCAAAGTCT CATGCCTTGG AGGACCCAGC AGGAATCCAA 120
   GACCTCTGAA AAGGACCGGC AGGGCTCTTG CCACGGCTGG GGGTGTGGTC ATGGTAACAC 180
   AGGTTTTCCA TCCATGGAAG GTACCTGAGG GATTTTCTCT TCCTCCCTAG GGCCAGCATC 240
   AGAGGAGTGA ATAGCTCAGT TAGCTCATCT CAGGGGCCAT GTGCCCTCGG AGGTGGTTTG 300
   CCACTTTCAC GGTTGGACTG AGTTGGAGAG AAACAGAGAC CCACCCAGGG GTGGGGACAA 360
   GCTCCCTGCA ACTCAGGACT TGCAGATCAC TTGCCCAAGT GGCTCCCTAG CTCCTGGCTC 420
   CTGGCCCGGG GCCTGGGACT CTCCCCGAAG TGGGGCTGGC CACTGTGAGG AACCGACTGG 480
   AGGCAGGGAC CTCTTGGATG CCCCAGGCAG TTGGGATGCC ACTTCTGATA AAGCACGTGG 540
   TGGCCACAGT AGGTGCTTGG TTGCTCCACA GCCTGGCCCG AGCTCAGCGC TGCAGAAAGA 600
   AAGTGAAAGG GAAAAAGAAC TGCGGGGAGG CGGGGAGGTA GGATGACCAG CGGACGAGCT 660
   GCCACAGACT TGCCGCGGCC CCAGAGCTGG CGGGAGGGAG AGGCCACCAG CAGCGCGCGC 720
   GGGAGCCCGG GGAACAGCGG CAGCTCACAG TGTGCCACCA TGGAGTTGGG GCCCCTAGAA 780
   GGTGGCTACC TGGAGCTTCT TAACAGCGAT GCTGACCCCC TGTGCCTCTA CCACTTCTAT 840
   GACCAGATGG ACCTGGCTGG AGAAGAAGAG ATTGAGCTCT ACTCAGAACC CGACACAGAC 900
   ACCATCAACT GCGACCAGTT CAGCAGGCTG TTGTGTGACA TGGAAGGTGA TGAAGAGACC 960
   AGGGAGGCTT ATGCCAATAT CGCGGAACTG GACCAGTATG TCTTCCAGGA CTCCCAGCTG 1020
   GAGGGCCTGA GCAAGGACAT TTTCAAGCAC ATAGGACCAG ATGAAGTGAT CGGTGAGAGT 1080
   ATGGAGATGC CAGCAGAAGT TGGGCAGAAA AGTCAGAAAA GACCCTTCCC AGAGGAGCTT 1140
   CCGGCAGACC TGAAGCACTG GAAGCCAGCT GAGCCCCCCA CTGTGGTGAC TGGCAGTCTC 1200
   CTAGTGGGAC CAGTGAGCGA CTGCTCCACC CTGCCCTGCC TGCCACTGCC TGCGCTGTTC 1260
   AACCAGGAGC CAGCCTCCGG CCAGATGCGC CTGGAGAAAA CCGACCAGAT TCCCATGCCT 1320
   TTCTCCAGTT CCTCGTTGAG CTGCCTGAAT CTCCCTGAGG GACCCATCCA GTTTGTCCCC 1380
   ACCATCTCCA CTCTGCCCCA TGGGCTCTGG CAAATCTCTG AGGCTGGAAC AGGGGTCTCC 1440
   AGTATATTCA TCTACCATGG TGAGGTGCCC CAGGCCAGCC AAGTACCCCC TCCCAGTGGA 1500
   TTCACTGTCC ACGGCCTCCC AACATCTCCA GACCGGCCAG GCTCCACCAG CCCCTTCGCT 1560
   CCATCAGCCA CTGACCTGCC CAGCATGCCT GAACCTGCCC TGACCTCCCG AGCAAACATG 1620
   ACAGAGCACA AGACGTCCCC CACCCAATGC CCGGCAGCTG GAGAGGTCTC CAACAAGCTT 1680
   CCAAAATGGC CTGAGCCGGT GGAGCAGTTC TACCGCTCAC TGCAGGACAC GTATGGTGCC 1740
   GAGCCCGCAG GCCCGGATGG CATCCTAGTG GAGGTGGATC TGGTGCAGGC CAGGCTGGAG 1800
   AGGAGCAGCA GCAAGAGCCT GGAGCGGGAA CTGGCCACCC CGGACTGGGC AGAACGGCAG 1860
   CTGGCCCAAG GAGGCCTGGC TGAGGTGCTG TTGGCTGCCA AGGAGCACCG GCGGCCGCGT 1920
   GAGACACGAG TGATTGCTGT GCTGGGCAAA GCTGGTCAGG GCAAGAGCTA TTGGGCTGGG 1980
   GCAGTGAGCC GGGCCTGGGC TTGTGGCCGG CTTCCCCAGT ACGACTTTGT CTTCTCTGTC 2040
   CCCTGCCATT GCTTGAACCG TCCGGGGGAT GCCTATGGCC TGCAGGATCT GCTCTTCTCC 2100
   CTGGGCCCAC AGCCACTCGT GGCGGCCGAT GAGGTTTTCA GCCACATCTT GAAGAGACCT 2160
   GACCGCGTTC TGCTCATCCT AGACGCCTTC GAGGAGCTGG AAGCGCAAGA TGGCTTCCTG 2220
   CACAGCACGT GCGGACCGGC ACCGGCGGAG CCCTGCTCCC TCCGGGGGCT GCTGGCCGGC 2280
   CTTTTCCAGA AGAAGCTGCT CCGAGGTTGC ACCCTCCTCC TCACAGCCCG GCCCCGGGGC 2340
   CGCCTGGTCC AGAGCCTGAG CAAGGCCGAC GCCCTATTTG AGCTGTCCGG CTTCTCCATG 2400
   GAGCAGGCCC AGGCATACGT GATGCGCTAC TTTGAGAGCT CAGGGATGAC AGAGCACCAA 2460
   GACAGAGCCC TGACGCTCCT CCGGGACCGG CCACTTCTTC TCAGTCACAG CCACAGCCCT 2520
   ACTTTGTGCC GGGCAGTGTG CCAGCTCTCA GAGGCCCTGC TGGAGCTTGG GGAGGACGCC 2580
   AAGCTGCCCT CCACGCTCAC GGGACTCTAT GTCGGCCTGC TGGGCCGTGC AGCCCTCGAC 2640
   AGCCCCCCCG GGGCCCTGGC AGAGCTGGCC AAGCTGGCCT GGGAGCTGGG CCGCAGACAT 2700
   CAAAGTACCC TACAGGAGGA CCAGTTCCCA TCCGCAGACG TGAGGACCTG GGCGATGGCC 2760
   AAAGGCTTAG TCCAACACCC ACCGCGGGCC GCAGAGTCCG AGCTGGCCTT CCCCAGCTTC 2820
   CTCCTGCAAT GCTTCCTGGG GGCCCTGTGG CTGGCTCTGA GTGGCGAAAT CAAGGACAAG 2880
   GAGCTCCCGC AGTACCTAGC ATTGACCCCA AGGAAGAAGA GGCCCTATGA CAACTGGCTG 2940
   GAGGGCGTGC CACGCTTTCT GGCTGGGCTG ATCTTCCAGC CTCCCGCCCG CTGCCTGGGA 3000
   GCCCTACTCG GGCCATCGGC GGCTGCCTCG GTGGACAGGA AGCAGAAGGT GCTTGCGAGG 3060
   TACCTGAAGC GGCTGCAGCC GGGGACACTG CGGGCGCGGC AGCTGCTTGA GCTGCTGCAC 3120
   TGCGCCCACG AGGCCGAGGA GGCTGGAATT TGGCAGCACG TGGTACAGGA GCTCCCCGGC 3180
   CGCCTCTCTT TTCTGGGCAC CCGCCTCACG CCTCCTGATG CACATGTACT GGGCAAGGCC 3240
   TTGGAGGCGG CGGGCCAAGA CTTCTCCCTG GACCTCCGCA GCACTGGCAT TTGCCCCTCT 3300
   GGATTGGGGA GCCTCGTGGG ACTCAGCTGT GTCACCCGTT TCAGGGCTGC CTTGAGCGAC 3360
   ACGGTGGCGC TGTGGGAGTC CCTGCGGCAG CATGGGGAGA CCAAGCTACT TCAGGCAGCA 3420
   GAGGAGAAGT TCACCATCGA GCCTTTCAAA GCCAAGTCCC TGAAGGATGT GGAAGACCTG 3480
   GGAAAGCTTG TGCAGACTCA GAGGACGAGA AGTTCCTCGG AAGACACAGC TGGGGAGCTC 3540
   CCTGCTGTTC GGGACCTAAA GAAACTGGAG TTTGCGCTGG GCCCTGTCTC AGGCCCCCAG 3600
   GCTTTCCCCA AACTGGTGCG GATCCTCACG GCCTTTTCCT CCCTGCAGCA TCTGGACCTG 3660
   GATGCGCTGA GTGAGAACAA GATCGGGGAC GAGGGTGTCT CGCAGCTCTC AGCCACCTTC 3720
   CCCCAGCTGA AGTCCTTGGA AACCCTCAAT CTGTCCCAGA ACAACATCAC TGACCTGGGT 3780
   GCCTACAAAC TCGCCGAGGC CCTGCCTTCG CTCGCTGCAT CCCTGCTCAG GCTAAGCTTG 3840
   TACAATAACT GCATCTGCGA CGTGGGAGCC GAGAGCTTGG CTCGTGTGCT TCCGGACATG 3900
   GTGTCCCTCC GGGTGATGGA CGCAAGTTCA CGGCTGCCGG GGCCCAGCAG CTCGCTGCCA 3960
   GCCTTCGGAG GTGTCCTCAT GTGGAGACGC TGGCGATGTG GACGCCCACC ATCCCATTCA 4020
   GTGTCCAGGA ACACCTGCAA CAACAGGATT CACGGATCAG CCTGAGATGA TCCCAGCTGT 4080
   GCTCTGGACA GGCATGTTCT CTGAGGACAC TAACCACGCT GGACCTTGAA CTGGGTACTT 4140
   GTGGACACAG CTCTTCTCCA GGCTGTATCC CATGAGGCCT CAGCATCCTG GCACCCGGCC 4200
   CCTGCTGGTT CAGGGTTGGC CCCTGCCCGG CTGCGGAATG AACCACATCT TGCTCTGCTG 4260
   ACAGACACAG GCCCGGCTCC AGGCTCCTTT AGCGCCCAGT TGGGTGGATG CCTGGTGGCA 4320
   GCTGCGGTCC ACCCAGGAGC CCCGAGGCCT TCTCTGAAGG ACATTGCGGA CAGCCACGGC 4380
   CAGGCCAGAG GGAGTGACAG AGGCAGCCCC ATTCTGCCTG CCCAGGCCCC TGCCACCCTG 4440
   GGGAGAAAGT ACTTCTTTTT TTTTATTTTT AGACAGAGTC TCACTGTTGC CCAGGCTGGC 4500
   GTGCAGTGGT GCGATCTGGG TTCACTGCAA CCTCCGCCTC TTGGGTTCAA GCGATTCTTC 4560
   TGCTTCAGCC TCCCGAGTAG CTGGGACTAC AGGCACCCAC CATCATGTCT GGCTAATTTT 4620
   TCATTTTTAG TAGAGACAGG GTTTTGCCAT GTTGGCCAGG CTGGTCTCAA ACTCTTGACC 4680
   TCAGGTGATC CACCCACCTC AGCCTCCCAA AGTGCTGGGG ATTACAAGCG TGAGCCACTG 4740
   CACCGGGCCA CAGAGAAAGT ACTTCTCCAC CCTGCTCTCC GACCAGACAC CTTGACAGGG 4800
   CACACCGGGC ACTCAGAAGA CACTGATGGG CAACCCCCAG CCTGCTAATT CCCCAGATTG 4860
   CAACAGGCTG GGCTTCAGTG GCAGGCTGCT TTTGTCTATG GGACTCAATG CACTGACATT 4920
   GTTGGCCAAA GCCAAAGCTA GGCCTGGCCA GATGCACCAG GCCCTTAGCA GGGAAACAGC 4980
   TAATGGGACA CTAATGGGGC GGTGAGAGGG GAACAGACTG GAAGCACAGC TTCATTTCCT 5040
   GTGTCTTTTT TCACTACATT ATAAATGTCT CTTTAATGTC ACAAAAAAAA AAAAAAAAAA 5100
   AAAAA 5105
(2) INFORMATIONS POUR LA SEQ ID NO: 4:
   (i) CARACTERISTIQUES DE LA SEQUENCE:
      (A) LONGUEUR: 717 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: promoteur cIIta de type I
   (xi) DESCRIPTION DE LA SEQUENCE: SEQ ID NO: 4:
   GTAAGTACTG TAACAGAGAC TAAATGCTAA GTAAGGCAGG CGTGGTGGCT CACACTTGTA    60
   ATCCCAGTAC TTTGGAGGAC TGAGGCAAGA GGATCACTTG AGCCCAGAAT TCAAGACCAG   120
   CCTGGGAAAC AGAGTGAGAC CCTGTCTCTA CTAAAAATAA AAAAATAAAA AATTAGCGGG   180
   GCATGATGGT GTGAGTCTGT AGTCCCAGCT ACTGGGAAAC AGAGATGGGA GGTTTGTTTG   240
   AGCCTGGGAA GTTGAGGCTG CAGTGAGTTA TGATCACGCC ACTGCACACC AGCCTGGGCA   300
   ACAGAGCAAG ACCCTGTCAA AAAAAAAAAA AAGGCTATGT AACACCCCCA AGTCACATAA   360
   TTGGCAAGGA GCAGCAGATC TGGGACTTGA ACATAGGCAG ATTAGCTCCA AGCCTATTTG   420
   CTTAACCTCT ATACCACAAT GCCTTCTTGC TATGGTAAAA CATCTGAAAA GACCTATTAC   480
   CCTATAGGTC CTCTAAGGAG GCATGTCGCC TTCCTCTTAG CAATACTAGA TTGGCTCCAA   540
   CAGAAGGCTG TGGGCTTCTC TGGCACATGC ACCTGGGTAG GACCCAGAGA ATATCTGTGG   600
   AGTCTGAATC AACCCAAAAG CCAATATCCA TCCGTTCATC AGGAACCCCA GCCTACAACG   660
   CAAAAGAGGA AATCTTCCTA AGTAGAAATA AACTGTAATA AATTGCAGAG GTTCCCT      717
(2) INFORMATIONS POUR LA SEQ ID NO: 5:
   (i) CARACTERISTIQUES DE LA SEQUENCE:
      (A) LONGUEUR: 133 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: promoteur cIIta de type II
   (xi) DESCRIPTION. DE LA SEQUENCE: SEQ ID NO: 5:
(2) INFORMATIONS POUR LA SEQ ID NO: 6:
   (i) CARACTERISTIQUES DE LA SEQUENCE:
      (A) LONGUEUR: 664 paires de bases
      (B) TYPE: nucléotide
      (C) NOMBRE: DE BRINS: simple
      (D) CONFIGURATION: linéaire
   (ii) TYPE DE MOLECULE: ADN (génomique)
   (ix) CARACTERISTIQUE:
      (A) NOM/CLE: promoteur cIIta de type IV
   (xi) DESCRIPTION DE LA SEQUENCE: SEQ ID NO: 6:
   GGGGAGAAGT CAGAGGTAAC CTTGCCCCCT CCCTCAATTC CAGATGAGGA AATTCAGGCC    60
   TGAAAAGGGA AAGTGACCAC CTCAAAGTCT CATGCCTTGG AGGACCCAGC AGGAATCCAA   120
   GACCTCTGAA AAGGACCGGC AGGGCTCTTG CCACGGCTGG GGGTGTGGTC ATGGTAACAC   180
   AGGTTTTCCA TCCATGGAAG GTACCTGAGG GATTTTCTCT TCCTCCCTAG GGCCAGCATC   240
   AGAGGAGTGA ATAGCTCAGT TAGCTCATCT CAGGGGCCAT GTGCCCTCGG AGGTGGTTTG   300
   CCACTTTCAC GGTTGGACTG AGTTGGAGAG AAACAGAGAC CCACCCAGGG GTGGGGACAA   360
   GCTCCCTGCA ACTCAGGACT TGCAGATCAC TTGCCCAAGT GGCTCCCTAG CTCCTGGCTC   420
   CTGGCCCGGG GCCTGGGACT CTCCCCGAAG TGGGGCTGGC CACTGTGAGG AACCGACTGG   480
   AGGCAGGGAC CTCTTGGATG CCCCAGGCAG TTGGGATGCC ACTTCTGATA AAGCACGTGG   540
   TGGCCACAGT AGGTGCTTGG TTGCTCCACA GCCTGGCCCG AGCTCAGCGC TGCAGAAAGA   600
   AAGTGAAAGG GAAAAAGAAC TGCGGGGAGG CGGGGAGGTA GGATGACCAG CGGACGAGCT   660
   GCCA                                                                664
(2) INFORMATIONS POUR LA SEQ ID NO: 7:
   (i) CARACTERISTIQUES DE LA SEQUENCE:
      (A) LONGUEUR: 4746 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: cIIta de type I
   (xi) DESCRIPTION DE LA SEQUENCE: SEQ ID NO: 7:
   CGTCCTGGTT TTCACTTCAT GTTTTGGATG CTGCATGCTG GGTGAGCGGA GATTCCAGGC 60
   ACTGGCCAGG GCAGCTGCCC TGACTCCAAG GGCTGCCATG AACAACTTCC AGGCCATCCT 120
   GACTCAGGTG AGAATGCTGC TCTCCAGCCA TCAGCCCAGC CTGGTGCAGG CCCTCTTGGA 180
   CAACCTGCTG AAGGAGGACC TCCTCTCCAG GGAATACCAC TGCACTCTGC TCCATGAGCC 240
   TGATAGTGAG GCTCTGGCCA GGAAGATCTC TTTGACCCTA CTAGAGAAAG GAGACCTGGA 300
   TTTGGCCCTC CTGGGGTGGG CCCGGAGTGG GCTGCAGCCC CCAGCAGCCG AGAGGGGCCC 360
   CGGCCACAGT GACCATGGTG GCAGCTCACA GTGTGCCACC ATGGAGTTGG GGCCCCTAGA 420
   AGGTGGCTAC CTGGAGCTTC TTAACAGCGA TGCTGACCCC CTGTGCCTCT ACCACTTCTA 480
   TGACCAGATG GACCTGGCTG GAGAAGAAGA GATTGAGCTC TACTCAGAAC CCGACACAGA 540
   CACCATCAAC TGCGACCAGT TCAGCAGGCT GTTGTGTGAC ATGGAAGGTG ATGAAGAGAC 600
   CAGGGAGGCT TATGCCAATA TCGCGGAACT GGACCAGTAT GTCTTCCAGG ACTCCCAGCT 660
   GGAGGGCCTG AGCAAGGACA TTTTCAAGCA CATAGGACCA GATGAAGTGA TCGGTGAGAG 720
   TATGGAGATG CCAGCAGAAG TTGGGCAGAA AAGTCAGAAA AGACCCTTCC CAGAGGAGCT 780
   TCCGGCAGAC CTGAAGCACT GGAAGCCAGC TGAGCCCCCC ACTGTGGTGA CTGGCAGTCT 840
   CCTAGTGGGA CCAGTGAGCG ACTGCTCCAC CCTGCCCTGC CTGCCACTGC CTGCGCTGTT 900
   CAACCAGGAG CCAGCCTCCG GCCAGATGCG CCTGGAGAAA ACCGACCAGA TTCCCATGCC 960
   TTTCTCCAGT TCCTCGTTGA GCTGCCTGAA TCTCCCTGAG GGACCCATCC AGTTTGTCCC 1020
   CACCATCTCC ACTCTGCCCC ATGGGCTCTG GCAAATCTCT GAGGCTGGAA CAGGGGTCTC 1080
   CAGTATATTC ATCTACCATG GTGAGGTGCC CCAGGCCAGC CAAGTACCCC CTCCCAGTGG 1140
   ATTCACTGTC CACGGCCTCC CAACATCTCC AGACCGGCCA GGCTCCACCA GCCCCTTCGC 1200
   TCCATCAGCC ACTGACCTGC CCAGCATGCC TGAACCTGCC CTGACCTCCC GAGCAAACAT 1260
   GACAGAGCAC AAGACGTCCC CCACCCAATG CCCGGCAGCT GGAGAGGTCT CCAACAAGCT 1320
   TCCAAAATGG CCTGAGCCGG TGGAGCAGTT CTACCGCTCA CTGCAGGACA CGTATGGTGC 1380
   CGAGCCCGCA GGCCCGGATG GCATCCTAGT GGAGGTGGAT CTGGTGCAGG CCAGGCTGGA 1440
   GAGGAGCAGC AGCAAGAGCC TGGAGCGGGA ACTGGCCACC CCGGACTGGG CAGAACGGCA 1500
   GCTGGCCCAA GGAGGCCTGG CTGAGGTGCT GTTGGCTGCC AAGGAGCACC GGCGGCCGCG 1560
   TGAGACACGA GTGATTGCTG TGCTGGGCAA AGCTGGTCAG GGCAAGAGCT ATTGGGCTGG 1620
   GGCAGTGAGC CGGGCCTGGG CTTGTGGCCG GCTTCCCCAG TACGACTTTG TCTTCTCTGT 1680
   CCCCTGCCAT TGCTTGAACC GTCCGGGGGA TGCCTATGGC CTGCAGGATC TGCTCTTCTC 1740
   CCTGGGCCCA CAGCCACTCG TGGCGGCCGA TGAGGTTTTC AGCCACATCT TGAAGAGACC 1800
   TGACCGCGTT CTGCTCATCC TAGACGCCTT CGAGGAGCTG GAAGCGCAAG ATGGCTTCCT 1860
   GCACAGCACG TGCGGACCGG CACCGGCGGA GCCCTGCTCC CTCCGGGGGC TGCTGGCCGG 1920
   CCTTTTCCAG AAGAAGCTGC TCCGAGGTTG CACCCTCCTC CTCACAGCCC GGCCCCGGGG 1980
   CCGCCTGGTC CAGAGCCTGA GCAAGGCCGA CGCCCTATTT GAGCTGTCCG GCTTCTCCAT 2040
   GGAGCAGGCC CAGGCATACG TGATGCGCTA CTTTGAGAGC TCAGGGATGA CAGAGCACCA 2100
   AGACAGAGCC CTGACGCTCC TCCGGGACCG GCCACTTCTT CTCAGTCACA GCCACAGCCC 2160
   TACTTTGTGC CGGGCAGTGT GCCAGCTCTC AGAGGCCCTG CTGGAGCTTG GGGAGGACGC 2220
   CAAGCTGCCC TCCACGCTCA CGGGACTCTA TGTCGGCCTG CTGGGCCGTG CAGCCCTCGA 2280
   CAGCCCCCCC GGGGCCCTGG CAGAGCTGGC CAAGCTGGCC TGGGAGCTGG GCCGCAGACA 2340
   TCAAAGTACC CTACAGGAGG ACCAGTTCCC ATCCGCAGAC GTGAGGACCT GGGCGATGGC 2400
   CAAAGGCTTA GTCCAACACC CACCGCGGGC CGCAGAGTCC GAGCTGGCCT TCCCCAGCTT 2460
   CCTCCTGCAA TGCTTCCTGG GGGCCCTGTG GCTGGCTCTG AGTGGCGAAA TCAAGGACAA 2520
   GGAGCTCCCG CAGTACCTAG CATTGACCCC AAGGAAGAAG AGGCCCTATG ACAACTGGCT 2580
   GGAGGGCGTG CCACGCTTTC TGGCTGGGCT GATCTTCCAG CCTCCCGCCC GCTGCCTGGG 2640
   AGCCCTACTC GGGCCATCGG CGGCTGCCTC GGTGGACAGG AAGCAGAAGG TGCTTGCGAG 2700
   GTACCTGAAG CGGCTGCAGC CGGGGACACT GCGGGCGCGG CAGCTGCTTG AGCTGCTGCA 2760
   CTGCGCCCAC GAGGCCGAGG AGGCTGGAAT TTGGCAGCAC GTGGTACAGG AGCTCCCCGG 2820
   CCGCCTCTCT TTTCTGGGCA CCCGCCTCAC GCCTCCTGAT GCACATGTAC TGGGCAAGGC 2880
   CTTGGAGGCG GCGGGCCAAG ACTTCTCCCT GGACCTCCGC AGCACTGGCA TTTGCCCCTC 2940
   TGGATTGGGG AGCCTCGTGG GACTCAGCTG TGTCACCCGT TTCAGGGCTG CCTTGAGCGA 3000
   CACGGTGGCG CTGTGGGAGT CCCTGCGGCA GCATGGGGAG ACCAAGCTAC TTCAGGCAGC 3060
   AGAGGAGAAG TTCACCATCG AGCCTTTCAA AGCCAAGTCC CTGAAGGATG TGGAAGACCT 3120
   GGGAAAGCTT GTGCAGACTC AGAGGACGAG AAGTTCCTCG GAAGACACAG CTGGGGAGCT 3180
   CCCTGCTGTT CGGGACCTAA AGAAACTGGA GTTTGCGCTG GGCCCTGTCT CAGGCCCCCA 3240
   GGCTTTCCCC AAACTGGTGC GGATCCTCAC GGCCTTTTCC TCCCTGCAGC ATCTGGACCT 3300
   GGATGCGCTG AGTGAGAACA AGATCGGGGA CGAGGGTGTC TCGCAGCTCT CAGCCACCTT 3360
   CCCCCAGCTG AAGTCCTTGG AAACCCTCAA TCTGTCCCAG AACAACATCA CTGACCTGGG 3420
   TGCCTACAAA CTCGCCGAGG CCCTGCCTTC GCTCGCTGCA TCCCTGCTCA GGCTAAGCTT 3480
   GTACAATAAC TGCATCTGCG ACGTGGGAGC CGAGAGCTTG GCTCGTGTGC TTCCGGACAT 3540
   GGTGTCCCTC CGGGTGATGG ACGCAAGTTC ACGGCTGCCG GGGCCCAGCA GCTCGCTGCC 3600
   AGCCTTCGGA GGTGTCCTCA TGTGGAGACG CTGGCGATGT GGACGCCCAC CATCCCATTC 3660
   AGTGTCCAGG AACACCTGCA ACAACAGGAT TCACGGATCA GCCTGAGATG ATCCCAGCTG 3720
   TGCTCTGGAC AGGCATGTTC TCTGAGGACA CTAACCACGC TGGACCTTGA ACTGGGTACT 3780
   TGTGGACACA GCTCTTCTCC AGGCTGTATC CCATGAGGCC TCAGCATCCT GGCACCCGGC 3840
   CCCTGCTGGT TCAGGGTTGG CCCCTGCCCG GCTGCGGAAT GAACCACATC TTGCTCTGCT 3900
   GACAGACACA GGCCCGGCTC CAGGCTCCTT TAGCGCCCAG TTGGGTGGAT GCCTGGTGGC 3960
   AGCTGCGGTC CACCCAGGAG CCCCGAGGCC TTCTCTGAAG GACATTGCGG ACAGCCACGG 4020
   CCAGGCCAGA GGGAGTGACA GAGGCAGCCC CATTCTGCCT GCCCAGGCCC CTGCCACCCT 4080
   GGGGAGAAAG TACTTCTTTT TTTTTATTTT TAGACAGAGT CTCACTGTTG CCCAGGCTGG 4140
   CGTGCAGTGG TGCGATCTGG GTTCACTGCA ACCTCCGCCT CTTGGGTTCA AGCGATTCTT 4200
   CTGCTTCAGC CTCCCGAGTA GCTGGGACTA CAGGCACCCA CCATCATGTC TGGCTAATTT 4260
   TTCATTTTTA GTAGAGACAG GGTTTTGCCA TGTTGGCCAG GCTGGTCTCA AACTCTTGAC 4320
   CTCAGGTGAT CCACCCACCT CAGCCTCCCA AAGTGCTGGG GATTACAAGC GTGAGCCACT 4380
   GCACCGGGCC ACAGAGAAAG TACTTCTCCA CCCTGCTCTC CGACCAGACA CCTTGACAGG 4440
   GCACACCGGG CACTCAGAAG ACACTGATGG GCAACCCCCA GCCTGCTAAT TCCCCAGATT 4500
   GCAACAGGCT GGGCTTCAGT GGCAGGCTGC TTTTGTCTAT GGGACTCAAT GCACTGACAT 4560
   TGTTGGCCAA AGCCAAAGCT AGGCCTGGCC AGATGCACCA GGCCCTTAGC AGGGAAACAG 4620
   CTAATGGGAC ACTAATGGGG CGGTGAGAGG GGAACAGACT GGAAGCACAG CTTCATTTCC 4680
   TGTGTCTTTT TTCACTACAT TATAAATGTC TCTTTAATGT CACAAAAAAA AAAAAAAAAA 4740
   AAAAAA                                                            4746
(2) INFORMATIONS POUR LA SEQ ID NO: 8:
   (i) CARACTERISTIQUES DE LA SEQUENCE:
      (A) LONGUEUR: 4431 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) MOM/CLE: cIIta de type II
   (xi) DESCRIPTION DE LA SEQUENCE: SEQ ID NO: 8:
   GACTCAGCCT TGAGGCTGGC GTCTGAGGCA ACCACAAGCC CAACGTGCAT GGTGGAAAGA 60
   TGACTGCAGC TCACAGTGTG CCACCATGGA GTTGGGGCCC CTAGAAGGTG GCTACCTGGA 120
   GCTTCTTAAC AGCGATGCTG ACCCCCTGTG CCTCTACCAC TTCTATGACC AGATGGACCT 180
   GGCTGGAGAA GAAGAGATTG AGCTCTACTC AGAACCCGAC ACAGACACCA TCAACTGCGA 240
   CCAGTTCAGC AGGCTGTTGT GTGACATGGA AGGTGATGAA GAGACCAGGG AGGCTTATGC 300
   CAATATCGCG GAACTGGACC AGTATGTCTT CCAGGACTCC CAGCTGGAGG GCCTGAGCAA 360
   GGACATTTTC AAGCACATAG GACCAGATGA AGTGATCGGT GAGAGTATGG AGATGCCAGC 420
   AGAAGTTGGG CAGAAAAGTC AGAAAAGACC CTTCCCAGAG GAGCTTCCGG CAGACCTGAA 480
   GCACTGGAAG CCAGCTGAGC CCCCCACTGT GGTGACTGGC AGTCTCCTAG TGGGACCAGT 540
   GAGCGACTGC TCCACCCTGC CCTGCCTGCC ACTGCCTGCG CTGTTCAACC AGGAGCCAGC 600
   CTCCGGCCAG ATGCGCCTGG AGAAAACCGA CCAGATTCCC ATGCCTTTCT CCAGTTCCTC 660
   GTTGAGCTGC CTGAATCTCC CTGAGGGACC CATCCAGTTT GTCCCCACCA TCTCCACTCT 720
   GCCCCATGGG CTCTGGCAAA TCTCTGAGGC TGGAACAGGG GTCTCCAGTA TATTCATCTA 780
   CCATGGTGAG GTGCCCCAGG CCAGCCAAGT ACCCCCTCCC AGTGGATTCA CTGTCCACGG 840
   CCTCCCAACA TCTCCAGACC GGCCAGGCTC CACCAGCCCC TTCGCTCCAT CAGCCACTGA 900
   CCTGCCCAGC ATGCCTGAAC CTGCCCTGAC CTCCCGAGCA AACATGACAG AGCACAAGAC 960
   GTCCCCCACC CAATGCCCGG CAGCTGGAGA GGTCTCCAAC AAGCTTCCAA AATGGCCTGA 1020
   GCCGGTGGAG CAGTTCTACC GCTCACTGCA GGACACGTAT GGTGCCGAGC CCGCAGGCCC 1080
   GGATGGCATC CTAGTGGAGG TGGATCTGGT GCAGGCCAGG CTGGAGAGGA GCAGCAGCAA 1140
   GAGCCTGGAG CGGGAACTGG CCACCCCGGA CTGGGCAGAA CGGCAGCTGG CCCAAGGAGG 1200
   CCTGGCTGAG GTGCTGTTGG CTGCCAAGGA GCACCGGCGG CCGCGTGAGA CACGAGTGAT 1260
   TGCTGTGCTG GGCAAAGCTG GTCAGGGCAA GAGCTATTGG GCTGGGGCAG TGAGCCGGGC 1320
   CTGGGCTTGT GGCCGGCTTC CCCAGTACGA CTTTGTCTTC TCTGTCCCCT GCCATTGCTT 1380
   GAACCGTCCG GGGGATGCCT ATGGCCTGCA GGATCTGCTC TTCTCCCTGG GCCCACAGCC 1440
   ACTCGTGGCG GCCGATGAGG TTTTCAGCCA CATCTTGAAG AGACCTGACC GCGTTCTGCT 1500
   CATCCTAGAC GCCTTCGAGG AGCTGGAAGC GCAAGATGGC TTCCTGCACA GCACGTGCGG 1560
   ACCGGCACCG GCGGAGCCCT GCTCCCTCCG GGGGCTGCTG GCCGGCCTTT TCCAGAAGAA 1620
   GCTGCTCCGA GGTTGCACCC TCCTCCTCAC AGCCCGGCCC CGGGGCCGCC TGGTCCAGAG 1680
   CCTGAGCAAG GCCGACGCCC TATTTGAGCT GTCCGGCTTC TCCATGGAGC AGGCCCAGGC 1740
   ATACGTGATG CGCTACTTTG AGAGCTCAGG GATGACAGAG CACCAAGACA GAGCCCTGAC 1800
   GCTCCTCCGG GACCGGCCAC TTCTTCTCAG TCACAGCCAC AGCCCTACTT TGTGCCGGGC 1860
   AGTGTGCCAG CTCTCAGAGG CCCTGCTGGA GCTTGGGGAG GACGCCAAGC TGCCCTCCAC 1920
   GCTCACGGGA CTCTATGTCG GCCTGCTGGG CCGTGCAGCC CTCGACAGCC CCCCCGGGGC 1980
   CCTGGCAGAG CTGGCCAAGC TGGCCTGGGA GCTGGGCCGC AGACATCAAA GTACCCTACA 2040
   GGAGGACCAG TTCCCATCCG CAGACGTGAG GACCTGGGCG ATGGCCAAAG GCTTAGTCCA 2100
   ACACCCACCG CGGGCCGCAG AGTCCGAGCT GGCCTTCCCC AGCTTCCTCC TGCAATGCTT 2160
   CCTGGGGGCC CTGTGGCTGG CTCTGAGTGG CGAAATCAAG GACAAGGAGC TCCCGCAGTA 2220
   CCTAGCATTG ACCCCAAGGA AGAAGAGGCC CTATGACAAC TGGCTGGAGG GCGTGCCACG 2280
   CTTTCTGGCT GGGCTGATCT TCCAGCCTCC CGCCCGCTGC CTGGGAGCCC TACTCGGGCC 2340
   ATCGGCGGCT GCCTCGGTGG ACAGGAAGCA GAAGGTGCTT GCGAGGTACC TGAAGCGGCT 2400
   GCAGCCGGGG ACACTGCGGG CGCGGCAGCT GCTTGAGCTG CTGCACTGCG CCCACGAGGC 2460
   CGAGGAGGCT GGAATTTGGC AGCACGTGGT ACAGGAGCTC CCCGGCCGCC TCTCTTTTCT 2520
   GGGCACCCGC CTCACGCCTC CTGATGCACA TGTACTGGGC AAGGCCTTGG AGGCGGCGGG 2580
   CCAAGACTTC TCCCTGGACC TCCGCAGCAC TGGCATTTGC CCCTCTGGAT TGGGGAGCCT 2640
   CGTGGGACTC AGCTGTGTCA CCCGTTTCAG GGCTGCCTTG AGCGACACGG TGGCGCTGTG 2700
   GGAGTCCCTG CGGCAGCATG GGGAGACCAA GCTACTTCAG GCAGCAGAGG AGAAGTTCAC 2760
   CATCGAGCCT TTCAAAGCCA AGTCCCTGAA GGATGTGGAA GACCTGGGAA AGCTTGTGCA 2820
   GACTCAGAGG ACGAGAAGTT CCTCGGAAGA CACAGCTGGG GAGCTCCCTG CTGTTCGGGA 2880
   CCTAAAGAAA CTGGAGTTTG CGCTGGGCCC TGTCTCAGGC CCCCAGGCTT TCCCCAAACT 2940
   GGTGCGGATC CTCACGGCCT TTTCCTCCCT GCAGCATCTG GACCTGGATG CGCTGAGTGA 3000
   GAACAAGATC GGGGACGAGG GTGTCTCGCA GCTCTCAGCC ACCTTCCCCC AGCTGAAGTC 3060
   CTTGGAAACC CTCAATCTGT CCCAGAACAA CATCACTGAC CTGGGTGCCT ACAAACTCGC 3120
   CGAGGCCCTG CCTTCGCTCG CTGCATCCCT GCTCAGGCTA AGCTTGTACA ATAACTGCAT 3180
   CTGCGACGTG GGAGCCGAGA GCTTGGCTCG TGTGCTTCCG GACATGGTGT CCCTCCGGGT 3240
   GATGGACGCA AGTTCACGGC TGCCGGGGCC CAGCAGCTCG CTGCCAGCCT TCGGAGGTGT 3300
   CCTCATGTGG AGACGCTGGC GATGTGGACG CCCACCATCC CATTCAGTGT CCAGGAACAC 3360
   CTGCAACAAC AGGATTCACG GATCAGCCTG AGATGATCCC AGCTGTGCTC TGGACAGGCA 3420
   TGTTCTCTGA GGACACTAAC CACGCTGGAC CTTGAACTGG GTACTTGTGG ACACAGCTCT 3480
   TCTCCAGGCT GTATCCCATG AGGCCTCAGC ATCCTGGCAC CCGGCCCCTG CTGGTTCAGG 3540
   GTTGGCCCCT GCCCGGCTGC GGAATGAACC ACATCTTGCT CTGCTGACAG ACACAGGCCC 3600
   GGCTCCAGGC TCCTTTAGCG CCCAGTTGGG TGGATGCCTG GTGGCAGCTG CGGTCCACCC 3660
   AGGAGCCCCG AGGCCTTCTC TGAAGGACAT TGCGGACAGC CACGGCCAGG CCAGAGGGAG 3720
   TGACAGAGGC AGCCCCATTC TGCCTGCCCA GGCCCCTGCC ACCCTGGGGA GAAAGTACTT 3780
   CTTTTTTTTT ATTTTTAGAC AGAGTCTCAC TGTTGCCCAG GCTGGCGTGC AGTGGTGCGA 3840
   TCTGGGTTCA CTGCAACCTC CGCCTCTTGG GTTCAAGCGA TTCTTCTGCT TCAGCCTCCC 3900
   GAGTAGCTGG GACTACAGGC ACCCACCATC ATGTCTGGCT AATTTTTCAT TTTTAGTAGA 3960
   GACAGGGTTT TGCCATGTTG GCCAGGCTGG TCTCAAACTC TTGACCTCAG GTGATCCACC 4020
   CACCTCAGCC TCCCAAAGTG CTGGGGATTA CAAGCGTGAG CCACTGCACC GGGCCACAGA 4080
   GAAAGTACTT CTCCACCCTG CTCTCCGACC AGACACCTTG ACAGGGCACA CCGGGCACTC 4140
   AGAAGACACT GATGGGCAAC CCCCAGCCTG CTAATTCCCC AGATTGCAAC AGGCTGGGCT 4200
   TCAGTGGCAG GCTGCTTTTG TCTATGGGAC TCAATGCACT GACATTGTTG GCCAAAGCCA 4260
   AAGCTAGGCC TGGCCAGATG CACCAGGCCC TTAGCAGGGA AACAGCTAAT GGGACACTAA 4320
   TGGGGCGGTG AGAGGGGAAC AGACTGGAAG CACAGCTTCA TTTCCTGTGT CTTTTTTCAC 4380
   TACATTATAA ATGTCTCTTT AATGTCACAA AAAAAAAAAA AAAAAAAAAA A 4431
(2) INFORMATIONS POUR LA SEQ ID NO: 9:
   (i) CARACTERISTIQUES DE LA SEQUENCE:
      (A) LONGUEUR: 4549 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: cIIta de type III
   (xi) DESCRIPTION DE LA SEQUENCE: SEQ ID NO: 9:
   TTAGTGATGA GGCTAGTGAT GAGGCTGTGT GCTTCTGAGC TGGGCATCCG AAGGCATCCT     60
   TGGGGAAGCT GAGGGCACGA GGAGGGGCTG CCAGACTCCG GGAGCTGCTG CCTGGCTGGG    120
   ATTCCTACAC AATGCGTTGC CTGGCTCCAC GCCCTGCTGG GTCCTACCTG TCAGAGCCCC    180
   AAGGCAGCTC ACAGTGTGCC ACCATGGAGT TGGGGCCCCT AGAAGGTGGC TACCTGGAGC    240
   TTCTTAACAG CGATGCTGAC CCCCTGTGCC TCTACCACTT CTATGACCAG ATGGACCTGG    300
   CTGGAGAAGA AGAGATTGAG CTCTACTCAG AACCCGACAC AGACACCATC AACTGCGACC    360
   AGTTCAGCAG GCTGTTGTGT GACATGGAAG GTGATGAAGA GACCAGGGAG GCTTATGCCA    420
   ATATCGCGGA ACTGGACCAG TATGTCTTCC AGGACTCCCA GCTGGAGGGC CTGAGCAAGG    480
   ACATTTTCAA GCACATAGGA CCAGATGAAG TGATCGGTGA GAGTATGGAG ATGCCAGCAG    540
   AAGTTGGGCA GAAAAGTCAG AAAAGACCCT TCCCAGAGGA GCTTCCGGCA GACCTGAAGC    600
   ACTGGAAGCC AGCTGAGCCC CCCACTGTGG TGACTGGCAG TCTCCTAGTG GGACCAGTGA    660
   GCGACTGCTC CACCCTGCCC TGCCTGCCAC TGCCTGCGCT GTTCAACCAG GAGCCAGCCT    720
   CCGGCCAGAT GCGCCTGGAG AAAACCGACC AGATTCCCAT GCCTTTCTCC AGTTCCTCGT    780
   TGAGCTGCCT GAATCTCCCT GAGGGACCCA TCCAGTTTGT CCCCACCATC TCCACTCTGC    840
   CCCATGGGCT CTGGCAAATC TCTGAGGCTG GAACAGGGGT CTCCAGTATA TTCATCTACC    900
   ATGGTGAGGT GCCCCAGGCC AGCCAAGTAC CCCCTCCCAG TGGATTCACT GTCCACGGCC    960
   TCCCAACATC TCCAGACCGG CCAGGCTCCA CCAGCCCCTT CGCTCCATCA GCCACTGACC   1020
   TGCCCAGCAT GCCTGAACCT GCCCTGACCT CCCGAGCAAA CATGACAGAG CACAAGACGT   1080
   CCCCCACCCA ATGCCCGGCA GCTGGAGAGG TCTCCAACAA GCTTCCAAAA TGGCCTGAGC   1140
   CGGTGGAGCA GTTCTACCGC TCACTGCAGG ACACGTATGG TGCCGAGCCC GCAGGCCCGG   1200
   ATGGCATCCT AGTGGAGGTG GATCTGGTGC AGGCCAGGCT GGAGAGGAGC AGCAGCAAGA   1260
   GCCTGGAGCG GGAACTGGCC ACCCCGGACT GGGCAGAACG GCAGCTGGCC CAAGGAGGCC   1320
   TGGCTGAGGT GCTGTTGGCT GCCAAGGAGC ACCGGCGGCC GCGTGAGACA CGAGTGATTG   1380
   CTGTGCTGGG CAAAGCTGGT CAGGGCAAGA GCTATTGGGC TGGGGCAGTG AGCCGGGCCT   1440
   GGGCTTGTGG CCGGCTTCCC CAGTACGACT TTGTCTTCTC TGTCCCCTGC CATTGCTTGA   1500
   ACCGTCCGGG GGATGCCTAT GGCCTGCAGG ATCTGCTCTT CTCCCTGGGC CCACAGCCAC   1560
   TCGTGGCGGC CGATGAGGTT TTCAGCCACA TCTTGAAGAG ACCTGACCGC GTTCTGCTCA   1620
   TCCTAGACGC CTTCGAGGAG CTGGAAGCGC AAGATGGCTT CCTGCACAGC ACGTGCGGAC 1680
   CGGCACCGGC GGAGCCCTGC TCCCTCCGGG GGCTGCTGGC CGGCCTTTTC CAGAAGAAGC 1740
   TGCTCCGAGG TTGCACCCTC CTCCTCACAG CCCGGCCCCG GGGCCGCCTG GTCCAGAGCC 1800
   TGAGCAAGGC CGACGCCCTA TTTGAGCTGT CCGGCTTCTC CATGGAGCAG GCCCAGGCAT 1860
   ACGTGATGCG CTACTTTGAG AGCTCAGGGA TGACAGAGCA CCAAGACAGA GCCCTGACGC 1920
   TCCTCCGGGA CCGGCCACTT CTTCTCAGTC ACAGCCACAG CCCTACTTTG TGCCGGGCAG 1980
   TGTGCCAGCT CTCAGAGGCC CTGCTGGAGC TTGGGGAGGA CGCCAAGCTG CCCTCCACGC 2040
   TCACGGGACT CTATGTCGGC CTGCTGGGCC GTGCAGCCCT CGACAGCCCC CCCGGGGCCC 2100
   TGGCAGAGCT GGCCAAGCTG GCCTGGGAGC TGGGCCGCAG ACATCAAAGT ACCCTACAGG 2160
   AGGACCAGTT CCCATCCGCA GACGTGAGGA CCTGGGCGAT GGCCAAAGGC TTAGTCCAAC 2220
   ACCCACCGCG GGCCGCAGAG TCCGAGCTGG CCTTCCCCAG CTTCCTCCTG CAATGCTTCC 2280
   TGGGGGCCCT GTGGCTGGCT CTGAGTGGCG AAATCAAGGA CAAGGAGCTC CCGCAGTACC 2340
   TAGCATTGAC CCCAAGGAAG AAGAGGCCCT ATGACAACTG GCTGGAGGGC GTGCCACGCT 2400
   TTCTGGCTGG GCTGATCTTC CAGCCTCCCG CCCGCTGCCT GGGAGCCCTA CTCGGGCCAT 2460
   CGGCGGCTGC CTCGGTGGAC AGGAAGCAGA AGGTGCTTGC GAGGTACCTG AAGCGGCTGC 2520
   AGCCGGGGAC ACTGCGGGCG CGGCAGCTGC TTGAGCTGCT GCACTGCGCC CACGAGGCCG 2580
   AGGAGGCTGG AATTTGGCAG CACGTGGTAC AGGAGCTCCC CGGCCGCCTC TCTTTTCTGG 2640
   GCACCCGCCT CACGCCTCCT GATGCACATG TACTGGGCAA GGCCTTGGAG GCGGCGGGCC 2700
   AAGACTTCTC CCTGGACCTC CGCAGCACTG GCATTTGCCC CTCTGGATTG GGGAGCCTCG 2760
   TGGGACTCAG CTGTGTCACC CGTTTCAGGG CTGCCTTGAG CGACACGGTG GCGCTGTGGG 2820
   AGTCCCTGCG GCAGCATGGG GAGACCAAGC TACTTCAGGC AGCAGAGGAG AAGTTCACCA 2880
   TCGAGCCTTT CAAAGCCAAG TCCCTGAAGG ATGTGGAAGA CCTGGGAAAG CTTGTGCAGA 2940
   CTCAGAGGAC GAGAAGTTCC TCGGAAGACA CAGCTGGGGA GCTCCCTGCT GTTCGGGACC 3000
   TAAAGAAACT GGAGTTTGCG CTGGGCCCTG TCTCAGGCCC CCAGGCTTTC CCCAAACTGG 3060
   TGCGGATCCT CACGGCCTTT TCCTCCCTGC AGCATCTGGA CCTGGATGCG CTGAGTGAGA 3120
   ACAAGATCGG GGACGAGGGT GTCTCGCAGC TCTCAGCCAC CTTCCCCCAG CTGAAGTCCT 3180
   TGGAAACCCT CAATCTGTCC CAGAACAACA TCACTGACCT GGGTGCCTAC AAACTCGCCG 3240
   AGGCCCTGCC TTCGCTCGCT GCATCCCTGC TCAGGCTAAG CTTGTACAAT AACTGCATCT   3300
   GCGACGTGGG AGCCGAGAGC TTGGCTCGTG TGCTTCCGGA CATGGTGTCC CTCCGGGTGA   3360
   TGGACGCAAG TTCACGGCTG CCGGGGCCCA GCAGCTCGCT GCCAGCCTTC GGAGGTGTCC   3420
   TCATGTGGAG ACGCTGGCGA TGTGGACGCC CACCATCCCA TTCAGTGTCC AGGAACACCT   3480
   GCAACAACAG GATTCACGGA TCAGCCTGAG ATGATCCCAG CTGTGCTCTG GACAGGCATG   3540
   TTCTCTGAGG ACACTAACCA CGCTGGACCT TGAACTGGGT ACTTGTGGAC ACAGCTCTTC   3600
   TCCAGGCTGT ATCCCATGAG GCCTCAGCAT CCTGGCACCC GGCCCCTGCT GGTTCAGGGT   3660
   TGGCCCCTGC CCGGCTGCGG AATGAACCAC ATCTTGCTCT GCTGACAGAC ACAGGCCCGG   3720
   CTCCAGGCTC CTTTAGCGCC CAGTTGGGTG GATGCCTGGT GGCAGCTGCG GTCCACCCAG   3780
   GAGCCCCGAG GCCTTCTCTG AAGGACATTG CGGACAGCCA CGGCCAGGCC AGAGGGAGTG   3840
   ACAGAGGCAG CCCCATTCTG CCTGCCCAGG CCCCTGCCAC CCTGGGGAGA AAGTACTTCT   3900
   TTTTTTTTAT TTTTAGACAG AGTCTCACTG TTGCCCAGGC TGGCGTGCAG TGGTGCGATC   3960
   TGGGTTCACT GCAACCTCCG CCTCTTGGGT TCAAGCGATT CTTCTGCTTC AGCCTCCCGA   4020
   GTAGCTGGGA CTACAGGCAC CCACCATCAT GTCTGGCTAA TTTTTCATTT TTAGTAGAGA   4080
   CAGGGTTTTG CCATGTTGGC CAGGCTGGTC TCAAACTCTT GACCTCAGGT GATCCACCCA   4140
   CCTCAGCCTC CCAAAGTGCT GGGGATTACA AGCGTGAGCC ACTGCACCGG GCCACAGAGA   4200
   AAGTACTTCT CCACCCTGCT CTCCGACCAG ACACCTTGAC AGGGCACACC GGGCACTCAG   4260
   AAGACACTGA TGGGCAACCC CCAGCCTGCT AATTCCCCAG ATTGCAACAG GCTGGGCTTC   4320
   AGTGGCAGGC TGCTTTTGTC TATGGGACTC AATGCACTGA CATTGTTGGC CAAAGCCAAA   4380
   GCTAGGCCTG GCCAGATGCA CCAGGCCCTT AGCAGGGAAA CAGCTAATGG GACACTAATG   4440
   GGGCGGTGAG AGGGGAACAG ACTGGAAGCA CAGCTTCATT TCCTGTGTCT TTTTTCACTA   4500
   CATTATAAAT GTCTCTTTAA TGTCACAAAA AAAAAAAAAA AAAAAAAAA              4549
(2) INFORMATIONS POUR LA SEQ ID NO: 10:
   (i) CARACTERISTIQUES DE LA SEQUENCE:
      (A) LONGUEUR: 4441 paires de bases
      (B) TYPE: nucléotide
      (C) NOMBRE: DE BRINS: simple
      (D) CONFIGURATION: linéaire
   (ii) TYPE DE MOLECULE: ADN (génomique)
   (ix) CARACTERISTIQUE:
      (A) NOM/CLE: cIIta de type IV
   (xi) DESCRIPTION DE LA SEQUENCE: SEQ ID NO: 10:
   CAGACTTGCC GCGGCCCCAG AGCTGGCGGG AGGGAGAGGC CACCAGCAGC GCGCGCGGGA 60
   GCCCGGGGAA CAGCGGCAGC TCACAGTGTG CCACCATGGA GTTGGGGCCC CTAGAAGGTG 120
   GCTACCTGGA GCTTCTTAAC AGCGATGCTG ACCCCCTGTG CCTCTACCAC TTCTATGACC 180
   AGATGGACCT GGCTGGAGAA GAAGAGATTG AGCTCTACTC AGAACCCGAC ACAGACACCA 240
   TCAACTGCGA CCAGTTCAGC AGGCTGTTGT GTGACATGGA AGGTGATGAA GAGACCAGGG 300
   AGGCTTATGC CAATATCGCG GAACTGGACC AGTATGTCTT CCAGGACTCC CAGCTGGAGG 360
   GCCTGAGCAA GGACATTTTC AAGCACATAG GACCAGATGA AGTGATCGGT GAGAGTATGG 420
   AGATGCCAGC AGAAGTTGGG CAGAAAAGTC AGAAAAGACC CTTCCCAGAG GAGCTTCCGG 480
   CAGACCTGAA GCACTGGAAG CCAGCTGAGC CCCCCACTGT GGTGACTGGC AGTCTCCTAG 540
   TGGGACCAGT GAGCGACTGC TCCACCCTGC CCTGCCTGCC ACTGCCTGCG CTGTTCAACC 600
   AGGAGCCAGC CTCCGGCCAG ATGCGCCTGG AGAAAACCGA CCAGATTCCC ATGCCTTTCT 660
   CCAGTTCCTC GTTGAGCTGC CTGAATCTCC CTGAGGGACC CATCCAGTTT GTCCCCACCA 720
   TCTCCACTCT GCCCCATGGG CTCTGGCAAA TCTCTGAGGC TGGAACAGGG GTCTCCAGTA 780
   TATTCATCTA CCATGGTGAG GTGCCCCAGG CCAGCCAAGT ACCCCCTCCC AGTGGATTCA 840
   CTGTCCACGG CCTCCCAACA TCTCCAGACC GGCCAGGCTC CACCAGCCCC TTCGCTCCAT 900
   CAGCCACTGA CCTGCCCAGC ATGCCTGAAC CTGCCCTGAC CTCCCGAGCA AACATGACAG 960
   AGCACAAGAC GTCCCCCACC CAATGCCCGG CAGCTGGAGA GGTCTCCAAC AAGCTTCCAA 1020
   AATGGCCTGA GCCGGTGGAG CAGTTCTACC GCTCACTGCA GGACACGTAT GGTGCCGAGC 1080
   CCGCAGGCCC GGATGGCATC CTAGTGGAGG TGGATCTGGT GCAGGCCAGG CTGGAGAGGA 1140
   GCAGCAGCAA GAGCCTGGAG CGGGAACTGG CCACCCCGGA CTGGGCAGAA CGGCAGCTGG 1200
   CCCAAGGAGG CCTGGCTGAG GTGCTGTTGG CTGCCAAGGA GCACCGGCGG CCGCGTGAGA 1260
   CACGAGTGAT TGCTGTGCTG GGCAAAGCTG GTCAGGGCAA GAGCTATTGG GCTGGGGCAG 1320
   TGAGCCGGGC CTGGGCTTGT GGCCGGCTTC CCCAGTACGA CTTTGTCTTC TCTGTCCCCT 1380
   GCCATTGCTT GAACCGTCCG GGGGATGCCT ATGGCCTGCA GGATCTGCTC TTCTCCCTGG 1440
   GCCCACAGCC ACTCGTGGCG GCCGATGAGG TTTTCAGCCA CATCTTGAAG AGACCTGACC 1500
   GCGTTCTGCT CATCCTAGAC GCCTTCGAGG AGCTGGAAGC GCAAGATGGC TTCCTGCACA 1560
   GCACGTGCGG ACCGGCACCG GCGGAGCCCT GCTCCCTCCG GGGGCTGCTG GCCGGCCTTT 1620
   TCCAGAAGAA GCTGCTCCGA GGTTGCACCC TCCTCCTCAC AGCCCGGCCC CGGGGCCGCC 1680
   TGGTCCAGAG CCTGAGCAAG GCCGACGCCC TATTTGAGCT GTCCGGCTTC TCCATGGAGC 1740
   AGGCCCAGGC ATACGTGATG CGCTACTTTG AGAGCTCAGG GATGACAGAG CACCAAGACA 1800
   GAGCCCTGAC GCTCCTCCGG GACCGGCCAC TTCTTCTCAG TCACAGCCAC AGCCCTACTT 1860
   TGTGCCGGGC AGTGTGCCAG CTCTCAGAGG CCCTGCTGGA GCTTGGGGAG GACGCCAAGC 1920
   TGCCCTCCAC GCTCACGGGA CTCTATGTCG GCCTGCTGGG CCGTGCAGCC CTCGACAGCC 1980
   CCCCCGGGGC CCTGGCAGAG CTGGCCAAGC TGGCCTGGGA GCTGGGCCGC AGACATCAAA 2040
   GTACCCTACA GGAGGACCAG TTCCCATCCG CAGACGTGAG GACCTGGGCG ATGGCCAAAG 2100
   GCTTAGTCCA ACACCCACCG CGGGCCGCAG AGTCCGAGCT GGCCTTCCCC AGCTTCCTCC 2160
   TGCAATGCTT CCTGGGGGCC CTGTGGCTGG CTCTGAGTGG CGAAATCAAG GACAAGGAGC 2220
   TCCCGCAGTA CCTAGCATTG ACCCCAAGGA AGAAGAGGCC CTATGACAAC TGGCTGGAGG 2280
   GCGTGCCACG CTTTCTGGCT GGGCTGATCT TCCAGCCTCC CGCCCGCTGC CTGGGAGCCC 2340
   TACTCGGGCC ATCGGCGGCT GCCTCGGTGG ACAGGAAGCA GAAGGTGCTT GCGAGGTACC 2400
   TGAAGCGGCT GCAGCCGGGG ACACTGCGGG CGCGGCAGCT GCTTGAGCTG CTGCACTGCG 2460
   CCCACGAGGC CGAGGAGGCT GGAATTTGGC AGCACGTGGT ACAGGAGCTC CCCGGCCGCC 2520
   TCTCTTTTCT GGGCACCCGC CTCACGCCTC CTGATGCACA TGTACTGGGC AAGGCCTTGG 2580
   AGGCGGCGGG CCAAGACTTC TCCCTGGACC TCCGCAGCAC TGGCATTTGC CCCTCTGGAT 2640
   TGGGGAGCCT CGTGGGACTC AGCTGTGTCA CCCGTTTCAG GGCTGCCTTG AGCGACACGG 2700
   TGGCGCTGTG GGAGTCCCTG CGGCAGCATG GGGAGACCAA GCTACTTCAG GCAGCAGAGG 2760
   AGAAGTTCAC CATCGAGCCT TTCAAAGCCA AGTCCCTGAA GGATGTGGAA GACCTGGGAA 2820
   AGCTTGTGCA GACTCAGAGG ACGAGAAGTT CCTCGGAAGA CACAGCTGGG GAGCTCCCTG 2880
   CTGTTCGGGA CCTAAAGAAA CTGGAGTTTG CGCTGGGCCC TGTCTCAGGC CCCCAGGCTT 2940
   TCCCCAAACT GGTGCGGATC CTCACGGCCT TTTCCTCCCT GCAGCATCTG GACCTGGATG 3000
   CGCTGAGTGA GAACAAGATC GGGGACGAGG GTGTCTCGCA GCTCTCAGCC ACCTTCCCCC 3060
   AGCTGAAGTC CTTGGAAACC CTCAATCTGT CCCAGAACAA CATCACTGAC CTGGGTGCCT 3120
   ACAAACTCGC CGAGGCCCTG CCTTCGCTCG CTGCATCCCT GCTCAGGCTA AGCTTGTACA 3180
   ATAACTGCAT CTGCGACGTG GGAGCCGAGA GCTTGGCTCG TGTGCTTCCG GACATGGTGT 3240
   CCCTCCGGGT GATGGACGCA AGTTCACGGC TGCCGGGGCC CAGCAGCTCG CTGCCAGCCT 3300
   TCGGAGGTGT CCTCATGTGG AGACGCTGGC GATGTGGACG CCCACCATCC CATTCAGTGT 3360
   CCAGGAACAC CTGCAACAAC AGGATTCACG GATCAGCCTG AGATGATCCC AGCTGTGCTC 3420
   TGGACAGGCA TGTTCTCTGA GGACACTAAC CACGCTGGAC CTTGAACTGG GTACTTGTGG 3480
   ACACAGCTCT TCTCCAGGCT GTATCCCATG AGGCCTCAGC ATCCTGGCAC CCGGCCCCTG 3540
   CTGGTTCAGG GTTGGCCCCT GCCCGGCTGC GGAATGAACC ACATCTTGCT CTGCTGACAG 3600
   ACACAGGCCC GGCTCCAGGC TCCTTTAGCG CCCAGTTGGG TGGATGCCTG GTGGCAGCTG 3660
   CGGTCCACCC AGGAGCCCCG AGGCCTTCTC TGAAGGACAT TGCGGACAGC CACGGCCAGG 3720
   CCAGAGGGAG TGACAGAGGC AGCCCCATTC TGCCTGCCCA GGCCCCTGCC ACCCTGGGGA 3780
   GAAAGTACTT CTTTTTTTTT ATTTTTAGAC AGAGTCTCAC TGTTGCCCAG GCTGGCGTGC 3840
   AGTGGTGCGA TCTGGGTTCA CTGCAACCTC CGCCTCTTGG GTTCAAGCGA TTCTTCTGCT 3900
   TCAGCCTCCC GAGTAGCTGG GACTACAGGC ACCCACCATC ATGTCTGGCT AATTTTTCAT 3960
   TTTTAGTAGA GACAGGGTTT TGCCATGTTG GCCAGGCTGG TCTCAAACTC TTGACCTCAG 4020
   GTGATCCACC CACCTCAGCC TCCCAAAGTG CTGGGGATTA CAAGCGTGAG CCACTGCACC 4080
   GGGCCACAGA GAAAGTACTT CTCCACCCTG CTCTCCGACC AGACACCTTG ACAGGGCACA 4140
   CCGGGCACTC AGAAGACACT GATGGGCAAC CCCCAGCCTG CTAATTCCCC AGATTGCAAC 4200
   AGGCTGGGCT TCAGTGGCAG GCTGCTTTTG TCTATGGGAC TCAATGCACT GACATTGTTG 4260
   GCCAAAGCCA AAGCTAGGCC TGGCCAGATG CACCAGGCCC TTAGCAGGGA AACAGCTAAT 4320
   GGGACACTAA TGGGGCGGTG AGAGGGGAAC AGACTGGAAG CACAGCTTCA TTTCCTGTGT 4380
   CTTTTTTCAC TACATTATAA ATGTCTCTTT AATGTCACAA AAAAAAAAAA AAAAAAAAAA 4440
   A 4441
(2) INFORMATIONS POUR LA SEQ ID NO: 11:
   (i) CARACTERISTIQUES DE LA SEQUENCE:
      (A) LONGUEUR: 4649 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: cIIta de type I
   (xi) DESCRIPTION DE LA SEQUENCE: SEQ ID NO: 11:
   ATGAACAACT TCCAGGCCAT CCTGACTCAG GTGAGAATGC TGCTCTCCAG CCATCAGCCC     60
   AGCCTGGTGC AGGCCCTCTT GGACAACCTG CTGAAGGAGG ACCTCCTCTC CAGGGAATAC    120
   CACTGCACTC TGCTCCATGA GCCTGATAGT GAGGCTCTGG CCAGGAAGAT CTCTTTGACC    180
   CTACTAGAGA AAGGAGACCT GGATTTGGCC CTCCTGGGGT GGGCCCGGAG TGGGCTGCAG    240
   CCCCCAGCAG CCGAGAGGGG CCCCGGCCAC AGTGACCATG GTGGCAGCTC ACAGTGTGCC    300
   ACCATGGAGT TGGGGCCCCT AGAAGGTGGC TACCTGGAGC TTCTTAACAG CGATGCTGAC    360
   CCCCTGTGCC TCTACCACTT CTATGACCAG ATGGACCTGG CTGGAGAAGA AGAGATTGAG    420
   CTCTACTCAG AACCCGACAC AGACACCATC AACTGCGACC AGTTCAGCAG GCTGTTGTGT    480
   GACATGGAAG GTGATGAAGA GACCAGGGAG GCTTATGCCA ATATCGCGGA ACTGGACCAG    540
   TATGTCTTCC AGGACTCCCA GCTGGAGGGC CTGAGCAAGG ACATTTTCAA GCACATAGGA    600
   CCAGATGAAG TGATCGGTGA GAGTATGGAG ATGCCAGCAG AAGTTGGGCA GAAAAGTCAG    660
   AAAAGACCCT TCCCAGAGGA GCTTCCGGCA GACCTGAAGC ACTGGAAGCC AGCTGAGCCC    720
   CCCACTGTGG TGACTGGCAG TCTCCTAGTG GGACCAGTGA GCGACTGCTC CACCCTGCCC    780
   TGCCTGCCAC TGCCTGCGCT GTTCAACCAG GAGCCAGCCT CCGGCCAGAT GCGCCTGGAG    840
   AAAACCGACC AGATTCCCAT GCCTTTCTCC AGTTCCTCGT TGAGCTGCCT GAATCTCCCT    900
   GAGGGACCCA TCCAGTTTGT CCCCACCATC TCCACTCTGC CCCATGGGCT CTGGCAAATC    960
   TCTGAGGCTG GAACAGGGGT CTCCAGTATA TTCATCTACC ATGGTGAGGT GCCCCAGGCC   1020
   AGCCAAGTAC CCCCTCCCAG TGGATTCACT GTCCACGGCC TCCCAACATC TCCAGACCGG   1080
   CCAGGCTCCA CCAGCCCCTT CGCTCCATCA GCCACTGACC TGCCCAGCAT GCCTGAACCT   1140
   GCCCTGACCT CCCGAGCAAA CATGACAGAG CACAAGACGT CCCCCACCCA ATGCCCGGCA   1200
   GCTGGAGAGG TCTCCAACAA GCTTCCAAAA TGGCCTGAGC CGGTGGAGCA GTTCTACCGC 1260
   TCACTGCAGG ACACGTATGG TGCCGAGCCC GCAGGCCCGG ATGGCATCCT AGTGGAGGTG 1320
   GATCTGGTGC AGGCCAGGCT GGAGAGGAGC AGCAGCAAGA GCCTGGAGCG GGAACTGGCC 1380
   ACCCCGGACT GGGCAGAACG GCAGCTGGCC CAAGGAGGCC TGGCTGAGGT GCTGTTGGCT 1440
   GCCAAGGAGC ACCGGCGGCC GCGTGAGACA CGAGTGATTG CTGTGCTGGG CAAAGCTGGT 1500
   CAGGGCAAGA GCTATTGGGC TGGGGCAGTG AGCCGGGCCT GGGCTTGTGG CCGGCTTCCC 1560
   CAGTACGACT TTGTCTTCTC TGTCCCCTGC CATTGCTTGA ACCGTCCGGG GGATGCCTAT 1620
   GGCCTGCAGG ATCTGCTCTT CTCCCTGGGC CCACAGCCAC TCGTGGCGGC CGATGAGGTT 1680
   TTCAGCCACA TCTTGAAGAG ACCTGACCGC GTTCTGCTCA TCCTAGACGC CTTCGAGGAG 1740
   CTGGAAGCGC AAGATGGCTT CCTGCACAGC ACGTGCGGAC CGGCACCGGC GGAGCCCTGC 1800
   TCCCTCCGGG GGCTGCTGGC CGGCCTTTTC CAGAAGAAGC TGCTCCGAGG TTGCACCCTC 1860
   CTCCTCACAG CCCGGCCCCG GGGCCGCCTG GTCCAGAGCC TGAGCAAGGC CGACGCCCTA 1920
   TTTGAGCTGT CCGGCTTCTC CATGGAGCAG GCCCAGGCAT ACGTGATGCG CTACTTTGAG 1980
   AGCTCAGGGA TGACAGAGCA CCAAGACAGA GCCCTGACGC TCCTCCGGGA CCGGCCACTT 2040
   CTTCTCAGTC ACAGCCACAG CCCTACTTTG TGCCGGGCAG TGTGCCAGCT CTCAGAGGCC 2100
   CTGCTGGAGC TTGGGGAGGA CGCCAAGCTG CCCTCCACGC TCACGGGACT CTATGTCGGC 2160
   CTGCTGGGCC GTGCAGCCCT CGACAGCCCC CCCGGGGCCC TGGCAGAGCT GGCCAAGCTG 2220
   GCCTGGGAGC TGGGCCGCAG ACATCAAAGT ACCCTACAGG AGGACCAGTT CCCATCCGCA 2280
   GACGTGAGGA CCTGGGCGAT GGCCAAAGGC TTAGTCCAAC ACCCACCGCG GGCCGCAGAG 2340
   TCCGAGCTGG CCTTCCCCAG CTTCCTCCTG CAATGCTTCC TGGGGGCCCT GTGGCTGGCT 2400
   CTGAGTGGCG AAATCAAGGA CAAGGAGCTC CCGCAGTACC TAGCATTGAC CCCAAGGAAG 2460
   AAGAGGCCCT ATGACAACTG GCTGGAGGGC GTGCCACGCT TTCTGGCTGG GCTGATCTTC 2520
   CAGCCTCCCG CCCGCTGCCT GGGAGCCCTA CTCGGGCCAT CGGCGGCTGC CTCGGTGGAC 2580
   AGGAAGCAGA AGGTGCTTGC GAGGTACCTG AAGCGGCTGC AGCCGGGGAC ACTGCGGGCG 2640
   CGGCAGCTGC TTGAGCTGCT GCACTGCGCC CACGAGGCCG AGGAGGCTGG AATTTGGCAG 2700
   CACGTGGTAC AGGAGCTCCC CGGCCGCCTC TCTTTTCTGG GCACCCGCCT CACGCCTCCT 2760
   GATGCACATG TACTGGGCAA GGCCTTGGAG GCGGCGGGCC AAGACTTCTC CCTGGACCTC 2820
   CGCAGCACTG GCATTTGCCC CTCTGGATTG GGGAGCCTCG TGGGACTCAG CTGTGTCACC 2880
   CGTTTCAGGG CTGCCTTGAG CGACACGGTG GCGCTGTGGG AGTCCCTGCG GCAGCATGGG 2940
   GAGACCAAGC TACTTCAGGC AGCAGAGGAG AAGTTCACCA TCGAGCCTTT CAAAGCCAAG 3000
   TCCCTGAAGG ATGTGGAAGA CCTGGGAAAG CTTGTGCAGA CTCAGAGGAC GAGAAGTTCC 3060
   TCGGAAGACA CAGCTGGGGA GCTCCCTGCT GTTCGGGACC TAAAGAAACT GGAGTTTGCG 3120
   CTGGGCCCTG TCTCAGGCCC CCAGGCTTTC CCCAAACTGG TGCGGATCCT CACGGCCTTT 3180
   TCCTCCCTGC AGCATCTGGA CCTGGATGCG CTGAGTGAGA ACAAGATCGG GGACGAGGGT 3240
   GTCTCGCAGC TCTCAGCCAC CTTCCCCCAG CTGAAGTCCT TGGAAACCCT CAATCTGTCC 3300
   CAGAACAACA TCACTGACCT GGGTGCCTAC AAACTCGCCG AGGCCCTGCC TTCGCTCGCT 3360
   GCATCCCTGC TCAGGCTAAG CTTGTACAAT AACTGCATCT GCGACGTGGG AGCCGAGAGC 3420
   TTGGCTCGTG TGCTTCCGGA CATGGTGTCC CTCCGGGTGA TGGACGCAAG TTCACGGCTG 3480
   CCGGGGCCCA GCAGCTCGCT GCCAGCCTTC GGAGGTGTCC TCATGTGGAG ACGCTGGCGA 3540
   TGTGGACGCC CACCATCCCA TTCAGTGTCC AGGAACACCT GCAACAACAG GATTCACGGA 3600
   TCAGCCTGAG ATGATCCCAG CTGTGCTCTG GACAGGCATG TTCTCTGAGG ACACTAACCA 3660
   CGCTGGACCT TGAACTGGGT ACTTGTGGAC ACAGCTCTTC TCCAGGCTGT ATCCCATGAG 3720
   GCCTCAGCAT CCTGGCACCC GGCCCCTGCT GGTTCAGGGT TGGCCCCTGC CCGGCTGCGG 3780
   AATGAACCAC ATCTTGCTCT GCTGACAGAC ACAGGCCCGG CTCCAGGCTC CTTTAGCGCC 3840
   CAGTTGGGTG GATGCCTGGT GGCAGCTGCG GTCCACCCAG GAGCCCCGAG GCCTTCTCTG 3900
   AAGGACATTG CGGACAGCCA CGGCCAGGCC AGAGGGAGTG ACAGAGGCAG CCCCATTCTG 3960
   CCTGCCCAGG CCCCTGCCAC CCTGGGGAGA AAGTACTTCT TTTTTTTTAT TTTTAGACAG 4020
   AGTCTCACTG TTGCCCAGGC TGGCGTGCAG TGGTGCGATC TGGGTTCACT GCAACCTCCG 4080
   CCTCTTGGGT TCAAGCGATT CTTCTGCTTC AGCCTCCCGA GTAGCTGGGA CTACAGGCAC 4140
   CCACCATCAT GTCTGGCTAA TTTTTCATTT TTAGTAGAGA CAGGGTTTTG CCATGTTGGC 4200
   CAGGCTGGTC TCAAACTCTT GACCTCAGGT GATCCACCCA CCTCAGCCTC CCAAAGTGCT 4260
   GGGGATTACA AGCGTGAGCC ACTGCACCGG GCCACAGAGA AAGTACTTCT CCACCCTGCT 4320
   CTCCGACCAG ACACCTTGAC AGGGCACACC GGGCACTCAG AAGACACTGA TGGGCAACCC 4380
   CCAGCCTGCT AATTCCCCAG ATTGCAACAG GCTGGGCTTC AGTGGCAGGC TGCTTTTGTC 4440
   TATGGGACTC AATGCACTGA CATTGTTGGC CAAAGCCAAA GCTAGGCCTG GCCAGATGCA 4500
   CCAGGCCCTT AGCAGGGAAA CAGCTAATGG GACACTAATG GGGCGGTGAG AGGGGAACAG 4560
   ACTGGAAGCA CAGCTTCATT TCCTGTGTCT TTTTTCACTA CATTATAAAT GTCTCTTTAA 4620
   TGTCACAAAA AAAAAAAAAA AAAAAAAAA 4649
(2) INFORMATIONS POUR LA SEQ ID NO: 12:
   (i) CARACTERISTIQUES DE LA SEQUENCE:
      (A) LONGUEUR: 4346 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: cIIta de type II
   (xi) DESCRIPTION DE LA SEQUENCE: SEQ ID NO: 12:
(2) INFORMATIONS POUR LA SEQ ID NO: 13:
   (i) CARACTERISTIQUES DE LA SEQUENCE:
      (A) LONGUEUR: 4418 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: cIIta de type III
   (xi) DESCRIPTION DE LA SEQUENCE: SEQ ID NO: 13:
   ATGCGTTGCC TGGCTCCACG CCCTGCTGGG TCCTACCTGT CAGAGCCCCA AGGCAGCTCA 60
   CAGTGTGCCA CCATGGAGTT GGGGCCCCTA GAAGGTGGCT ACCTGGAGCT TCTTAACAGC 120
   GATGCTGACC CCCTGTGCCT CTACCACTTC TATGACCAGA TGGACCTGGC TGGAGAAGAA 180
   GAGATTGAGC TCTACTCAGA ACCCGACACA GACACCATCA ACTGCGACCA GTTCAGCAGG 240
   CTGTTGTGTG ACATGGAAGG TGATGAAGAG ACCAGGGAGG CTTATGCCAA TATCGCGGAA 300
   CTGGACCAGT ATGTCTTCCA GGACTCCCAG CTGGAGGGCC TGAGCAAGGA CATTTTCAAG 360
   CACATAGGAC CAGATGAAGT GATCGGTGAG AGTATGGAGA TGCCAGCAGA AGTTGGGCAG 420
   AAAAGTCAGA AAAGACCCTT CCCAGAGGAG CTTCCGGCAG ACCTGAAGCA CTGGAAGCCA 480
   GCTGAGCCCC CCACTGTGGT GACTGGCAGT CTCCTAGTGG GACCAGTGAG CGACTGCTCC 540
   ACCCTGCCCT GCCTGCCACT GCCTGCGCTG TTCAACCAGG AGCCAGCCTC CGGCCAGATG 600
   CGCCTGGAGA AAACCGACCA GATTCCCATG CCTTTCTCCA GTTCCTCGTT GAGCTGCCTG 660
   AATCTCCCTG AGGGACCCAT CCAGTTTGTC CCCACCATCT CCACTCTGCC CCATGGGCTC 720
   TGGCAAATCT CTGAGGCTGG AACAGGGGTC TCCAGTATAT TCATCTACCA TGGTGAGGTG 780
   CCCCAGGCCA GCCAAGTACC CCCTCCCAGT GGATTCACTG TCCACGGCCT CCCAACATCT 840
   CCAGACCGGC CAGGCTCCAC CAGCCCCTTC GCTCCATCAG CCACTGACCT GCCCAGCATG 900
   CCTGAACCTG CCCTGACCTC CCGAGCAAAC ATGACAGAGC ACAAGACGTC CCCCACCCAA 960
   TGCCCGGCAG CTGGAGAGGT CTCCAACAAG CTTCCAAAAT GGCCTGAGCC GGTGGAGCAG 1020
   TTCTACCGCT CACTGCAGGA CACGTATGGT GCCGAGCCCG CAGGCCCGGA TGGCATCCTA 1080
   GTGGAGGTGG ATCTGGTGCA GGCCAGGCTG GAGAGGAGCA GCAGCAAGAG CCTGGAGCGG 1140
   GAACTGGCCA CCCCGGACTG GGCAGAACGG CAGCTGGCCC AAGGAGGCCT GGCTGAGGTG 1200
   CTGTTGGCTG CCAAGGAGCA CCGGCGGCCG CGTGAGACAC GAGTGATTGC TGTGCTGGGC 1260
   AAAGCTGGTC AGGGCAAGAG CTATTGGGCT GGGGCAGTGA GCCGGGCCTG GGCTTGTGGC 1320
   CGGCTTCCCC AGTACGACTT TGTCTTCTCT GTCCCCTGCC ATTGCTTGAA CCGTCCGGGG 1380
   GATGCCTATG GCCTGCAGGA TCTGCTCTTC TCCCTGGGCC CACAGCCACT CGTGGCGGCC 1440
   GATGAGGTTT TCAGCCACAT CTTGAAGAGA CCTGACCGCG TTCTGCTCAT CCTAGACGCC 1500
   TTCGAGGAGC TGGAAGCGCA AGATGGCTTC CTGCACAGCA CGTGCGGACC GGCACCGGCG 1560
   GAGCCCTGCT CCCTCCGGGG GCTGCTGGCC GGCCTTTTCC AGAAGAAGCT GCTCCGAGGT 1620
   TGCACCCTCC TCCTCACAGC CCGGCCCCGG GGCCGCCTGG TCCAGAGCCT GAGCAAGGCC 1680
   GACGCCCTAT TTGAGCTGTC CGGCTTCTCC ATGGAGCAGG CCCAGGCATA CGTGATGCGC 1740
   TACTTTGAGA GCTCAGGGAT GACAGAGCAC CAAGACAGAG CCCTGACGCT CCTCCGGGAC 1800
   CGGCCACTTC TTCTCAGTCA CAGCCACAGC CCTACTTTGT GCCGGGCAGT GTGCCAGCTC 1860
   TCAGAGGCCC TGCTGGAGCT TGGGGAGGAC GCCAAGCTGC CCTCCACGCT CACGGGACTC 1920
   TATGTCGGCC TGCTGGGCCG TGCAGCCCTC GACAGCCCCC CCGGGGCCCT GGCAGAGCTG 1980
   GCCAAGCTGG CCTGGGAGCT GGGCCGCAGA CATCAAAGTA CCCTACAGGA GGACCAGTTC 2040
   CCATCCGCAG ACGTGAGGAC CTGGGCGATG GCCAAAGGCT TAGTCCAACA CCCACCGCGG 2100
   GCCGCAGAGT CCGAGCTGGC CTTCCCCAGC TTCCTCCTGC AATGCTTCCT GGGGGCCCTG 2160
   TGGCTGGCTC TGAGTGGCGA AATCAAGGAC AAGGAGCTCC CGCAGTACCT AGCATTGACC 2220
   CCAAGGAAGA AGAGGCCCTA TGACAACTGG CTGGAGGGCG TGCCACGCTT TCTGGCTGGG 2280
   CTGATCTTCC AGCCTCCCGC CCGCTGCCTG GGAGCCCTAC TCGGGCCATC GGCGGCTGCC 2340
   TCGGTGGACA GGAAGCAGAA GGTGCTTGCG AGGTACCTGA AGCGGCTGCA GCCGGGGACA 2400
   CTGCGGGCGC GGCAGCTGCT TGAGCTGCTG CACTGCGCCC ACGAGGCCGA GGAGGCTGGA 2460
   ATTTGGCAGC ACGTGGTACA GGAGCTCCCC GGCCGCCTCT CTTTTCTGGG CACCCGCCTC 2520
   ACGCCTCCTG ATGCACATGT ACTGGGCAAG GCCTTGGAGG CGGCGGGCCA AGACTTCTCC 2580
   CTGGACCTCC GCAGCACTGG CATTTGCCCC TCTGGATTGG GGAGCCTCGT GGGACTCAGC 2640
   TGTGTCACCC GTTTCAGGGC TGCCTTGAGC GACACGGTGG CGCTGTGGGA GTCCCTGCGG 2700
   CAGCATGGGG AGACCAAGCT ACTTCAGGCA GCAGAGGAGA AGTTCACCAT CGAGCCTTTC 2760
   AAAGCCAAGT CCCTGAAGGA TGTGGAAGAC CTGGGAAAGC TTGTGCAGAC TCAGAGGACG 2820
   AGAAGTTCCT CGGAAGACAC AGCTGGGGAG CTCCCTGCTG TTCGGGACCT AAAGAAACTG 2880
   GAGTTTGCGC TGGGCCCTGT CTCAGGCCCC CAGGCTTTCC CCAAACTGGT GCGGATCCTC 2940
   ACGGCCTTTT CCTCCCTGCA GCATCTGGAC CTGGATGCGC TGAGTGAGAA CAAGATCGGG 3000
   GACGAGGGTG TCTCGCAGCT CTCAGCCACC TTCCCCCAGC TGAAGTCCTT GGAAACCCTC 3060
   AATCTGTCCC AGAACAACAT CACTGACCTG GGTGCCTACA AACTCGCCGA GGCCCTGCCT 3120
   TCGCTCGCTG CATCCCTGCT CAGGCTAAGC TTGTACAATA ACTGCATCTG CGACGTGGGA 3180
   GCCGAGAGCT TGGCTCGTGT GCTTCCGGAC ATGGTGTCCC TCCGGGTGAT GGACGCAAGT 3240
   TCACGGCTGC CGGGGCCCAG CAGCTCGCTG CCAGCCTTCG GAGGTGTCCT CATGTGGAGA 3300
   CGCTGGCGAT GTGGACGCCC ACCATCCCAT TCAGTGTCCA GGAACACCTG CAACAACAGG 3360
   ATTCACGGAT CAGCCTGAGA TGATCCCAGC TGTGCTCTGG ACAGGCATGT TCTCTGAGGA 3420
   CACTAACCAC GCTGGACCTT GAACTGGGTA CTTGTGGACA CAGCTCTTCT CCAGGCTGTA 3480
   TCCCATGAGG CCTCAGCATC CTGGCACCCG GCCCCTGCTG GTTCAGGGTT GGCCCCTGCC 3540
   CGGCTGCGGA ATGAACCACA TCTTGCTCTG CTGACAGACA CAGGCCCGGC TCCAGGCTCC 3600
   TTTAGCGCCC AGTTGGGTGG ATGCCTGGTG GCAGCTGCGG TCCACCCAGG AGCCCCGAGG 3660
   CCTTCTCTGA AGGACATTGC GGACAGCCAC GGCCAGGCCA GAGGGAGTGA CAGAGGCAGC 3720
   CCCATTCTGC CTGCCCAGGC CCCTGCCACC CTGGGGAGAA AGTACTTCTT TTTTTTTATT 3780
   TTTAGACAGA GTCTCACTGT TGCCCAGGCT GGCGTGCAGT GGTGCGATCT GGGTTCACTG 3840
   CAACCTCCGC CTCTTGGGTT CAAGCGATTC TTCTGCTTCA GCCTCCCGAG TAGCTGGGAC 3900
   TACAGGCACC CACCATCATG TCTGGCTAAT TTTTCATTTT TAGTAGAGAC AGGGTTTTGC 3960
   CATGTTGGCC AGGCTGGTCT CAAACTCTTG ACCTCAGGTG ATCCACCCAC CTCAGCCTCC 4020
   CAAAGTGCTG GGGATTACAA GCGTGAGCCA CTGCACCGGG CCACAGAGAA AGTACTTCTC 4080
   CACCCTGCTC TCCGACCAGA CACCTTGACA GGGCACACCG GGCACTCAGA AGACACTGAT 4140
   GGGCAACCCC CAGCCTGCTA ATTCCCCAGA TTGCAACAGG CTGGGCTTCA GTGGCAGGCT 4200
   GCTTTTGTCT ATGGGACTCA ATGCACTGAC ATTGTTGGCC AAAGCCAAAG CTAGGCCTGG 4260
   CCAGATGCAC CAGGCCCTTA GCAGGGAAAC AGCTAATGGG ACACTAATGG GGCGGTGAGA 4320
   GGGGAACAGA CTGGAAGCAC AGCTTCATTT CCTGTGTCTT TTTTCACTAC ATTATAAATG 4380
   TCTCTTTAAT GTCACAAAAA AAAAAAAAAA AAAAAAAA 4418
(2) INFORMATIONS POUR LA SEQ ID NO: 14:
   (i) CARACTERISTIQUES DE LA SEQUENCE:
      (A) LONGUEUR: 4366 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: cIIta de type IV
   (xi) DESCRIPTION DE LA SEQUENCE. SEQ ID NO: 14:
   GCAGCTCACA GTGTGCCACC ATGGAGTTGG GGCCCCTAGA AGGTGGCTAC CTGGAGCTTC 60
   TTAACAGCGA TGCTGACCCC CTGTGCCTCT ACCACTTCTA TGACCAGATG GACCTGGCTG 120
   GAGAAGAAGA GATTGAGCTC TACTCAGAAC CCGACACAGA CACCATCAAC TGCGACCAGT 180
   TCAGCAGGCT GTTGTGTGAC ATGGAAGGTG ATGAAGAGAC CAGGGAGGCT TATGCCAATA 240
   TCGCGGAACT GGACCAGTAT GTCTTCCAGG ACTCCCAGCT GGAGGGCCTG AGCAAGGACA 300
   TTTTCAAGCA CATAGGACCA GATGAAGTGA TCGGTGAGAG TATGGAGATG CCAGCAGAAG 360
   TTGGGCAGAA AAGTCAGAAA AGACCCTTCC CAGAGGAGCT TCCGGCAGAC CTGAAGCACT 420
   GGAAGCCAGC TGAGCCCCCC ACTGTGGTGA CTGGCAGTCT CCTAGTGGGA CCAGTGAGCG 480
   ACTGCTCCAC CCTGCCCTGC CTGCCACTGC CTGCGCTGTT CAACCAGGAG CCAGCCTCCG 540
   GCCAGATGCG CCTGGAGAAA ACCGACCAGA TTCCCATGCC TTTCTCCAGT TCCTCGTTGA 600
   GCTGCCTGAA TCTCCCTGAG GGACCCATCC AGTTTGTCCC CACCATCTCC ACTCTGCCCC 660
   ATGGGCTCTG GCAAATCTCT GAGGCTGGAA CAGGGGTCTC CAGTATATTC ATCTACCATG 720
   GTGAGGTGCC CCAGGCCAGC CAAGTACCCC CTCCCAGTGG ATTCACTGTC CACGGCCTCC 780
   CAACATCTCC AGACCGGCCA GGCTCCACCA GCCCCTTCGC TCCATCAGCC ACTGACCTGC 840
   CCAGCATGCC TGAACCTGCC CTGACCTCCC GAGCAAACAT GACAGAGCAC AAGACGTCCC 900
   CCACCCAATG CCCGGCAGCT GGAGAGGTCT CCAACAAGCT TCCAAAATGG CCTGAGCCGG 960
   TGGAGCAGTT CTACCGCTCA CTGCAGGACA CGTATGGTGC CGAGCCCGCA GGCCCGGATG 1020
   GCATCCTAGT GGAGGTGGAT CTGGTGCAGG CCAGGCTGGA GAGGAGCAGC AGCAAGAGCC 1080
   TGGAGCGGGA ACTGGCCACC CCGGACTGGG CAGAACGGCA GCTGGCCCAA GGAGGCCTGG 1140
   CTGAGGTGCT GTTGGCTGCC AAGGAGCACC GGCGGCCGCG TGAGACACGA GTGATTGCTG 1200
   TGCTGGGCAA AGCTGGTCAG GGCAAGAGCT ATTGGGCTGG GGCAGTGAGC CGGGCCTGGG 1260
   CTTGTGGCCG GCTTCCCCAG TACGACTTTG TCTTCTCTGT CCCCTGCCAT TGCTTGAACC 1320
   GTCCGGGGGA TGCCTATGGC CTGCAGGATC TGCTCTTCTC CCTGGGCCCA CAGCCACTCG 1380
   TGGCGGCCGA TGAGGTTTTC AGCCACATCT TGAAGAGACC TGACCGCGTT CTGCTCATCC 1440
   TAGACGCCTT CGAGGAGCTG GAAGCGCAAG ATGGCTTCCT GCACAGCACG TGCGGACCGG 1500
   CACCGGCGGA GCCCTGCTCC CTCCGGGGGC TGCTGGCCGG CCTTTTCCAG AAGAAGCTGC 1560
   TCCGAGGTTG CACCCTCCTC CTCACAGCCC GGCCCCGGGG CCGCCTGGTC CAGAGCCTGA 1620
   GCAAGGCCGA CGCCCTATTT GAGCTGTCCG GCTTCTCCAT GGAGCAGGCC CAGGCATACG 1680
   TGATGCGCTA CTTTGAGAGC TCAGGGATGA CAGAGCACCA AGACAGAGCC CTGACGCTCC 1740
   TCCGGGACCG GCCACTTCTT CTCAGTCACA GCCACAGCCC TACTTTGTGC CGGGCAGTGT 1800
   GCCAGCTCTC AGAGGCCCTG CTGGAGCTTG GGGAGGACGC CAAGCTGCCC TCCACGCTCA 1860
   CGGGACTCTA TGTCGGCCTG CTGGGCCGTG CAGCCCTCGA CAGCCCCCCC GGGGCCCTGG 1920
   CAGAGCTGGC CAAGCTGGCC TGGGAGCTGG GCCGCAGACA TCAAAGTACC CTACAGGAGG 1980
   ACCAGTTCCC ATCCGCAGAC GTGAGGACCT GGGCGATGGC CAAAGGCTTA GTCCAACACC 2040
   CACCGCGGGC CGCAGAGTCC GAGCTGGCCT TCCCCAGCTT CCTCCTGCAA TGCTTCCTGG 2100
   GGGCCCTGTG GCTGGCTCTG AGTGGCGAAA TCAAGGACAA GGAGCTCCCG CAGTACCTAG 2160
   CATTGACCCC AAGGAAGAAG AGGCCCTATG ACAACTGGCT GGAGGGCGTG CCACGCTTTC 2220
   TGGCTGGGCT GATCTTCCAG CCTCCCGCCC GCTGCCTGGG AGCCCTACTC GGGCCATCGG 2280
   CGGCTGCCTC GGTGGACAGG AAGCAGAAGG TGCTTGCGAG GTACCTGAAG CGGCTGCAGC 2340
   CGGGGACACT GCGGGCGCGG CAGCTGCTTG AGCTGCTGCA CTGCGCCCAC GAGGCCGAGG 2400
   AGGCTGGAAT TTGGCAGCAC GTGGTACAGG AGCTCCCCGG CCGCCTCTCT TTTCTGGGCA 2460
   CCCGCCTCAC GCCTCCTGAT GCACATGTAC TGGGCAAGGC CTTGGAGGCG GCGGGCCAAG 2520
   ACTTCTCCCT GGACCTCCGC AGCACTGGCA TTTGCCCCTC TGGATTGGGG AGCCTCGTGG 2580
   GACTCAGCTG TGTCACCCGT TTCAGGGCTG CCTTGAGCGA CACGGTGGCG CTGTGGGAGT 2640
   CCCTGCGGCA GCATGGGGAG ACCAAGCTAC TTCAGGCAGC AGAGGAGAAG TTCACCATCG 2700
   AGCCTTTCAA AGCCAAGTCC CTGAAGGATG TGGAAGACCT GGGAAAGCTT GTGCAGACTC 2760
   AGAGGACGAG AAGTTCCTCG GAAGACACAG CTGGGGAGCT CCCTGCTGTT CGGGACCTAA 2820
   AGAAACTGGA GTTTGCGCTG GGCCCTGTCT CAGGCCCCCA GGCTTTCCCC AAACTGGTGC 2880
   GGATCCTCAC GGCCTTTTCC TCCCTGCAGC ATCTGGACCT GGATGCGCTG AGTGAGAACA 2940
   AGATCGGGGA CGAGGGTGTC TCGCAGCTCT CAGCCACCTT CCCCCAGCTG AAGTCCTTGG 3000
   AAACCCTCAA TCTGTCCCAG AACAACATCA CTGACCTGGG TGCCTACAAA CTCGCCGAGG 3060
   CCCTGCCTTC GCTCGCTGCA TCCCTGCTCA GGCTAAGCTT GTACAATAAC TGCATCTGCG 3120
   ACGTGGGAGC CGAGAGCTTG GCTCGTGTGC TTCCGGACAT GGTGTCCCTC CGGGTGATGG 3180
   ACGCAAGTTC ACGGCTGCCG GGGCCCAGCA GCTCGCTGCC AGCCTTCGGA GGTGTCCTCA 3240
   TGTGGAGACG CTGGCGATGT GGACGCCCAC CATCCCATTC AGTGTCCAGG AACACCTGCA 3300
   ACAACAGGAT TCACGGATCA GCCTGAGATG ATCCCAGCTG TGCTCTGGAC AGGCATGTTC 3360
   TCTGAGGACA CTAACCACGC TGGACCTTGA ACTGGGTACT TGTGGACACA GCTCTTCTCC 3420
   AGGCTGTATC CCATGAGGCC TCAGCATCCT GGCACCCGGC CCCTGCTGGT TCAGGGTTGG 3480
   CCCCTGCCCG GCTGCGGAAT GAACCACATC TTGCTCTGCT GACAGACACA GGCCCGGCTC 3540
   CAGGCTCCTT TAGCGCCCAG TTGGGTGGAT GCCTGGTGGC AGCTGCGGTC CACCCAGGAG 3600
   CCCCGAGGCC TTCTCTGAAG GACATTGCGG ACAGCCACGG CCAGGCCAGA GGGAGTGACA 3660
   GAGGCAGCCC CATTCTGCCT GCCCAGGCCC CTGCCACCCT GGGGAGAAAG TACTTCTTTT 3720
   TTTTTATTTT TAGACAGAGT CTCACTGTTG CCCAGGCTGG CGTGCAGTGG TGCGATCTGG 3780
   GTTCACTGCA ACCTCCGCCT CTTGGGTTCA AGCGATTCTT CTGCTTCAGC CTCCCGAGTA 3840
   GCTGGGACTA CAGGCACCCA CCATCATGTC TGGCTAATTT TTCATTTTTA GTAGAGACAG 3900
   GGTTTTGCCA TGTTGGCCAG GCTGGTCTCA AACTCTTGAC CTCAGGTGAT CCACCCACCT 3960
   CAGCCTCCCA AAGTGCTGGG GATTACAAGC GTGAGCCACT GCACCGGGCC ACAGAGAAAG 4020
   TACTTCTCCA CCCTGCTCTC CGACCAGACA CCTTGACAGG GCACACCGGG CACTCAGAAG 4080
   ACACTGATGG GCAACCCCCA GCCTGCTAAT TCCCCAGATT GCAACAGGCT GGGCTTCAGT 4140
   GGCAGGCTGC TTTTGTCTAT GGGACTCAAT GCACTGACAT TGTTGGCCAA AGCCAAAGCT 4200
   AGGCCTGGCC AGATGCACCA GGCCCTTAGC AGGGAAACAG CTAATGGGAC ACTAATGGGG 4260
   CGGTGAGAGG GGAACAGACT GGAAGCACAG CTTCATTTCC TGTGTCTTTT TTCACTACAT 4320
   TATAAATGTC TCTTTAATGT CACAAAAAAA AAAAAAAAAA AAAAAA 4366
(2) INFORMATIONS POUR LA SEQ ID NO: 15:
   (i) CARACTERISTIQUES DE LA SEQUENCE:
      (A) LONGUEUR: 2480 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) ORIGINE:
      (A) ORGANISME: 901-3390
   (xi) DESCRIPTION DE LA SEQUENCE: SEQ ID NO: 15:
   CCTGAACCTG CCCTGACCTC CCGAGCAAAC ATGACAGAGC ACAAGACGTC CCCCACCCAA 60
   TGCCCGGCAG CTGGAGAGGT CTCCAACAAG CTTCCAAAAT GGCCTGAGCC GGTGGAGCAG 120
   TTCTACCGCT CACTGCAGGA CACGTATGGT GCCGAGCCCG CAGGCCCGGA TGGCATCCTA 180
   GTGGAGGTGG ATCTGGTGCA GGCCAGGCTG GAGAGGAGCA GCAGCAAGAG CCTGGAGCGG 240
   GAACTGGCCA CCCCGGACTG GGCAGAACGG CAGCTGGCCC AAGGAGGCCT GGCTGAGGTG 300
   CTGTTGGCTG CCAAGGAGCA CCGGCGGCCG CGTGAGACAC GAGTGATTGC TGTGCTGGGC 360
   AAAGCTGGTC AGGGCAAGAG CTATTGGGCT GGGGCAGTGA GCCGGGCCTG GGCTTGTGGC 420
   CGGCTTCCCC AGTACGACTT TGTCTTCTCT GTCCCCTGCC ATTGCTTGAA CCGTCCGGGG 480
   GATGCCTATG GCCTGCAGGA TCTGCTCTTC TCCCTGGGCC CACAGCCACT CGTGGCGGCC 540
   GATGAGGTTT TCAGCCACAT CTTGAAGAGA CCTGACCGCG TTCTGCTCAT CCTAGACGCC 600
   TTCGAGGAGC TGGAAGCGCA AGATGGCTTC CTGCACAGCA CGTGCGGACC GGCACCGGCG 660
   GAGCCCTGCT CCCTCCGGGG GCTGCTGGCC GGCCTTTTCC AGAAGAAGCT GCTCCGAGGT 720
   TGCACCCTCC TCCTCACAGC CCGGCCCCGG GGCCGCCTGG TCCAGAGCCT GAGCAAGGCC 780
   GACGCCCTAT TTGAGCTGTC CGGCTTCTCC ATGGAGCAGG CCCAGGCATA CGTGATGCGC 840
   TACTTTGAGA GCTCAGGGAT GACAGAGCAC CAAGACAGAG CCCTGACGCT CCTCCGGGAC 900
   CGGCCACTTC TTCTCAGTCA CAGCCACAGC CCTACTTTGT GCCGGGCAGT GTGCCAGCTC 960
   TCAGAGGCCC TGCTGGAGCT TGGGGAGGAC GCCAAGCTGC CCTCCACGCT CACGGGACTC 1020
   TATGTCGGCC TGCTGGGCCG TGCAGCCCTC GACAGCCCCC CCGGGGCCCT GGCAGAGCTG 1080
   GCCAAGCTGG CCTGGGAGCT GGGCCGCAGA CATCAAAGTA CCCTACAGGA GGACCAGTTC 1140
   CCATCCGCAG ACGTGAGGAC CTGGGCGATG GCCAAAGGCT TAGTCCAACA CCCACCGCGG 1200
   GCCGCAGAGT CCGAGCTGGC CTTCCCCAGC TTCCTCCTGC AATGCTTCCT GGGGGCCCTG 1260
   TGGCTGGCTC TGAGTGGCGA AATCAAGGAC AAGGAGCTCC CGCAGTACCT AGCATTGACC 1320
   CCAAGGAAGA AGAGGCCCTA TGACAACTGG CTGGAGGGCG TGCCACGCTT TCTGGCTGGG 1380
   CTGATCTTCC AGCCTCCCGC CCGCTGCCTG GGAGCCCTAC TCGGGCCATC GGCGGCTGCC 1440
   TCGGTGGACA GGAAGCAGAA GGTGCTTGCG AGGTACCTGA AGCGGCTGCA GCCGGGGACA 1500
   CTGCGGGCGC GGCAGCTGCT TGAGCTGCTG CACTGCGCCC ACGAGGCCGA GGAGGCTGGA 1560
   ATTTGGCAGC ACGTGGTACA GGAGCTCCCC GGCCGCCTCT CTTTTCTGGG CACCCGCCTC 1620
   ACGCCTCCTG ATGCACATGT ACTGGGCAAG GCCTTGGAGG CGGCGGGCCA AGACTTCTCC 1680
   CTGGACCTCC GCAGCACTGG CATTTGCCCC TCTGGATTGG GGAGCCTCGT GGGACTCAGC 1740
   TGTGTCACCC GTTTCAGGGC TGCCTTGAGC GACACGGTGG CGCTGTGGGA GTCCCTGCGG 1800
   CAGCATGGGG AGACCAAGCT ACTTCAGGCA GCAGAGGAGA AGTTCACCAT CGAGCCTTTC 1860
   AAAGCCAAGT CCCTGAAGGA TGTGGAAGAC CTGGGAAAGC TTGTGCAGAC TCAGAGGACG 1920
   AGAAGTTCCT CGGAAGACAC AGCTGGGGAG CTCCCTGCTG TTCGGGACCT AAAGAAACTG 1980
   GAGTTTGCGC TGGGCCCTGT CTCAGGCCCC CAGGCTTTCC CCAAACTGGT GCGGATCCTC 2040
   ACGGCCTTTT CCTCCCTGCA GCATCTGGAC CTGGATGCGC TGAGTGAGAA CAAGATCGGG 2100
   GACGAGGGTG TCTCGCAGCT CTCAGCCACC TTCCCCCAGC TGAAGTCCTT GGAAACCCTC 2160
   AATCTGTCCC AGAACAACAT CACTGACCTG GGTGCCTACA AACTCGCCGA GGCCCTGCCT 2220
   TCGCTCGCTG CATCCCTGCT CAGGCTAAGC TTGTACAATA ACTGCATCTG CGACGTGGGA 2280
   GCCGAGAGCT TGGCTCGTGT GCTTCCGGAC ATGGTGTCCC TCCGGGTGAT GGACGCAAGT 2340
   TCACGGCTGC CGGGGCCCAG CAGCTCGCTG CCAGCCTTCG GAGGTGTCCT CATGTGGAGA 2400
   CGCTGGCGAT GTGGACGCCC ACCATCCCAT TCAGTGTCCA GGAACACCTG CAACAACAGG 2460
   ATTCACGGAT CAGCCTGAGA 2480
(2) INFORMATIONS POUR LA SEQ ID NO: 16:
   (i) CARACTERISTIQUES DE LA SEQUENCE:
      (A) LONGUEUR: 1207 acides aminés
      (B) TYPE: peptide
      (D) CONFIGURATION: linéaire
   (ii) TYPE DE MOLECULE: peptide
   (ix) CARACTERISTIQUE:
      (A) NOM/CLE: cIIta de type I
   (xi) DESCRIPTION DE LA SEQUENCE: SEQ ID NO: 16:
   Met Asn Asn Phe Gln Ala Ile Leu Thr Gln Val Arg Met Leu Leu Ser
   1 5 10 15
   Ser His Gln Pro Ser Leu Val Gln Ala Leu Leu Asp Asn Leu Leu Lys
   20 25 30
   Glu Asp Leu Leu Ser Arg Glu Tyr His Cys Thr Leu Leu His Glu Pro
   35 40 45
   Asp Ser Glu Ala Leu Ala Arg Lys Ile Ser Leu Thr Leu Leu Glu Lys
   50 55 60
   Gly Asp Leu Asp Leu Ala Leu Leu Gly Trp Ala Arg Ser Gly Leu Gln
   65 70 75 80
   Pro Pro Ala Ala Glu Arg Gly Pro Gly His Ser Asp His Gly Gly Ser
   85 90 95
   Ser Gln Cys Ala Thr Met Glu Leu Gly Pro Leu Glu Gly Gly Tyr Leu
   100 105 110
   Glu Leu Leu Asn Ser Asp Ala Asp Pro Leu Cys Leu Tyr His Phe Tyr
   115 120 125
   Asp Gln Met Asp Leu Ala Gly Glu Glu Glu Ile Glu Leu Tyr Ser Glu
   130 135 140
   Pro Asp Thr Asp Thr Ile Asn Cys Asp Gln Phe Ser Arg Leu Leu Cys
   145                 150                 155                 160
   Asp Met Glu Gly Asp Glu Glu Thr Arg Glu Ala Tyr Ala Asn Ile Ala
                   165                 170                 175
   Glu Leu Asp Gln Tyr Val Phe Gln Asp Ser Gln Leu Glu Gly Leu Ser
               180                 185                 190
   Lys Asp Ile Phe Lys His Ile Gly Pro Asp Glu Val Ile Gly Glu Ser
           195                 200                 205
   Met Glu Met Pro Ala Glu Val Gly Gln Lys Ser Gln Lys Arg Pro Phe
       210                 215                 220
   Pro Glu Glu Leu Pro Ala Asp Leu Lys His Trp Lys Pro Ala Glu Pro
   225                 230                 235                 240
   Pro Thr Val Val Thr Gly Ser Leu Leu Val Gly Pro Val Ser Asp Cys
                   245                 250                 255
   Ser Thr Leu Pro Cys Leu Pro Leu Pro Ala Leu Phe Asn Gln Glu Pro
               260                 265                 270
   Ala Ser Gly Gln Met Arg Leu Glu Lys Thr Asp Gln Ile Pro Met Pro
           275                 280                 285
   Phe Ser Ser Ser Ser Leu Ser Cys Leu Asn Leu Pro Glu Gly Pro Ile
       290                 295                 300
   Gln Phe Val Pro Thr Ile Ser Thr Leu Pro His Gly Leu Trp Gln Ile
   305                 310                 315                 320
   Ser Glu Ala Gly Thr Gly Val Ser Ser Ile Phe Ile Tyr His Gly Glu
                   325                 330                 335
   Val Pro Gln Ala Ser Gln Val Pro Pro Pro Ser Gly Phe Thr Val His
               340                 345                 350
   Gly Leu Pro Thr Ser Pro Asp Arg Pro Gly Ser Thr Ser Pro Phe Ala
           355                 360                 365
   Pro Ser Ala Thr Asp Leu Pro Ser Met Pro Glu Pro Ala Leu Thr Ser
       370                 375                 380
   Arg Ala Asn Met Thr Glu His Lys Thr Ser Pro Thr Gln Cys Pro Ala
   385                 390                 395                 400
   Ala Gly Glu Val Ser Asn Lys Leu Pro Lys Trp Pro Glu Pro Val Glu
                   405                 410                 415
   Gln Phe Tyr Arg Ser Leu Gln Asp Thr Tyr Gly Ala Glu Pro Ala Gly
               420                 425                 430
   Pro Asp Gly Ile Leu Val Glu Val Asp Leu Val Gln Ala Arg Leu Glu
   435 440 445
   Arg Ser Ser Ser Lys Ser Leu Glu Arg Glu Leu Ala Thr Pro Asp Trp
   450 455 460
   Ala Glu Arg Gln Leu Ala Gln Gly Gly Leu Ala Glu Val Leu Leu Ala
   465 470 475 480
   Ala Lys Glu His Arg Arg Pro Arg Glu Thr Arg Val Ile Ala Val Leu
   485 490 495
   Gly Lys Ala Gly Gln Gly Lys Ser Tyr Trp Ala Gly Ala Val Ser Arg
   500 505 510
   Ala Trp Ala Cys Gly Arg Leu Pro Gln Tyr Asp Phe Val Phe Ser Val
   515 520 525
   Pro Cys His Cys Leu Asn Arg Pro Gly Asp Ala Tyr Gly Leu Gln Asp
   530 535 540
   Leu Leu Phe Ser Leu Gly Pro Gln Pro Leu Val Ala Ala Asp Glu Val
   545 550 555 560
   Phe Ser His Ile Leu Lys Arg Pro Asp Arg Val Leu Leu Ile Leu Asp
   565 570 575
   Ala Phe Glu Glu Leu Glu Ala Gln Asp Gly Phe Leu His Ser Thr Cys
   580 585 590
   Gly Pro Ala Pro Ala Glu Pro Cys Ser Leu Arg Gly Leu Leu Ala Gly
   595 600 605
   Leu Phe Gln Lys Lys Leu Leu Arg Gly Cys Thr Leu Leu Leu Thr Ala
   610 615 620
   Arg Pro Arg Gly Arg Leu Val Gln Ser Leu Ser Lys Ala Asp Ala Leu
   625 630 635 640
   Phe Glu Leu Ser Gly Phe Ser Met Glu Gln Ala Gln Ala Tyr Val Met
   645 650 655
   Arg Tyr Phe Glu Ser Ser Gly Met Thr Glu His Gln Asp Arg Ala Leu
   660 665 670
   Thr Leu Leu Arg Asp Arg Pro Leu Leu Leu Ser His Ser His Ser Pro
   675 680 685
   Thr Leu Cys Arg Ala Val Cys Gln Leu Ser Glu Ala Leu Leu Glu Leu
   690 695 700
   Gly Glu Asp Ala Lys Leu Pro Ser Thr Leu Thr Gly Leu Tyr Val Gly
   705 710 715 720
   Leu Leu Gly Arg Ala Ala Leu Asp Ser Pro Pro Gly Ala Leu Ala Glu
                   725                 730                 735
   Leu Ala Lys Leu Ala Trp Glu Leu Gly Arg Arg His Gln Ser Thr Leu
               740                 745                 750
   Gln Glu Asp Gln Phe Pro Ser Ala Asp Val Arg Thr Trp Ala Met Ala
           755                 760                 765
   Lys Gly Leu Val Gln His Pro Pro Arg Ala Ala Glu Ser Glu Leu Ala
       770                 775                 780
   Phe Pro Ser Phe Leu Leu Gln Cys Phe Leu Gly Ala Leu Trp Leu Ala
   785                 790                 795                 800
   Leu Ser Gly Glu Ile Lys Asp Lys Glu Leu Pro Gln Tyr Leu Ala Leu
                   805                 810                 815
   Thr Pro Arg Lys Lys Arg Pro Tyr Asp Asn Trp Leu Glu Gly Val Pro
               820                 825                 830
   Arg Phe Leu Ala Gly Leu Ile Phe Gln Pro Pro Ala Arg Cys Leu Gly
           835                 840                 845
   Ala Leu Leu Gly Pro Ser Ala Ala Ala Ser Val Asp Arg Lys Gln Lys
       850                 855                 860
   Val Leu Ala Arg Tyr Leu Lys Arg Leu Gln Pro Gly Thr Leu Arg Ala
   865                 870                 875                 880
   Arg Gln Leu Leu Glu Leu Leu His Cys Ala His Glu Ala Glu Glu Ala
                   885                 890                 895
   Gly Ile Trp Gln His Val Val Gln Glu Leu Pro Gly Arg Leu Ser Phe
               900                 905                 910
   Leu Gly Thr Arg Leu Thr Pro Pro Asp Ala His Val Leu Gly Lys Ala
           915                 920                 925
   Leu Glu Ala Ala Gly Gln Asp Phe Ser Leu Asp Leu Arg Ser Thr Gly
       930                 935                 940
   Ile Cys Pro Ser Gly Leu Gly Ser Leu Val Gly Leu Ser Cys Val Thr
   945                 950                 955                 960
   Arg Phe Arg Ala Ala Leu Ser Asp Thr Val Ala Leu Trp Glu Ser Leu
                   965                 970                 975
   Arg Gln His Gly Glu Thr Lys Leu Leu Gln Ala Ala Glu Glu Lys Phe
               980                 985                 990
   Thr Ile Glu Pro Phe Lys Ala Lys Ser Leu Lys Asp Val Glu Asp Leu
           995                 1000                1005
   Gly Lys Leu Val Gln Thr Gln Arg Thr Arg Ser Ser Ser Glu Asp Thr
       1010                1015                1020
   Ala Gly Glu Leu Pro Ala Val Arg Asp Leu Lys Lys Leu Glu Phe Ala
   1025                1030                1035                1040
   Leu Gly Pro Val Ser Gly Pro Gln Ala Phe Pro Lys Leu Val Arg Ile
                   1045                1050                1055
   Leu Thr Ala Phe Ser Ser Leu Gln His Leu Asp Leu Asp Ala Leu Ser
               1060                1065                1070
   Glu Asn Lys Ile Gly Asp Glu Gly Val Ser Gln Leu Ser Ala Thr Phe
           1075                1080                1085
   Pro Gln Leu Lys Ser Leu Glu Thr Leu Asn Leu Ser Gln Asn Asn Ile
       1090                1095                1100
   Thr Asp Leu Gly Ala Tyr Lys Leu Ala Glu Ala Leu Pro Ser Leu Ala
   1105                1110                1115                1120
   Ala Ser Leu Leu Arg Leu Ser Leu Tyr Asn Asn Cys Ile Cys Asp Val
                   1125                1130                1135
   Gly Ala Glu Ser Leu Ala Arg Val Leu Pro Asp Met Val Ser Leu Arg
               1140                1145                1150
   Val Met Asp Val Gln Tyr Asn Lys Phe Thr Ala Ala Gly Ala Gln Gln
           1155                1160                1165
   Leu Ala Ala Ser Leu Arg Arg Cys Pro His Val Glu Thr Leu Ala Met
       1170                1175                1180
   Trp Thr Pro Thr Ile Pro Phe Ser Val Gln Glu His Leu Gln Gln Gln
   1185                1190                1195                1200
   Asp Ser Arg Ile Ser Leu Arg
                   1205
(2) INFORMATIONS POUR LA SEQ ID NO: 17:
   (i) CARACTERISTIQUES DE LA SEQUENCE:
      (A) LONGUEUR: 1106 acides aminés
      (B) TYPE: peptide
      (D) CONFIGURATION: linéaire
   (ii) TYPE DE MOLECULE: peptide
   (ix) CARACTERISTIQUE:
      (A) NOM/CLE: cIIta de type I
   (xi) DESCRIPTION DE LA SEQUENCE: SEQ ID NO: 17:
   Met Glu Leu Gly Pro Leu Glu Gly Gly Tyr Leu Glu Leu Leu Asn Ser
   1               5                   10                  15
   Asp Ala Asp Pro Leu Cys Leu Tyr His Phe Tyr Asp Gln Met Asp Leu
               20                  25                  30
   Ala Gly Glu Glu Glu Ile Glu Leu Tyr Ser Glu Pro Asp Thr Asp Thr
           35                  40                  45
   Ile Asn Cys Asp Gln Phe Ser Arg Leu Leu Cys Asp Met Glu Gly Asp
       50                  55                  60
   Glu Glu Thr Arg Glu Ala Tyr Ala Asn Ile Ala Glu Leu Asp Gln Tyr
   65                  70                  75                  80
   Val Phe Gln Asp Ser Gln Leu Glu Gly Leu Ser Lys Asp Ile Phe Lys
                   85                  90                  95
   His Ile Gly Pro Asp Glu Val Ile Gly Glu Ser Met Glu Met Pro Ala
               100                 105                 110
   Glu Val Gly Gln Lys Ser Gln Lys Arg Pro Phe Pro Glu Glu Leu Pro
           115                 120                 125
   Ala Asp Leu Lys His Trp Lys Pro Ala Glu Pro Pro Thr Val Val Thr
       130                 135                 140
   Gly Ser Leu Leu Val Gly Pro Val Ser Asp Cys Ser Thr Leu Pro Cys
   145                 150                 155                 160
   Leu Pro Leu Pro Ala Leu Phe Asn Gln Glu Pro Ala Ser Gly Gln Met
                   165                 170                 175
   Arg Leu Glu Lys Thr Asp Gln Ile Pro Met Pro Phe Ser Ser Ser Ser
               180                 185                 190
   Leu Ser Cys Leu Asn Leu Pro Glu Gly Pro Ile Gln Phe Val Pro Thr
           195                 200                 205
   Ile Ser Thr Leu Pro His Gly Leu Trp Gln Ile Ser Glu Ala Gly Thr
       210                 215                 220
   Gly Val Ser Ser Ile Phe Ile Tyr His Gly Glu Val Pro Gln Ala Ser
   225                 230                 235                 240
   Gln Val Pro Pro Pro Ser Gly Phe Thr Val His Gly Leu Pro Thr Ser
                   245                 250                 255
   Pro Asp Arg Pro Gly Ser Thr Ser Pro Phe Ala Pro Ser Ala Thr Asp
               260                 265                 270
   Leu Pro Ser Met Pro Glu Pro Ala Leu Thr Ser Arg Ala Asn Met Thr
           275                 280                 285
   Glu His Lys Thr Ser Pro Thr Gln Cys Pro Ala Ala Gly Glu Val Ser
       290                 295                 300
   Asn Lys Leu Pro Lys Trp Pro Glu Pro Val Glu Gln Phe Tyr Arg Ser
   305                 310                 315                 320
   Leu Gln Asp Thr Tyr Gly Ala Glu Pro Ala Gly Pro Asp Gly Ile Leu
                   325                 330                 335
   Val Glu Val Asp Leu Val Gln Ala Arg Leu Glu Arg Ser Ser Ser Lys
               340                 345                 350
   Ser Leu Glu Arg Glu Leu Ala Thr Pro Asp Trp Ala Glu Arg Gln Leu
           355                 360                 365
   Ala Gln Gly Gly Leu Ala Glu Val Leu Leu Ala Ala Lys Glu His Arg
       370                 375                 380
   Arg Pro Arg Glu Thr Arg Val Ile Ala Val Leu Gly Lys Ala Gly Gln
   385                 390                 395                 400
   Gly Lys Ser Tyr Trp Ala Gly Ala Val Ser Arg Ala Trp Ala Cys Gly
                   405                 410                 415
   Arg Leu Pro Gln Tyr Asp Phe Val Phe Ser Val Pro Cys His Cys Leu
               420                 425                 430
   Asn Arg Pro Gly Asp Ala Tyr Gly Leu Gln Asp Leu Leu Phe Ser Leu
           435                 440                 445
   Gly Pro Gln Pro Leu Val Ala Ala Asp Glu Val Phe Ser His Ile Leu
       450                 455                 460
   Lys Arg Pro Asp Arg Val Leu Leu Ile Leu Asp Ala Phe Glu Glu Leu
   465                 470                 475                 480
   Glu Ala Gln Asp Gly Phe Leu His Ser Thr Cys Gly Pro Ala Pro Ala
                   485                 490                 495
   Glu Pro Cys Ser Leu Arg Gly Leu Leu Ala Gly Leu Phe Gln Lys Lys
               500                 505                 510
   Leu Leu Arg Gly Cys Thr Leu Leu Leu Thr Ala Arg Pro Arg Gly Arg
           515                 520                 525
   Leu Val Gln Ser Leu Ser Lys Ala Asp Ala Leu Phe Glu Leu Ser Gly
       530                 535                 540
   Phe Ser Met Glu Gln Ala Gln Ala Tyr Val Met Arg Tyr Phe Glu Ser
   545                 550                 555                 560
   Ser Gly Met Thr Glu His Gln Asp Arg Ala Leu Thr Leu Leu Arg Asp
   565 570 575
   Arg Pro Leu Leu Leu Ser His Ser His Ser Pro Thr Leu Cys Arg Ala
   580 585 590
   Val Cys Gln Leu Ser Glu Ala Leu Leu Glu Leu Gly Glu Asp Ala Lys
   595 600 605
   Leu Pro Ser Thr Leu Thr Gly Leu Tyr Val Gly Leu Leu Gly Arg Ala
   610 615 620
   Ala Leu Asp Ser Pro Pro Gly Ala Leu Ala Glu Leu Ala Lys Leu Ala
   625 630 635 640
   Trp Glu Leu Gly Arg Arg His Gln Ser Thr Leu Gln Glu Asp Gln Phe
   645 650 655
   Pro Ser Ala Asp Val Arg Thr Trp Ala Met Ala Lys Gly Leu Val Gln
   660 665 670
   His Pro Pro Arg Ala Ala Glu Ser Glu Leu Ala Phe Pro Ser Phe Leu
   675 680 685
   Leu Gln Cys Phe Leu Gly Ala Leu Trp Leu Ala Leu Ser Gly Glu Ile
   690 695 700
   Lys Asp Lys Glu Leu Pro Gln Tyr Leu Ala Leu Thr Pro Arg Lys Lys
   705 710 715 720
   Arg Pro Tyr Asp Asn Trp Leu Glu Gly Val Pro Arg Phe Leu Ala Gly
   725 730 735
   Leu Ile Phe Gln Pro Pro Ala Arg Cys Leu Gly Ala Leu Leu Gly Pro
   740 745 750
   Ser Ala Ala Ala Ser Val Asp Arg Lys Gln Lys Val Leu Ala Arg Tyr
   755 760 765
   Leu Lys Arg Leu Gln Pro Gly Thr Leu Arg Ala Arg Gln Leu Leu Glu
   770 775 780
   Leu Leu His Cys Ala His Glu Ala Glu Glu Ala Gly Ile Trp Gln His
   785 790 795 800
   Val Val Gln Glu Leu Pro Gly Arg Leu Ser Phe Leu Gly Thr Arg Leu
   805 810 815
   Thr Pro Pro Asp Ala His Val Leu Gly Lys Ala Leu Glu Ala Ala Gly
   820 825 830
   Gln Asp Phe Ser Leu Asp Leu Arg Ser Thr Gly Ile Cys Pro Ser Gly
   835 840 845
   Leu Gly Ser Leu Val Gly Leu Ser Cys Val Thr Arg Phe Arg Ala Ala
       850                 855                 860
   Leu Ser Asp Thr Val Ala Leu Trp Glu Ser Leu Arg Gln His Gly Glu
   865                 870                 875                 880
   Thr Lys Leu Leu Gln Ala Ala Glu Glu Lys Phe Thr Ile Glu Pro Phe
                   885                 890                 895
   Lys Ala Lys Ser Leu Lys Asp Val Glu Asp Leu Gly Lys Leu Val Gln
               900                 905                 910
   Thr Gln Arg Thr Arg Ser Ser Ser Glu Asp Thr Ala Gly Glu Leu Pro
           915                 920                 925
   Ala Val Arg Asp Leu Lys Lys Leu Glu Phe Ala Leu Gly Pro Val Ser
       930                 935                 940
   Gly Pro Gln Ala Phe Pro Lys Leu Val Arg Ile Leu Thr Ala Phe Ser
   945                 950                 955                 960
   Ser Leu Gln His Leu Asp Leu Asp Ala Leu Ser Glu Asn Lys Ile Gly
                   965                 970                 975
   Asp Glu Gly Val Ser Gln Leu Ser Ala Thr Phe Pro Gln Leu Lys Ser
               980                 985                 990
   Leu Glu Thr Leu Asn Leu Ser Gln Asn Asn Ile Thr Asp Leu Gly Ala
           995                 1000                1005
   Tyr Lys Leu Ala Glu Ala Leu Pro Ser Leu Ala Ala Ser Leu Leu Arg
       1010                1015                1020
   Leu Ser Leu Tyr Asn Asn Cys Ile Cys Asp Val Gly Ala Glu Ser Leu
   1025                1030                1035                1040
   Ala Arg Val Leu Pro Asp Met Val Ser Leu Arg Val Met Asp Val Gln
                   1045                1050                1055
   Tyr Asn Lys Phe Thr Ala Ala Gly Ala Gln Gln Leu Ala Ala Ser Leu
               1060                1065                1070
   Arg Arg Cys Pro His Val Glu Thr Leu Ala Met Trp Thr Pro Thr Ile
           1075                1080                1085
   Pro Phe Ser Val Gln Glu His Leu Gln Gln Gln Asp Ser Arg Ile Ser
       1090                1095                1100
   Leu Arg
   1105
(2) INFORMATIONS POUR LA SEQ ID NO: 18:
   (i) CARACTERISTIQUES DE LA SEQUENCE:
      (A) LONGUEUR: 1130 acides aminés
      (B) TYPE: peptide
      (D) CONFIGURATION: linéaire
   (ii) TYPE DE MOLECULE: peptide
   (ix) CAR4CTERISTIQUE:
      (A) NOM/CLE: cIIta
   (xi) DESCRIPTION DE LA SEQUENCE: SEQ ID NO: 18:
   Met Arg Cys Leu Ala Pro Arg Pro Ala Gly Ser Tyr Leu Ser Glu Pro
   1               5                   10                  15
   Gln Gly Ser Ser Gln Cys Ala Thr Met Glu Leu Gly Pro Leu Glu Gly
               20                  25                  30
   Gly Tyr Leu Glu Leu Leu Asn Ser Asp Ala Asp Pro Leu Cys Leu Tyr
           35                  40                  45
   His Phe Tyr Asp Gln Met Asp Leu Ala Gly Glu Glu Glu Ile Glu Leu
       50                  55                  60
   Tyr Ser Glu Pro Asp Thr Asp Thr Ile Asn Cys Asp Gln Phe Ser Arg
   65                  70                  75                  80
   Leu Leu Cys Asp Met Glu Gly Asp Glu Glu Thr Arg Glu Ala Tyr Ala
                   85                  90                  95
   Asn Ile Ala Glu Leu Asp Gln Tyr Val Phe Gln Asp Ser Gln Leu Glu
               100                 105                 110
   Gly Leu Ser Lys Asp Ile Phe Lys His Ile Gly Pro Asp Glu Val Ile
           115                 120                 125
   Gly Glu Ser Met Glu Met Pro Ala Glu Val Gly Gln Lys Ser Gln Lys
       130                 135                 140
   Arg Pro Phe Pro Glu Glu Leu Pro Ala Asp Leu Lys His Trp Lys Pro
   145                 150                 155                 160
   Ala Glu Pro Pro Thr Val Val Thr Gly Ser Leu Leu Val Gly Pro Val
                   165                 170                 175
   Ser Asp Cys Ser Thr Leu Pro Cys Leu Pro Leu Pro Ala Leu Phe Asn
               180                 185                 190
   Gln Glu Pro Ala Ser Gly Gln Met Arg Leu Glu Lys Thr Asp Gln Ile
           195                 200                 205
   Pro Met Pro Phe Ser Ser Ser Ser Leu Ser Cys Leu Asn Leu Pro Glu
       210                 215                 220
   Gly Pro Ile Gln Phe Val Pro Thr Ile Ser Thr Leu Pro His Gly Leu
   225                 230                 235                 240
   Trp Gln Ile Ser Glu Ala Gly Thr Gly Val Ser Ser Ile Phe Ile Tyr
                   245                 250                 255
   His Gly Glu Val Pro Gln Ala Ser Gln Val Pro Pro Pro Ser Gly Phe
               260                 265                 270
   Thr Val His Gly Leu Pro Thr Ser Pro Asp Arg Pro Gly Ser Thr Ser
           275                 280                 285
   Pro Phe Ala Pro Ser Ala Thr Asp Leu Pro Ser Met Pro Glu Pro Ala
       290                 295                 300
   Leu Thr Ser Arg Ala Asn Met Thr Glu His Lys Thr Ser Pro Thr Gln
   305                 310                 315                 320
   Cys Pro Ala Ala Gly Glu Val Ser Asn Lys Leu Pro Lys Trp Pro Glu
                   325                 330                 335
   Pro Val Glu Gln Phe Tyr Arg Ser Leu Gln Asp Thr Tyr Gly Ala Glu
               340                 345                 350
   Pro Ala Gly Pro Asp Gly Ile Leu Val Glu Val Asp Leu Val Gln Ala
           355                 360                 365
   Arg Leu Glu Arg Ser Ser Ser Lys Ser Leu Glu Arg Glu Leu Ala Thr
       370                 375                 380
   Pro Asp Trp Ala Glu Arg Gln Leu Ala Gln Gly Gly Leu Ala Glu Val
   385                 390                 395                 400
   Leu Leu Ala Ala Lys Glu His Arg Arg Pro Arg Glu Thr Arg Val Ile
                   405                 410                 415
   Ala Val Leu Gly Lys Ala Gly Gln Gly Lys Ser Tyr Trp Ala Gly Ala
               420                 425                 430
   Val Ser Arg Ala Trp Ala Cys Gly Arg Leu Pro Gln Tyr Asp Phe Val
           435                 440                 445
   Phe Ser Val Pro Cys His Cys Leu Asn Arg Pro Gly Asp Ala Tyr Gly
       450                 455                 460
   Leu Gln Asp Leu Leu Phe Ser Leu Gly Pro Gln Pro Leu Val Ala Ala
   465                 470                 475                 480
   Asp Glu Val Phe Ser His Ile Leu Lys Arg Pro Asp Arg Val Leu Leu
                   485                 490                 495
   Ile Leu Asp Ala Phe Glu Glu Leu Glu Ala Gln Asp Gly Phe Leu His
               500                 505                 510
   Ser Thr Cys Gly Pro Ala Pro Ala Glu Pro Cys Ser Leu Arg Gly Leu
   515 520 525
   Leu Ala Gly Leu Phe Gln Lys Lys Leu Leu Arg Gly Cys Thr Leu Leu
   530 535 540
   Leu Thr Ala Arg Pro Arg Gly Arg Leu Val Gln Ser Leu Ser Lys Ala
   545 550 555 560
   Asp Ala Leu Phe Glu Leu Ser Gly Phe Ser Met Glu Gln Ala Gln Ala
   565 570 575
   Tyr Val Met Arg Tyr Phe Glu Ser Ser Gly Met Thr Glu His Gln Asp
   580 585 590
   Arg Ala Leu Thr Leu Leu Arg Asp Arg Pro Leu Leu Leu Ser His Ser
   595 600 605
   His Ser Pro Thr Leu Cys Arg Ala Val Cys Gln Leu Ser Glu Ala Leu
   610 615 620
   Leu Glu Leu Gly Glu Asp Ala Lys Leu Pro Ser Thr Leu Thr Gly Leu
   625 630 635 640
   Tyr Val Gly Leu Leu Gly Arg Ala Ala Leu Asp Ser Pro Pro Gly Ala
   645 650 655
   Leu Ala Glu Leu Ala Lys Leu Ala Trp Glu Leu Gly Arg Arg His Gln
   660 665 670
   Ser Thr Leu Gln Glu Asp Gln Phe Pro Ser Ala Asp Val Arg Thr Trp
   675 680 685
   Ala Met Ala Lys Gly Leu Val Gln His Pro Pro Arg Ala Ala Glu Ser
   690 695 700
   Glu Leu Ala Phe Pro Ser Phe Leu Leu Gln Cys Phe Leu Gly Ala Leu
   705 710 715 720
   Trp Leu Ala Leu Ser Gly Glu Ile Lys Asp Lys Glu Leu Pro Gln Tyr
   725 730 735
   Leu Ala Leu Thr Pro Arg Lys Lys Arg Pro Tyr Asp Asn Trp Leu Glu
   740 745 750
   Gly Val Pro Arg Phe Leu Ala Gly Leu Ile Phe Gln Pro Pro Ala Arg
   755 760 765
   Cys Leu Gly Ala Leu Leu Gly Pro Ser Ala Ala Ala Ser Val Asp Arg
   770 775 780
   Lys Gln Lys Val Leu Ala Arg Tyr Leu Lys Arg Leu Gln Pro Gly Thr
   785 790 795 800
   Leu Arg Ala Arg Gln Leu Leu Glu Leu Leu His Cys Ala His Glu Ala
   805 810 815
   Glu Glu Ala Gly Ile Trp Gln His Val Val Gln Glu Leu Pro Gly Arg
   820 825 830
   Leu Ser Phe Leu Gly Thr Arg Leu Thr Pro Pro Asp Ala His Val Leu
   835 840 845
   Gly Lys Ala Leu Glu Ala Ala Gly Gln Asp Phe Ser Leu Asp Leu Arg
   850 855 860
   Ser Thr Gly Ile Cys Pro Ser Gly Leu Gly Ser Leu Val Gly Leu Ser
   865 870 875 880
   Cys Val Thr Arg Phe Arg Ala Ala Leu Ser Asp Thr Val Ala Leu Trp
   885 890 895
   Glu Ser Leu Arg Gln His Gly Glu Thr Lys Leu Leu Gln Ala Ala Glu
   900 905 910
   Glu Lys Phe Thr Ile Glu Pro Phe Lys Ala Lys Ser Leu Lys Asp Val
   915 920 925
   Glu Asp Leu Gly Lys Leu Val Gln Thr Gln Arg Thr Arg Ser Ser Ser
   930 935 940
   Glu Asp Thr Ala Gly Glu Leu Pro Ala Val Arg Asp Leu Lys Lys Leu
   945 950 955 960
   Glu Phe Ala Leu Gly Pro Val Ser Gly Pro Gln Ala Phe Pro Lys Leu
   965 970 975
   Val Arg Ile Leu Thr Ala Phe Ser Ser Leu Gln His Leu Asp Leu Asp
   980 985 990
   Ala Leu Ser Glu Asn Lys Ile Gly Asp Glu Gly Val Ser Gln Leu Ser
   995 1000 1005
   Ala Thr Phe Pro Gln Leu Lys Ser Leu Glu Thr Leu Asn Leu Ser Gln
   1010 1015 1020
   Asn Asn Ile Thr Asp Leu Gly Ala Tyr Lys Leu Ala Glu Ala Leu Pro
   1025 1030 1035 1040
   Ser Leu Ala Ala Ser Leu Leu Arg Leu Ser Leu Tyr Asn Asn Cys Ile
   1045 1050 1055
   Cys Asp Val Gly Ala Glu Ser Leu Ala Arg Val Leu Pro Asp Met Val
   1060 1065 1070
   Ser Leu Arg Val Met Asp Val Gln Tyr Asn Lys Phe Thr Ala Ala Gly
   1075 1080 1085
   Ala Gln Gln Leu Ala Ala Ser Leu Arg Arg Cys Pro His Val Glu Thr
       1090                1095                1100
   Leu Ala Met Trp Thr Pro Thr Ile Pro Phe Ser Val Gln Glu His Leu
   1105                1110                1115                1120
   Gln Gln Gln Asp Ser Arg Ile Ser Leu Arg
                   1125                1130
(2) INFORMATIONS POUR LA SEQ ID NO: 19:
   (i) CARACTERISTIQUES DE LA SEQUENCE:
      (A) LONGUEUR: 830 acides aminés
      (B) TYPE: peptide
      (D) CONFIGURATION: linéaire
   (ii) TYPE DE MOLECULE: peptide
   (xi) DESCRIPTION DE LA SEQUENCE: SEQ ID NO: 19:
   Pro Glu Pro Ala Leu Thr Ser Arg Ala Asn Met Thr Glu His Lys Thr
   1               5                   10                  15
   Ser Pro Thr Gln Cys Pro Ala Ala Gly Glu Val Ser Asn Lys Leu Pro
               20                  25                  30
   Lys Trp Pro Glu Pro Val Glu Gln Phe Tyr Arg Ser Leu Gln Asp Thr
           35                  40                  45
   Tyr Gly Ala Glu Pro Ala Gly Pro Asp Gly Ile Leu Val Glu Val Asp
       50                  55                  60
   Leu Val Gln Ala Arg Leu Glu Arg Ser Ser Ser Lys Ser Leu Glu Arg
   65                  70                  75                  80
   Glu Leu Ala Thr Pro Asp Trp Ala Glu Arg Gln Leu Ala Gln Gly Gly
                   85                  90                  95
   Leu Ala Glu Val Leu Leu Ala Ala Lys Glu His Arg Arg Pro Arg Glu
               100                 105                 110
   Thr Arg Val Ile Ala Val Leu Gly Lys Ala Gly Gln Gly Lys Ser Tyr
           115                 120                 125
   Trp Ala Gly Ala Val Ser Arg Ala Trp Ala Cys Gly Arg Leu Pro Gln
       130                 135                 140
   Tyr Asp Phe Val Phe Ser Val Pro Cys His Cys Leu Asn Arg Pro Gly
   145                 150                 155                 160
   Asp Ala Tyr Gly Leu Gln Asp Leu Leu Phe Ser Leu Gly Pro Gln Pro
                   165                 170                 175
   Leu Val Ala Ala Asp Glu Val Phe Ser His Ile Leu Lys Arg Pro Asp
   180 185 190
   Arg Val Leu Leu Ile Leu Asp Ala Phe Glu Glu Leu Glu Ala Gln Asp
   195 200 205
   Gly Phe Leu His Ser Thr Cys Gly Pro Ala Pro Ala Glu Pro Cys Ser
   210 215 220
   Leu Arg Gly Leu Leu Ala Gly Leu Phe Gln Lys Lys Leu Leu Arg Gly
   225 230 235 240
   Cys Thr Leu Leu Leu Thr Ala Arg Pro Arg Gly Arg Leu Val Gln Ser
   245 250 255
   Leu Ser Lys Ala Asp Ala Leu Phe Glu Leu Ser Gly Phe Ser Met Glu
   260 265 270
   Gln Ala Gln Ala Tyr Val Met Arg Tyr Phe Glu Ser Ser Gly Met Thr
   275 280 285
   Glu His Gln Asp Arg Ala Leu Thr Leu Leu Arg Asp Arg Pro Leu Leu
   290 295 300
   Leu Ser His Ser His Ser Pro Thr Leu Cys Arg Ala Val Cys Gln Leu
   305 310 315 320
   Ser Glu Ala Leu Leu Glu Leu Gly Glu Asp Ala Lys Leu Pro Ser Thr
   325 330 335
   Leu Thr Gly Leu Tyr Val Gly Leu Leu Gly Arg Ala Ala Leu Asp Ser
   340 345 350
   Pro Pro Gly Ala Leu Ala Glu Leu Ala Lys Leu Ala Trp Glu Leu Gly
   355 360 365
   Arg Arg His Gln Ser Thr Leu Gln Glu Asp Gln Phe Pro Ser Ala Asp
   370 375 380
   Val Arg Thr Trp Ala Met Ala Lys Gly Leu Val Gln His Pro Pro Arg
   385 390 395 400
   Ala Ala Glu Ser Glu Leu Ala Phe Pro Ser Phe Leu Leu Gln Cys Phe
   405 410 415
   Leu Gly Ala Leu Trp Leu Ala Leu Ser Gly Glu Ile Lys Asp Lys Glu
   420 425 430
   Leu Pro Gln Tyr Leu Ala Leu Thr Pro Arg Lys Lys Arg Pro Tyr Asp
   435 440 445
   Asn Trp Leu Glu Gly Val Pro Arg Phe Leu Ala Gly Leu Ile Phe Gln
   450 455 460
   Pro Pro Ala Arg Cys Leu Gly Ala Leu Leu Gly Pro Ser Ala Ala Ala
   465 470 475 480
   Ser Val Asp Arg Lys Gln Lys Val Leu Ala Arg Tyr Leu Lys Arg Leu
   485 490 495
   Gln Pro Gly Thr Leu Arg Ala Arg Gln Leu Leu Glu Leu Leu His Cys
   500 505 510
   Ala His Glu Ala Glu Glu Ala Gly Ile Trp Gln His Val Val Gln Glu
   515 520 525
   Leu Pro Gly Arg Leu Ser Phe Leu Gly Thr Arg Leu Thr Pro Pro Asp
   530 535 540
   Ala His Val Leu Gly Lys Ala Leu Glu Ala Ala Gly Gln Asp Phe Ser
   545 550 555 560
   Leu Asp Leu Arg Ser Thr Gly Ile Cys Pro Ser Gly Leu Gly Ser Leu
   565 570 575
   Val Gly Leu Ser Cys Val Thr Arg Phe Arg Ala Ala Leu Ser Asp Thr
   580 585 590
   Val Ala Leu Trp Glu Ser Leu Arg Gln His Gly Glu Thr Lys Leu Leu
   595 600 605
   Gln Ala Ala Glu Glu Lys Phe Thr Ile Glu Pro Phe Lys Ala Lys Ser
   610 615 620
   Leu Lys Asp Val Glu Asp Leu Gly Lys Leu Val Gln Thr Gln Arg Thr
   625 630 635 640
   Arg Ser Ser Ser Glu Asp Thr Ala Gly Glu Leu Pro Ala Val Arg Asp
   645 650 655
   Leu Lys Lys Leu Glu Phe Ala Leu Gly Pro Val Ser Gly Pro Gln Ala
   660 665 670
   Phe Pro Lys Leu Val Arg Ile Leu Thr Ala Phe Ser Ser Leu Gln His
   675 680 685
   Leu Asp Leu Asp Ala Leu Ser Glu Asn Lys Ile Gly Asp Glu Gly Val
   690 695 700
   Ser Gln Leu Ser Ala Thr Phe Pro Gln Leu Lys Ser Leu Glu Thr Leu
   705 710 715 720
   Asn Leu Ser Gln Asn Asn Ile Thr Asp Leu Gly Ala Tyr Lys Leu Ala
   725 730 735
   Glu Ala Leu Pro Ser Leu Ala Ala Ser Leu Leu Arg Leu Ser Leu Tyr
   740 745 750
   Asn Asn Cys Ile Cys Asp Val Gly Ala Glu Ser Leu Ala Arg Val Leu
           755                 760                 765
   Pro Asp Met Val Ser Leu Arg Val Met Asp Val Gln Tyr Asn Lys Phe
       770                 775                 780
   Thr Ala Ala Gly Ala Gln Gln Leu Ala Ala Ser Leu Arg Arg Cys Pro
   785                 790                 795                 800
   His Val Glu Thr Leu Ala Met Trp Thr Pro Thr Ile Pro Phe Ser Val
                   805                 810                 815
   Gln Glu His Leu Gln Gln Gln Asp Ser Arg Ile Ser Leu Arg
               820                 825                 830
(2) INFORMATIONS POUR LA SEQ ID NO: 20:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce P1
   (xi) DESCRIPTION DE LA SEQUENCE: SEQ ID NO: 20:
      GTCCAGTTCC GCGATATTGG 20
(2) INFORMATIONS POUR LA SEQ ID NO: 21:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce P2
   (xi) DESCRIPTION DE LA SEQUENCE: SEQ ID NO: 21:
      TCCCTGGTCT CTTCATCA 18
(2) INFORMATIONS POUR LA SEQ ID NO: 22:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce d'adaptation ADXSC
   (xi) DESCRIPTION DE LA SEQUENCE: SEQ ID NO: 22:
      GACTCGAGTC GACATCG 17
(2) INFORMATIONS POUR LA SEQ ID NO: 23:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce d'adaptation XSCT17
   (xi) DESCRIPTION DE LA SEQUENCE: SEQ ID NO: 23:
      GACTCGAGTC GACATCGAT 19
(2) INFORMATIONS POUR LA SEQ ID NO: 24:
   (i) CARACTERISTIQUES DE LA SEQUENCE :
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce bétaGP5'
   (xi) DESCRIPTION DE LA SEQUENCE: SEQ ID NO: 24:
      TCCCCCAAAA CAGACAGAAT GG 22
(2) INFORMATIONS POUR LA SEQ ID NO: 25:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: amorce bétaGP3'
   (xi) DESCRIPTION DE LA SEQUENCE: SEQ ID NO: 25:
      GTCACAGTGC AGTTCACTCA G 21

## Description

La présente invention concerne de nouvelles séquences d'acide nucléique susceptibles d'être impliquées dans le contrôle et la régulation de l'expression de gènes codant pour des molécules MHC de type II, et leur utilisation, notamment en tant que médicament pour le traitement d'affections pour lesquelles il est souhaitable d'agir sur le niveau d'expression de gènes codant pour des molécules MHC de type II.

Les molécules du complexe majeur d'histocompatibilité (désigné ci-après MHC), de classe II sont des glycoprotéines hétérodimériques transmembranaires directement impliquées dans l'activation des lymphocytes T helper CD4+ au cours de la réponse immunitaire.

Chez l'homme, ce complexe de classe Il est représenté par les molécules appartenant au système HLA (Human Leucocyte Antigen). Les gènes codant pour les chaînes α et β constituant les molécules HLA-DR, HLA-DQ et HLA-DP sont localisés au niveau de la région D du chromosome 6.

L'expression de ces gènes est parfaitement régulée. Contrairement aux gènes codant pour les molécules MHC de type I qui sont exprimés de façon ubiquitaire, les gènes codant pour les molécules du MHC de classe II sont exprimés soit de manière constitutive uniquement dans quelques types cellulaires tels que les lymphocytes B, les lymphocytes T activés, les macrophages, les cellules de l'épithélium thymique, les cellules dendritiques telles que par exemple les cellules de Langerhans, soit de manière induite après stimulation, par exemple par des cytokines, et plus particulièrement par l'interféron γ (INF γ) ou l'interleukine 4 (IL4), dans plusieurs autres types cellulaires tels que par exemple les cellules appartenant à la lignée des macrophages ou des monocytes, les cellules endothéliales, les fibroblastes, les cellules musculaires ou les cellules cancéreuses telles que par exemple des cellules de mélanome.

En outre, dans les lymphocytes B, l'expression des gènes codant pour les molécules MHC de classe II, est transitoire. En effet, la différenciation des cellules B en plasmocytes produisant les immunoglobulines, s'accompagne de l'extinction de certains gènes, dont ceux codant pour le MHC de classe II.

De même, il a été montré que le taux d'expression des molécules MHC de type II constitue un facteur déterminant du processus d'activation des cellules T.

En conséquence, il apparaît clairement que les mécanismes moléculaires de régulation de l'expression de ces gènes constituent un élément clé de l'efficacité de la réponse immunitaire. Tout défaut dans ce procédé de régulation peut aboutir à d'importants troubles immunologiques, ou maladies auto-immunes. Ainsi, dans certains cas, une expression anormale des gènes MHC de classe II a été observée à la surface de cellules qui normalement ne devraient pas examorce de tels gènes. De même, une sur-expression de ces gènes peut être observée qui conduit à une activation aberrante et non contrôlée des lymphocytes CD4+ [BOTTAZZO et al., 1986, Immunol. Rev., 94, 137-169]. De telles manifestations pourraient être, au moins en partie, responsables d'affections telles que le diabète insulino dépendant, la sclérose en plaque, la polyarthrite rhumatoïde ou le lupus érythémateux. A l'inverse, chez certains patients, une immunodéficience a pu être mise en évidence résultant d'un trouble au niveau de l'expression des gènes MHC de classe II. Citons pour exemple le syndrome BLS (bare lymphocytes syndrome) qui est une affection autosomique récessive pour laquelle l'expression des gènes MHC de classe II est très limitée, voire inexistante, ce qui se traduit par l'absence de réponses immunitaires cellulaire et humorale et s'accompagne de nombreuses infections, souvent fatales.

Plusieurs équipes scientifiques ont analysé les mécanismes de régulation de l'expression des gènes MHC de classe II et ont identifié un certain nombre de molécules transactivatrices susceptibles de se fixer, directement ou indirectement, sur des séquences promotrices spécifiques desdits gènes [pour une revue, voir MACH et al., 1996, Annu. Rev. Immunol. 14, 301-331].

Le déposant a précédemment identifié et caractérisé l'un de ces facteurs, le facteur CIITA (class II transactivator)[STEIMLE et al., 1993, Cell 75, 135-146 et EP 648836]. Le document WO 9606107 montre en outre qu'il existe deux domaines au sein du facteur CIITA plus impliqués dans l'activation de la transcription des gènes MHC de classe II, et plus particulièrement le domaine défini par la séquence SEQ ID N° 21 de la présente invention, correspondant à la traduction de la séquence d'acide nucléique selon la SEQ ID N°17. Néanmoins, de manière surprenante et contrairement à ce qui est observé pour les autres facteurs impliqués dans la régulation de l'expression des gènes MHC de classe II (COOSWELL et al., 1991, Crit. Rev. Immunol. 11, 87-112), STEIMLE et al. ont montré que l'expression du facteur CIITA coïncide étroitement avec l'expression des gènes MHC de classe II et est absolument requise tant pour l'expression constitutive que pour l'induction desdits gènes MHC. Par ailleurs, SILACCI et al (1994, J. Exp. Med., 180, 1329-1336) ont montré que l'extinction des gènes MHC de classe II lors de la différenciation des plasmocytes est associée à l'extinction du gène codant pour le facteur CIITA.

Par ailleurs, LENNON et al. (1997, Immunogenetics , 45, 266-273) ont identifié la séquence promotrice d'un gène CIITA responsable de l'expression différentielle de ce facteur dans les cellules B. Toutefois, l'existence de cette seule séquence ne permet pas d'expliquer pourquoi on observe une expression différentielle du facteur CIITA dans différents types cellulaires. En outre, elle ne rend pas compte de l'induction par les cytokines.

Le déposant a aujourd'hui identifié, à partir d'échantillons issus de différents tissus d'origine humaine, l'organisation complexe des séquences assurant le contrôle de l'expression du facteur CIITA, isolé et caractérisé d'autres régions promotrices et mis en évidence l'existence de plusieurs formes du facteur CIITA, et également de différents gènes CIITA.

Par « gène CIITA », on entend désigner une séquence d'acide nucléique constituée par une partie promoteur (P), une partie non traduite (NT), et une partie codante (Prot), la partie codante codant pour l'une des formes du facteur CIITA identifiée.

Plus précisément, les inventeurs ont identifié un certain nombres de séquences d'acide nucléique de gènes CIITA et donc susceptibles notamment d'être impliquées dans le contrôle et la régulation de l'expression de gènes codant pour des molécules MHC de classe II. Par l'expression « séquence d'acide nucléique de gènes CIITA », on entend que la séquence en cause comprend tout ou partie d'une séquence d'acide nucléique correspondant aux ARNm issus de différents tissus ou lignées cellulaires exprimant une activité CIITA de manière constitutive ou après induction. Il peut donc s'agir aussi bien de séquences au moins partiellement codantes que par exemple de séquences intervenant dans le contrôle de l'expression , notamment des séquences ayant une activité de promoteur transcriptionnel.

Par «séquence d'acide nucléique», on entend un fragment d'ADN et/ou d'ARN, double brin ou simple brin, naturel isolé, ou de synthèse, désignant un enchaînement précis de nucléotides, modifiés ou non, permettant de définir un fragment ou une région d'un acide nucléique.

Par «polypeptide», on entend désigner un enchaînement précis d'amino-acides, indépendamment de sa taille ou de sa fonction, naturel isolé ou de synthèse, modifié ou non.

Par «variant allélique» d'un polypeptide, on entend désigner l'ensemble des polypeptides mutés et des polymorphismes pouvant exister chez l'être humain, obtenus notamment par troncature, substitution, délétion ou addition de résidus d'amino-acides, ainsi que les variants artificiels mis en oeuvre in vitro.

Par «séquence d'acide nucléique présentant une activité de promoteur transcriptionnel», on entend une séquence d'acide nucléique permettant de contrôler, c'est à dire d'initier et/ou de moduler, la transcription d'au moins un gène, homologue ou hétérologue, localisé en aval de ladite séquence. De même, on parlera de la fonction promotrice desdites séquences.

Par » séquence d'acide nucléique homologue à une première séquence d'acide nucléique », on entend une séquence d'acide nucléique qui présente naturellement un lien fonctionnel avec ladite première séquence. Ainsi par exemple, selon l'invention , une séquence d'acide nucléique présentant une activité de promoteur CITA, c'est-a-dire dirigeant naturellement la transcription d'une séquence d'acide nucléique codant pour un facteur CIITA est considérée comme homologue à cette même séquence d'acide nucléique codant pour un gène CIITA. Dans le cas contraire, on parlera de « séquence d'acide nucléique hétérologue ».

Par «gène reporteur», on entend toute séquence d'acide nucléique localisée en aval d'une seconde séquence d'acide nucléique, permettant d'analyser l'activité de promoteur transcriptionnel de ladite seconde séquence. En effet, la transcription de ce gène reporteur se traduit par l'apparition d'un produit (ARN ou polypeptide) aisément détectable selon les techniques conventionnelles bien connues.

Il doit être compris que la présente invention ne concerne pas les séquences nucléotidiques génomiques dans leur environnement chromosomique naturel, c'est à dire à l'état naturel, il s'agit de séquences qui ont été isolées, c'est à dire qu'elles ont été prélevées directement ou indirectement, par exemple par copie (ADNc), et que leur environnement a été, au moins partiellement, modifié.

L'invention a ainsi pour objet une séquence d'acide nucléique comprenant tout ou partie de la séquence d'acide nucléique d'un gène CIITA, SEQ ID N°3 ou de sa séquence complémentaire, telle que ou ladite partie ou sa séquence complémentaire présente une activité de promoteur transcriptionnel induite par une cytokine, ou brin comprend la partie non traduite de la séquence SEQ ID N°3.

Parmi les séquences particulièrement intéressantes, il faut citer celles comprenant SEQ ID n°6, ou sa séquence complémentaire.

Certaines séquences identifiées selon l'invention sont capables d'examorce leur activité de promoteur transcriptionnel, après induction par une cytokine, telle que par exemple l'interféron γ ou l'interleukine 4. Un exemple préféré d'une telle séquence est représenté par la séquence comprenant tout ou partie d'une séquence identifiée SEQ ID n°6, ou sa séquence complémentaire.

L'invention concerne également les séquences d'acide nucléique comprenant x tout ou partie de la séquence. SEQ ID N°10 ou de sa séquence complémentaire.

La présente invention concerne en outre une séquence d'acide nucléique comprenant au moins une séquence présentant une activité de promoteur transcriptionnel, telle que notamment les séquences comprenant tout ou partie de la séquence, SEQ ID n°6, ou de sa séquence complémentaire, localisée en amont d'au moins une séquence d'acide nucléique hétérologue ou homologue, telle que par exemple une séquence d'acide nucléique comprenant tout ou partie d'une séquence choisie parmi :

les séquences d'acide nucléique identifiées SEQ ID N°7, SEQ ID N°8, SEQ ID N°9, SEQ ID N°11, SEQ ID N°12, SEQ ID N°13, SEQ ID N°14 et la SEQ ID N°15, et leurs séquences complémentaires.

Il convient de préciser que dans ce cas, il est possible d'avoir au moins deux séquences présentant une activité de promoteur transcriptionnel et/ou au moins deux séquences d'acide nucléique hétérologues ou homologues, situées l'une par rapport à l'autre de manière contiguë, éloignée, dans le même sens ou en sens inverse, pour autant que la fonction de promoteur transcriptionnel ou la transcription desdites séquences ne soit pas affectée.

De même, dans ce type de constructions d'acide nucléique, il est possible d'introduire des séquences nucléiques «neutres» ou introns qui n'affectent pas la transcription et sont épissées avant l'étape de traduction. De telles séquences et leurs utilisations sont largement décrites dans la littérature.

Selon l'invention, les séquences d'acide nucléique ou leurs fragments peuvent notamment coder pour tout ou partie de polypeptides ayant la séquence en acides aminés d'un facteur CIITA tels que décrits dans la présente invention.

On dira alors qu'elles codent pour des polypeptides CIITA

Elles peuvent aussi être utilisés comme sonde ou comme dans des procédés de détection ou d'identification ou d'amplification enzymatique d'acide nucléique. Dans ce cas, les fragments présentent une taille minimale de 10 bases, et on préférera des fragments de 20 bases, et de préférence de 30 bases.

La présente invention concerne également une séquence d'acide nucléique ayant une séquence complémentaire à une séquence cible appartenant à un gène ou à un ARN dont on désire bloquer spécifiquement l'expression. Une telle séquence constitue un oligonucléotide antisens qui hybride à la séquence dont il est complémentaire et peut ainsi bloquer l'expression de l'ARNm portant cette séquence. Le terme « oligonucléotide » est ici utilisé de façon générale pour désigner un polynucléotide de 2 à 100, et plus généralement de 5 à 50 nucléotides en série ribo-désoxyribo- ou mixte. Selon l'invention, une telle séquence est capable d'hybridation avec une séquence d'acide nucléique comprenant une séquence présentant une activité de promoteur transcriptionnel ou avec une séquence d'acide nucléique comprenant une séquence telle que définie précédemment et capable en outre soit de bloquer l'activité promotrice de ladite séquence, soit d'inhiber la synthèse du polypeptide codé par ladite séquence.

Les conditions d'hybridation, selon l'invention, sont déterminées afin d'assurer au moins 95% d'homologie, L'homme du métier dispose des connaissances suffisantes pour lui permettre de définir lesdites conditions.

Si les séquences décrites sont, en général, les séquences normales, l'invention concerne également les séquences mutées dans la mesure où elles comportent au moins une mutation ponctuelle et de préférence moins de 20 mutations au total.

De préférence, la présente invention concerne des séquences nucléotidiques dans lesquelles les mutations ponctuelles ne sont pas muettes, c'est à dire qu'elles conduisent soit à une modification de la régulation de l'efficacité ou de la spécificité cellulaire de la transcription du gène localisé en aval de ladite séquence, soit à une modification de la séquence codante affectant l'expression du gène CIITA, soit à une modification de l'amino-acide codé par rapport à la séquence normale affectant la fonction du facteur CIITA correspondant.

La présente invention concerne notamment une séquence d'acide nucléique comprenant une mutation affectant la fonction de promoteur transcriptionnel de ladite séquence. De manière préférée, cette mutation porte sur les régions impliquées dans l'activité de promoteur transcriptionnel permettant la fixation de facteurs impliqués dans l'étape d'initiation, d'activation ou de modulation de la transcription, ou plus généralement dans la transcription. Ces régions peuvent par exemple consister en au moins un site impliqué dans le processus de transcription choisi parmi le groupe consistant en le site NF-GMb (Shannon et al., 1988. Proc.Natl.Acad.Sci.USA,85, 674-678), le site NF-IL6 (Akira et Kishimoto, 1992, Immunil. Rev. 127, 25-50), le site PEA3 (Wasylyk et al., 1989, EMBO J., 8, 3371-3378), le site AP1 (Pollock et Treisman, 1990, Nucleic Acid Res.18, 6197-6204), la boîte CCAAT (Dorn et al., 1987, Cell, 50, 863-872)la boite E2A (Murre et al., 1959, Cell, 56, 777-783), le site IRF1/2 (Tanaka et al., 1993, Molecular and Cellular Biology, 13, 4531-4538), le site MYC (Agira et al., 1989, EMBO J., 8, 4273-4279), le site OCT (Rosales et al , 1987, EMBO J., 6,3015-3025), le site NF.GMa (Shannon et al., 1988, Proc. Natl. Acad. Sci. USA, 85, 674-678), la boîte GAS (Pelligrini et Schindler, 1993, Trends Biochem. Sci., 18, 338-342), la boîte E (Blackwell et al., 1990, Science, 250, 1149-1151) et le site NfκB (Sen et Baltimore, 1986, Cell, 46, 705-716).

La présente invention concerne également une séquence nucléotidique pouvant comporter des nucléotides non naturels, notamment des nucléotides soufrés par exemple ou de structure α ou β, ou des nucléotides marqués par un marqueur qui est, à titre d'exemple, choisi dans le groupe consistant en une enzyme, la biotine, l'iminobiotine, un composant fluorescent, un composant radioactif, un composant chemioluminescent, un composant d'électrodensité, un composant magnétique, un antigène, un haptène et un anticorps.

La présente invention concerne également des vecteurs de clonage ou d'expression comportant au moins une séquence nucléotidique telle que décrite précédemment.

Ces vecteurs de clonage ou d'expression peuvent en outre comporter des éléments assurant l'expression de la séquence dans la cellule hôte, notamment des séquences promotrices et/ou des séquences de régulation efficaces dans ladite cellule, s'il s'agit d'une séquence codante.

S'il s'agit d'une séquence ayant une activité de promoteur transcriptionnel, le vecteur comportera en outre des séquences d'acide nucléique homologues ou hétérologues que l'on souhaite examorce dans ladite cellule.

De manière préférentielle, ces vecteurs de clonage ou d'expression comprennent au moins un gène d'intérêt placé sous le contrôle d'au moins une séquence d'acide nucléique telle que décrite précédemment et présentant une activité de promoteur transcriptionnel.

Ledit gène d'intérêt pourra par exemple être choisi parmi le groupe consistant en les gènes codant pour le facteur CIITA, les chaînes α et β des molécules HLA-DR, HLA-DQ et/ou HLA-DP et les gènes reporteurs tels que par exemple le gène codant pour la β globine de lapin.

Le vecteur en cause peut être choisi parmi les vecteurs à réplication autonome ou parmi les vecteurs d'intégration chromosomique.

Dans le cas de système à réplication autonome, en fonction de la cellule hôte, procaryote ou eucaryote, on utilisera de préférence des systèmes de type plasmidiques ou des systèmes viraux, les vecteurs virus pouvant notamment être des adénovirus, des poxvirus ou des virus herpétiques. L'homme du métier connaît les technologies utilisables pour chacun de ces virus.

Lorsque l'on souhaitera l'intégration de la séquence dans les chromosomes de la cellule hôte, il sera nécessaire de prévoir de part et d'autre de la séquence d'acide nucléique à intégrer une ou plusieurs séquences provenant de la cellule hôte afin d'assurer la recombinaison. Il s'agit là également de procédés qui sont largement décrits dans la littérature antérieure. On pourra notamment utiliser des systèmes de type plasmidique ou viral, tels que par exemple, les rétrovirus ou les AAV (Association Adénovirus Virus).

L'invention concerne également les cellules procaryotes ou eucaryotes transformées par un vecteur tel que décrit précédemment, notamment afin d'assurer l'expression d'au moins une des formes du facteur CIITA identifiées selon l'invention.

Parmi les cellules utilisables pour la réalisation de l'invention, il faut citer bien entendu les cellules procaryotes, les cellules de levure et les cellules animales, en particulier les cultures de cellules de mammifère.

De manière préférentielle, la cellule hôte est choisie dans le groupe consistant en cellule dendritique, lymphocyte B, lymphocyte T, macrophage, monocyte, cellule de l'épithélium thymique, cellule musculaire, fibroblaste, cellule endothéliale et cellule cancéreuse, notamment cellule de mélanome.

Les cellules ainsi obtenues peuvent permettre de préparer des polypeptides CIITA naturels ou mutés, mais également des fragments de ces polypeptides.

Ces cellules peuvent également être utilisées à titre de modèle afin d'étudier les modes de régulation de la fonction de promoteur transcriptionnel des séquences identifiées selon l'invention et d'identifier des inhibiteurs spécifiques, dont l'action pourrait éventuellement être ciblée dans un type cellulaire donné. Ces cellules peuvent en outre être utilisées à titre de modèle afin d'étudier les interactions entre les différents facteurs CIITA isolés, ou leurs variants, et les régions dirigeant la transcription des gènes codant pour les molécules du MHC de classe Il, et surtout afin de sélectionner les variants du facteurs CIITA susceptibles d'agir à titre d'agoniste ou d'antagoniste sur le récepteur de CITTA Ces types de modèle cellulaire peuvent être réalisés en mettant en oeuvre des techniques connues de génie génétique. Par ailleurs, l'utilisation de tels modèles cellulaires en vue de tester des composés pharmaceutiques est bien connue de l'homme de l'art.

La présente invention concerne également des organismes tels que les animaux, en particulier des souris, dont le génome a été génétiquement modifié afin d'intégrer au moins une des séquences d'acide nucléique selon l'invention. Ici encore, ces animaux peuvent être utilisés comme animaux modèles afin de tester l'efficacité de certains produits pharmaceutiques.

La présente invention concerne également un procédé de production d'un polypeptide CIITA, notamment tel que défini dans la SEQ ID N° 17, comprenant (i) la culture d'une cellule hôte transformée par un vecteur comprenant une séquence d'acide nucléique codant pour un polypeptide CIITA tel que décrit précédemment, dans des conditions de culture appropriées permettant la production dudit polypeptide et (ii) la récupération dudit polypeptide.

La récupération dudit polypeptide peut être effectuée de façon intracellulaire ou de façon extracellulaire dans le milieu de culture lorsque le vecteur a été conçu pour assurer l'excrétion du polypeptide par le biais, par exemple, d'une séquence «leader», le polypeptide étant sous la forme d'un pré-polypeptide. Les constructions permettant la sécrétion des polypeptides sont connues, aussi bien pour les systèmes procaryotes que les systèmes eucaryotes.

La présente invention concerne également un polypeptide CIITA susceptible d'être obtenu par la mise en oeuvre du procédé décrit ci-dessus.

La présente invention concerne en outre les polypeptides CIITA correspondant aux séquences d'acide nucléique décrites précédemment, sous forme non naturelle, c'est à dire qu'elles ne sont pas prises dans leur environnement naturel mais obtenues par purification à partir de sources naturelles ou bien obtenues par recombinaison génétique.

L'invention concerne également les mêmes polypeptides obtenus par synthèse chimique et pouvant comporter des amino-acides non naturels. L'invention porte également sur lesdits polypeptides sous une forme totalement ou partiellement rétro et/ou inverso présentant une activité équivalente à celle observée pour le facteur CIITA natif ou un de ses variants selon la présente invention, ou pour le moins une activité immunologique identique à celle du facteur CIITA parent.

Par ailleurs, les polypeptides, et plus particulièrement leurs variants, tels que décrits précédemment, peuvent présenter la même fonction transactivatrice de l'expression des gènes codant pour les molécules MHC de classe II qu'un facteur CIITA, ou pour le moins, la même capacité à se fixer sur le site de fixation spécifique d'un facteur CIITA au cours de l'expression desdits gènes.

La présente invention concerne en outre un anticorps dirigé contre l'un quelconque des polypeptides décrits précédemment ou contre un polypeptide comprenant au moins une mutation affectant la fonction du facteur CIITA comme décrit ci-dessous, et plus particulièrement un anticorps polyclonal ou monoclonal obtenu par réaction immunologique d'un organisme humain ou animal avec un agent immunogène comprenant au moins l'un desdits polypeptides.

L'invention concerne également des molécules capables d'inhiber soit la fonction activatrice de l'expression des gènes codant pour les molécules MHC de classe II des polypeptides identifiés selon l'invention, soit leur capacité à se fixer sur le site de fixation de CIITA. Il peut s'agir de polypeptides comprenant au moins une mutation affectant la fonction du facteur CIITA. Un tel polypeptide modifié, consistant par exemple en un analogue structural dudit polypeptide, peut jouer le rôle de leurre. Il peut également s'agir d'anticorps tels que présentés ci-dessus qui sont capables par exemple de bloquer soit tout ou partie du facteur CIITA susceptible de réagir avec son récepteur spécifique, soit une région du facteur CIITA capable d'interagir avec au moins un autre facteur transactivateur lors de l'expression des gènes codant pour les molécules MHC de classe II.

L'invention concerne également des molécules capables d'inhiber spécifiquement l'induction par les cytokines de l'expression des gènes codant pour les molécules MHC de classe II. Ces molécules consistent notamment en tout ou partie d'une séquence d'acide nucléique comprenant au moins une mutation affectant la fonction de promoteur transcriptionnel de ladite séquence et en ce que la ou les mutations sont localisées dans la séquence d'acide nucléique identifiée SEQ ID n°6, ou sa séquence complémentaire.

La présente invention concerne également des compositions pharmaceutiques comportant à titre de principe actif au moins une substance telle qu'une séquence d'acide nucléique ou une molécule inhibitrice telles que définies précédemment. Plus particulièrement, l'invention concerne une composition pharmaceutique pour le traitement d'affections pour lesquelles une augmentation de l'expression des gènes codant pour les molécules MHC de classe II est désirée, notamment dans un type cellulaire. Par ailleurs, il est possible d'observer cette augmentation de l'expression des gènes codant pour les molécules MHC de classe II après induction par une cytokine, et plus particulièrement par l'interféron γ ou l'interleukine 4, notamment lorsque ladite composition pharmaceutique comprend au moins une substance consistant en une séquence d'acide nucléique susceptible d'activation par ladite cytokine telle que décrite précédemment. L'invention porte en outre sur une dite composition pharmaceutique pour le traitement d'affections pour lesquelles une réduction de l'expression des gènes codant pour les molécules MHC de classe II est désirée, et plus particulièrement sur une composition pharmaceutique comprenant à titre de principe actif a) soit une séquence d'acide nucléique selon l'invention dont la séquence est modifiée de manière à ce que l'activité promotrice de ladite séquence soit affectée, ou qui conduit à la production d'un polypeptide CIITA inactif tel que précédemment décrit, b) soit un polypeptide CIITA inactif.

L'invention concerne par ailleurs un vaccin utilisable notamment pour le traitement de cancer ou de maladies auto-immunes caractérisé en ce qu'il comprend au moins l'une des compositions pharmaceutiques présentées ci-dessus.

Enfin, la présente invention concerne plus particulièrement des méthodes de diagnostic d'une prédisposition à une affection liée à un trouble de l'expression des gènes codant pour les molécules MHC de classe II caractérisée en ce qu'on effectue un prélèvement biologique chez un patient, on détermine la présence d'au moins une mutation au niveau soit des séquences présentant une activité de promoteur transcriptionnel, soit des séquences codant pour l'un des facteurs CIITA identifiés selon la présente invention, par analyse desdites séquences d'acide nucléique et comparaison avec les séquences sauvages selon l'invention, la présence d'au moins une telle mutation étant indicative d'une prédisposition dudit patient à ladite affection.

De nombreuses affections, directement ou indirectement, liées à un trouble de l'expression des gènes codant pour les molécules MHC de classe II sont décrites dans la littérature. Citons pour exemple, des affections telles que le diabète insulino dépendant, la sclérose en plaque, la polyarthrite rhumatoide ou le lupus érythémateux dont une des composantes pourrait être une sur-expression des gènes codant pour les molécules MHC de classeII ; ou à l'inverse le syndrome BLS (bare lymphocytes syndrome) qui est associé à une sévère une immunodéficience.

Parmi les mutations qui sont recherchées, il faut citer plus particulièrement les mutations affectant la fonction promotrice des séquences d'acide nucléique, des mutations qui affectent la spécificité cellulaire de ladite fonction promotrice, ou des mutations qui affectent l'induction par une cytokine de ladite fonction promotrice.

La séquence d'acide nucléique analysée peut aussi bien être un ADN génomique, un ADNc ou un ARN.

Les outils de diagnostiques basés sur la présente invention peuvent permettre un diagnostic positif et différentiel chez un sujet pris isolément ou bien un diagnostic présymptômatique chez un sujet à risque.

Les méthodes permettant de mettre en évidence une mutation dans un gène par rapport au gène naturel sont, bien entendu, très nombreuses, elles peuvent être mises en oeuvre par l'étude de l'ADN génomique, de l'ADNc, de l'ARN et/ou du polypeptide. On peut essentiellement les diviser en deux grandes catégories, le premier type de méthode est celui dans lequel la présence d'une mutation est détectée par comparaison de la séquence mutée avec la séquence correspondante naturelle non mutée, et le second type dans lequel la présence de la mutation est détectée de façon indirecte. De façon avantageuse,la mutation peut être détectée par la mise en évidence de misappariements dûs à la présence de la mutation après analyse par hybridation réalisée à l'aide d'au moins une sonde oligonucléotidique spécifique de la mutation recherchée.

Dans chacun des cas, on préférera en général les méthodes dans lesquelles tout ou partie de la séquence correspondant à tout ou partie de la séquence identifiée SEQ ID n°3 est amplifiée préalablement à la mise en évidence de la mutation. Ces méthodes d'amplification sont bien connues.

Par ailleurs, les facteurs mutés CIITA trouvés chez les sujets présentant des troubles de l'expression des gènes codant pour les molécules MHC de type II peuvent présenter une antigénicité différente des facteurs CIITA naturels identifiés SEQ ID n°16, SEQ ID n°17 ou SEQ ID n°18. Il est donc possible de réaliser un diagnostic ou pronostic d'une susceptibilité à des troubles liés à une dérégulation de l'expression des gènes codant pour les molécules MHC de type II, en mettant en évidence le produit du gène muté de CIITA, par exemple à l'aide d'anticorps, notamment d'anticorps monoclonaux, tels que décrits précédemment.

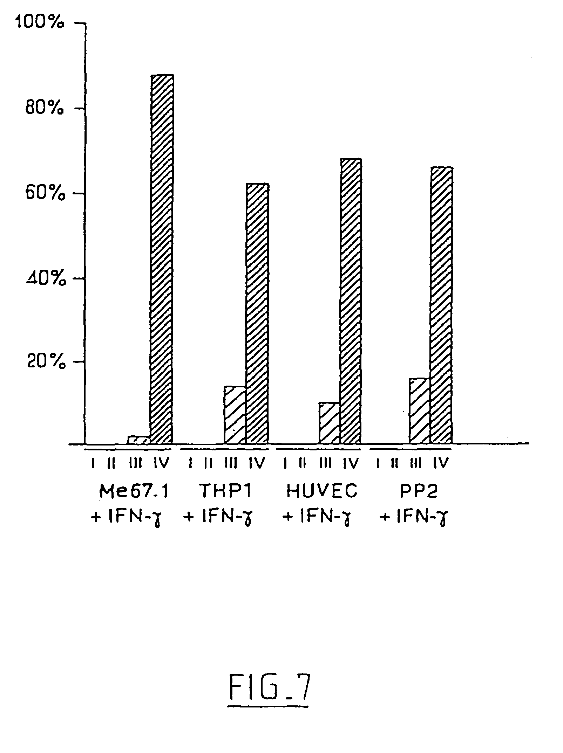

D'autres caractéristiques et avantages de la présente invention apparaîtront à la lecture des exemples ci-après, illustrés par les figures 1 à 9. Toutefois, l'invention ne saurait se limiter au contenu desdits exemples.

Légendes des figures :
La Figure 1 représente les quatres extrémités 5' des ARNm CIITA identifiées selon l'exemple 1. Les régions codantes sont indiquées par des boites larges, les régions 5' non traduites par des boites plus petites. Les régions non homologues sont indiquées par un remplissage distinct. La position des deux s P 1 et P2 utilisées pour l'amplification par RACE-PCR est indiquée.
La Figure 2 indique la séquence et la position des différents sites de fixation de facteurs de transcription connus identifiés sur la séquence de la 5' région flanquante du gène CIITA de type I. Le site majeur d'initiation de la transcription est également indiqué par une flèche en +1.
La Figure 3 indique la séquence et la position des différents sites de fixation de facteurs de transcription connus identifiés sur la séquence de la 5' région flanquante du gène CIITA de type III. Le site majeur d'initiation de la transcription est également indiqué par une flèche en +1.
La Figure 4 indique la séquence et la position des différents sites de fixation de facteurs de transcription connus identifiés sur la séquence de la 5' région flanquante du gène CIITA de type IV. Le site majeur d'initiation de la transcription est également indiqué par une flèche en +1.
La Figure 5 est une représentation schématique des sondes utilisées dans les tests de protection à la RNAse lors de l'analyse des profils d'expression des différents ARNm CIITA. Les différentes sondes sont proposées avec leurs tailles «avant» et «après» digestion par la RNAse. Chacune des sondes correspond à une partie de l'exon 1 et à 226 bases communes dans chacun des ARNm.
La Figure 6 est une représentation schématique de l'expression différentielle des quatre types de transcrits CIITA. La quantité de chacun des types d'ARNm est indiquée en pourcentage par rapport à la quantité totale d'expression de CIITA mesurée à l'aide du contrôle interne et après quantification par PhosphoImager des fragments obtenus suite à l'analyse de protection par la RNAse.
La Figure 7 est une représentation schématique du même type que celle de la Figure 6, sauf que l'expresssion des transcrits CIITA est observée après induction par l'interféron α(+IFNγ).
La figure 8 représenté l'organisation du promoteur IV humain sauvage et muté. Les séquence et positions des éléments cis conservés sont indiqués. Les mutations ponctuelles introduites au niveau des éléments GAS, boîte E et IRF-1 sont précisées en dessous de la séquence sauvage.
La figure 9 représente l'analyse fonctionnelle du promoteur IV sauvage (wt) et des mutants Gm, Em et Im. Le taux de stimulation de l'expression du gène reporteur analysé est indiqué en % par rapport au taux observé avec le plasmide reporteur sauvage (100 %). Les résultats indiqués correspondent à la moyenne de trois expériences indépendantes, la déviation standard est précisée.

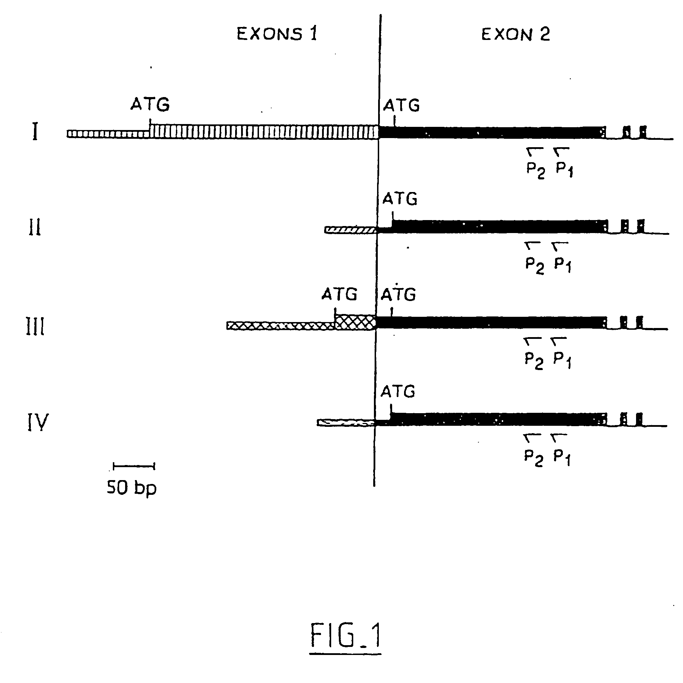

L'invention est illustrée également par les identificateurs de séquence SEQ ID N° 1 à SEQ ID N° 25, lesquels représentent :
- SEQ ID N°1 à SEQ ID N°3 : les séquences des trois types d'ADNc correspondant aux gènes CIITA (séquences désignées I, Il et IV à la figure 1), identifiées selon l'invention ;
- SEQ ID N°4 à SEQ ID N°6 : les séquences identifiées comme présentant une activité de promoteur transcriptionnel dans les gènes CIITA de forme I, II et IV et désignés respectivement PI, PII et PIV;
- SEQ ID N° 7 à SEQ ID N° 10 : les séquences correspondant respectivement aux différents gènes CIITA de forme I à IV, dépourvus des séquences présentant une activité de promoteur transcriptionnel ;
- SEQ ID N° 11: la séquence correspondant à la partie codante du gène
- SEQ ID N° 12 :la séquence correspondant à la partie codante du gène CIITA de forme II ;
- SEQ ID N°13 : la séquence correspondant à la partie codante du gène CIITA de forme III ;
- SEQ ID N°14 : la séquence correspondant à la partie codante du gène CIITA de forme IV , y compris une partie non traduite;
- SEQ ID N° 15 :un fragment de la séquence SEQ ID N°14, correspondant aux nucléotides 901 à 3390, compté à partir du premier nucléotide de la SEQ ID N°13 ;
- SEQ ID N°16: la traduction en amino acides de SEQ ID N°11, correspondant à un facteur CIITA de forme I présentant 101 amino-acides supplémentaires à l'extrémité N terminale, par rapport à SEQ ID N°17 ;
- SEQ ID N° 17 : la traduction en amino acides de la partie codante des gènes CIITA de forme I à IV, à partir d'un ATG localisé 21 bases en aval de l'extrémité 5' de l'exon 2 commun (figure 1);
- SEQ ID N° 18 : la traduction en amino acides du gène CIITA de forme III, au départ d'un second ATG, correspondant à un facteur CIITA présentant 24 amino-acides supplémentaires à l'extrémité N terminale ;
- SEQ ID N° 19 : la traduction en amino-acides de SEQ ID N°15,
- SEQ ID N° 20 à 25 : amorces de PCR.

### EXEMPLES.

### Exemple 1:

Les ARN cytoplasmiques ou totaux ont été extraits à partir de différentes lignées cellulaires : Raji (lymphome de Burkitt), Mann (lymphocyte B humain), CEM (lignée lymphoblastoïde T), THP1 (monocyte), PP2 (fibroblaste), Me67 (mélanome) après induction par l'interféron γ et HUVEC ( cellule endothéliale humaine),selon la technique décrite par Wilkinson ( 1988, Nucleic Acid Res. 16, 10933). L'ARN total issu de la lignée BCl (cellules dendritiques)a été préparé à l'aide d'un réactif au Trizol (Gibco Brl). Les ARN issus de rate, thymus, amygdale et rein humains ont été gracieusement fournis par P Sapino.

L'extrémité 5' des ARN obtenus a été analysée par la technique RACE PCR (Frohman et al., 1988, Proc. Natl. Acad. Sci. USA, 85, 8998-9002) selon les instructions du fabricant (Gibco BRL) avec les modifications suivantes. Après transcription inverse des ARN et avant l'étape d'amplification, une queue de dATP est ajoutée à l'extrémité des ADNc. Lors de l'amplification PCR, 5µl d'ADNc-dA isolé sont ajoutés à 40µl d'un mélange d'amplification renfermant 200µM de chacun des dNTP et 25 pmol d's spécifiques du gène codant pour le facteur CIITA PI (5'-GTCCAGTTCCGCGATATTGG-3') et P2 (5'-TCCCTGGTCTCTTCATCA-3'), 25 pmol d' d'adaptation ADXSC (5'-GACTCGAGTCGACATCG-3') et 10pmol d' d'adaptation XSCT17 (5'-GACTCGAGTCGACATCGAT-3'). Après une préincubation de 5 minutes à 95°C, 2 unités de Taq polymérase sont ajoutées et l'amplification est réalisée en 30 cycles de 45 secondes à 94°C, 25 secondes à 54°C, 2 minutes à 72°C. La dernière incubation est réalisée pendant 10 minutes à 72°C.

Ces amplifications ont permis de mettre en évidence quatre types d'ADNc correspondant au facteur CIITA. L'analyse des séquences de ces acides nucléiques a montré que ces acides nucléiques présentaient tous une extrémité 3' commune (Exon2) mais divergaient totalement au niveau de leur extrémité 5', définissant ainsi quatre types différents d'exon 1. Ces quatre séquences (Exon 1 variable + Exon 2 commun) sont identifiées I, II, III et IV (Figure I).

Comme l'indique l'analyse des séquences, ces quatre transcrits I, II, III et IV présentent une phase de lecture commune débutant à l'ATG localisé 21 bases en aval de l'extrémité 5' de l'Exon 2 commun (Figure 1). Dans le cas des séquences II et IV, cet ATG est le premier codon d'initiation. Dans le cas des séquences I et III, un autre ATG est observé qui conduit à la synthèse d'un facteur CIITA présentant 101 ou 24 amino-acides supplémentaires, respectivement, à l'extrémité N terminale du polypeptide traduit.

### Exemple 2:

Le site d'initiation de la transcription des différents ARNm CIITA humains identifiés a été testé par protection à la RNAse en utilisant des fragments d'ADN spécifiques des différents Exons 1.

Pour les transcrits de type I, trois fragments protégés ont été identifiés à partir d'acide nucléique isolé de foie. Le fragment majeur correspond au site d'initiation de la transcription localisé 380 bases en amont de l'extrémité 3' de l'Exon 1 (Figure 1). Ce site a été défini comme nucléotide +1 des ARNm de type I. Les deux autres transcrits sont obtenus à partir des sites d'initiation localisés en positions -14 et +8. Les localisations de ces sites d'initiation sont compatibles avec l'utilisation, au cours de la traduction, des signaux ATG identifiés dans l'Exemple 1.

Pour les transcrits de type III, plusieurs fragments protégés ont été identifiés à partir d'acide nucléique isolé de lymphocytes B. Le transcrit majeur correspond à une initiation à partir de la position 183 bases en amont de l'extrémité 3' de l'Exon 1 et définit la position +1 des transcrits de type III. Deux autres sites d'initiation sont localisés aux positions -8 et -4. D'autres sites mineurs sont identifiés aux positions -23 et +34. Ces sites d'initiation sont compatibles avec l'utilisation des deux sites ATG localisés dans l'Exemple 1.

Pour les transcrits de type IV, de nombreux fragments protégés ont été identifiés à partir d'acides nucléiques isolés de cellules de mélanome induites par l'interféron γ. Le transcrit majeur correspond à une initiation de la transcription localisée 75 bases en amont de l'extrémité 3' de l'Exon 1 qui définit la position +1 des transcrits de type IV. Un second site d'initiation majeur est observé à la position +17, ainsi que six sites mineurs localisés entre les positions -54 et +69 de l'Exon 1. Ces sites d'initiation sont compatibles avec l'utilisation de l'ATG localisé 21 bases en aval de l'extrémité 5' de l'Exon 2 (Exemple 1).

La présence de sites d'initiation distincts pour chacun des ARN I, II, III et IV suggère que les régions promotrices contrôlant l'expression des gènes correspondants sont distinctes (notée PI, PII, PIII et PIV).

### Exemple 3:

Ayant identifié les divergences de séquence observées au niveau des extrémités 5' des ARNm (Exon 1 et séquence non traduite), le déposant a ensuite isolé les séquences génomiques, incluant les régions promotrices, des gènes I, II, III et IV à partir d'une banque phagique λ renfermant le génome humain.

La comparaison des séquences correspondant aux quatre promoteurs PI, PII, PIII et PIV ne montre pas d'homologie significative. Aucune de ces régions ne renferme de boîte TATA ou GC. Cette dernière observation explique le nombre important de sites d'initiation observés pour un transcrit donné.

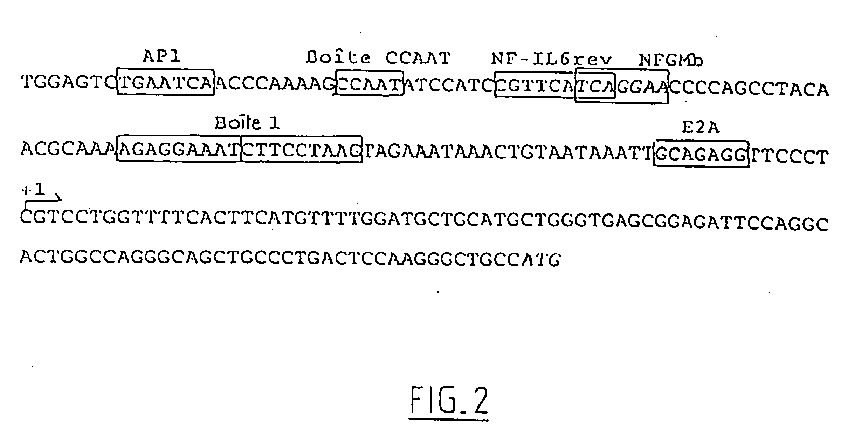

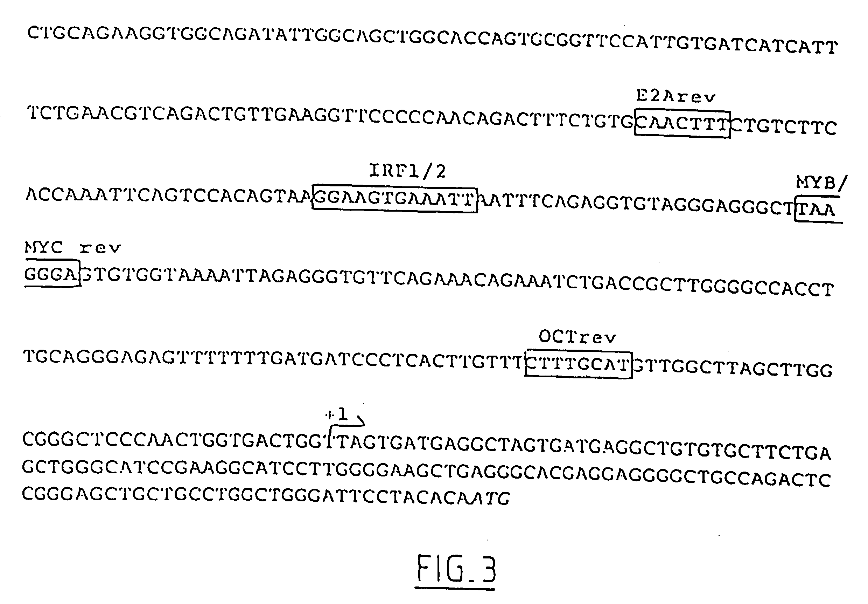

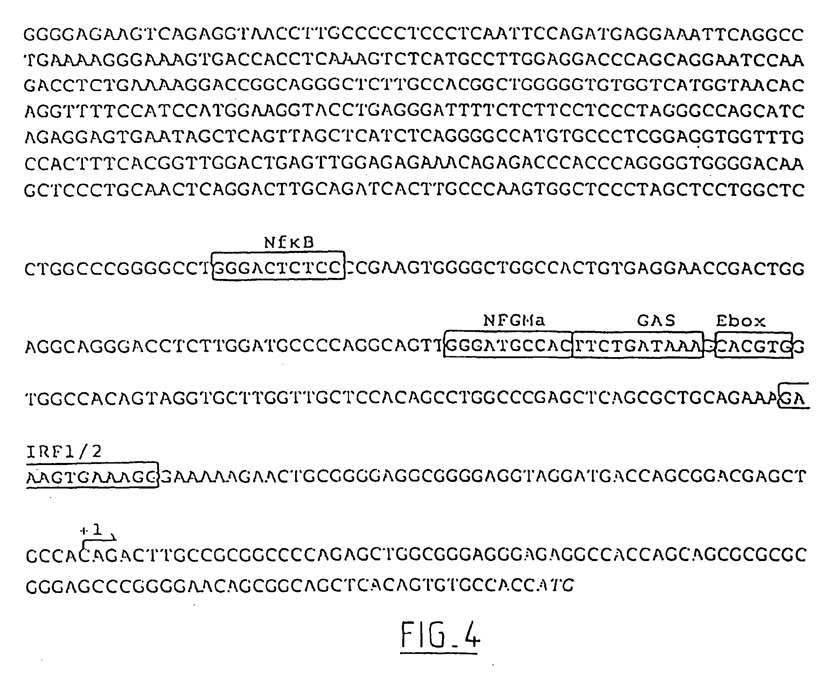

Par contre, plusieurs sites correspondant à des sites de fixation d'éléments agissant en cis au cours de la transcription d'autres gènes ont pu être identifiés. Ainsi, le promoteur PI renferme un site NF-GMb, un site NF-IL6, deux sites NF-IL6 inversés, un site PEA3 et un site PEA3 en sens inverse, un site AP1 et une boîte CCAAT (Figure 2). De même, le promoteur PIII renferme une boite E2A en sens inverse, un site IRF1/2, un site MYC en sens inverse et un site OCT en sens inverse (Figure 3). Dans le promoteur IV sont retrouvés un site NF-GMa, une boîte GAS , une boîte E, un site IRF1/2 et un site NfkB (Figure 4).

### Exemple 4 :

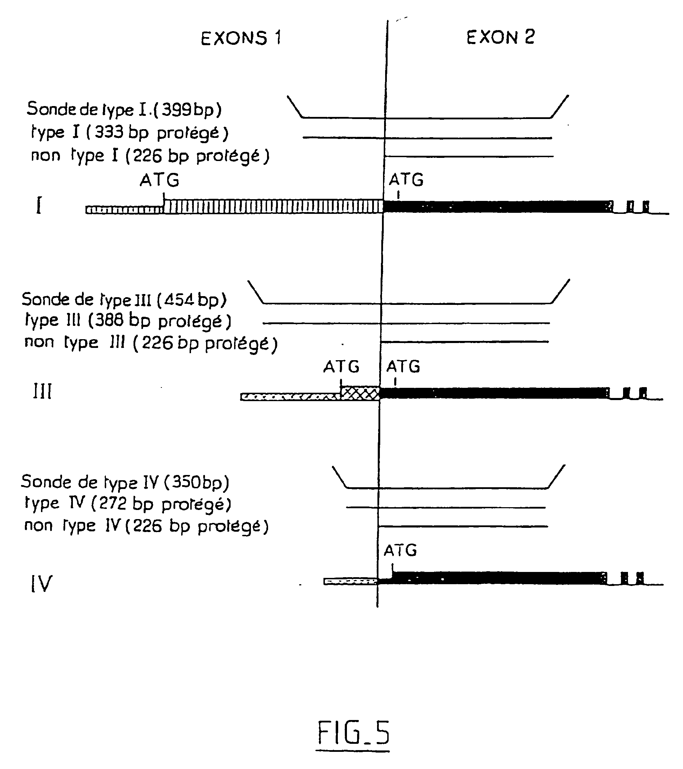

Afin d'étudier le profil d'expression de ces différents gènes dans divers types cellulaires, quatre fragments d'ADNc spécifiques de chacune des formes d'ARNm ont été préparé comme sonde de protection à la RNAse. Ces sondes sont représentées à la Figure 5. Un contrôle interne permettant d'évaluer l'expression totale des gènes codant pour CIITA est utilisé (du nucléotide 1152, site PstI, au nucléotide 1344, site NcoI, protégeant 193 bases de la région commune des ARNs (Exon 2). Les tests de protection à la RNAse sont réalisés sur 25 µg d'ARN comme précédemment décrit (Steimle et al., 1993, Cell, 75, 135-146). Les résultats sont quantifiés par utilisation d'un PhosphorImager. La fonction promotrice est quantifiée comme étant le rapport de l'expression d'un type spécifique d'ARNm par rapport à l'expression totale des gènes codant pour CIITA mesurée à partir du contrôle interne.

Les ARNm issus de différents tissus ou lignées cellulaires exprimant le gène CIITA de manière constitutive ou après induction par l'interféron y ont eté analysés.

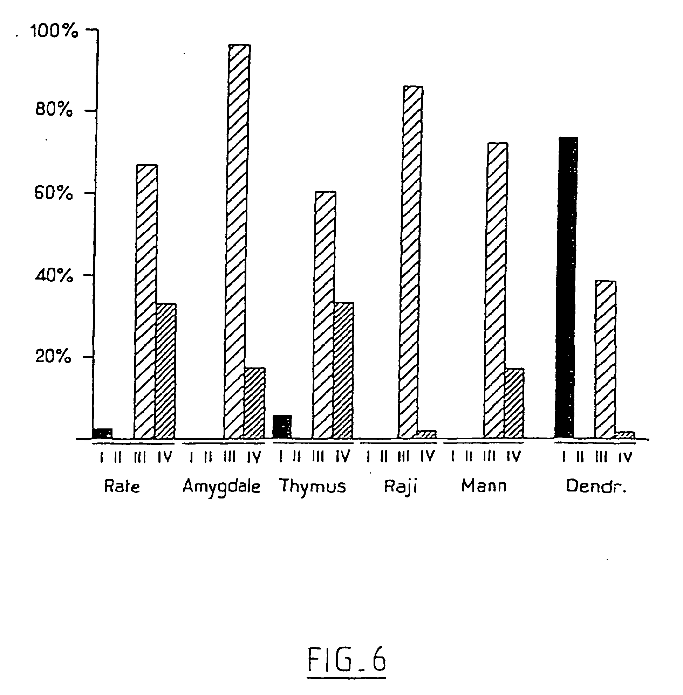

Les résultats (Tableau 1 et Figure 6) montrent qu'il existe un usage différentiel des promoteurs PI, PII, PIII et PIV. Ainsi, il a été montré que les ARNm de type I, résultant de l'utilisation du PI, sont très fortement exprimés dans les cellules dendritiques (Figure 6), plus faiblement dans la rate et le thymus, et pas du tout dans les autres tissus ou lignées cellulaires.

Les ARNm de type III sont détectés à un fort taux dans différentes lignées cellulaires de lymphocytes B, ainsi que dans des tissus riches en lymphocytes B tels que la rate et les amygdales, ou le thymus (Figure 6). Par contre, ces ARNm de type III sont très faiblement exprimés dans les cellules dendritiques ou dans des cellules inductibles par l'interféron γ (Me67.1, THP1, HUVEC et PP2).

Les ARNm de type IV sont la forme principalement exprimée après induction par l'interféron γ. Ce fait a pu être observé dans différentes lignées cellulaires inductibles telles que Me67.1 (mélanome), THP1 (monocyte), HUVEC (cellules endothéliales) et PP2 (fibroblastes). Par contre, ces ARNm ne sont que faiblement exprimés dans les lymphocytes B ou les cellules dendritiques (Figure 5).

### Exemple 5 :

L'activité fonctionnelle et la spécificité tissulaire des promoteurs PIII et PIV ont été analysées par transfection cellulaire avec des constructions associant un gène reporteur et un promoteur. Etant donné que les ARNm de type III sont majoritairement exprimés dans les lymphocytes B et que les ARNm de type IV sont préférentiellement exprimés dans les cellules inductibles par l'interféron γ, les lignées cellulaires tests choisies sont la lignée Raji (lymphocyte B) et Me67.8 (mélanome). Le gène reporteur choisi est le gène codant pour la P globine de lapin. La région promotrice à tester est clonée en amont de ce gène dans le plasmide pGβG(+) (Sperisen et al., 1992, PCR. Methods Appl. 1, 164-170). Les plasmides pIII-974 et pIII-322 renferment les fragments -974(NheI)/+101 (HpaII) et -322 (Pstl)/+101 (HpaII) des régions génomiques localisées en 5' de l'Exon 1 de type III, respectivement. Les plasmides pIV-950 et pIV-461 renferment les fragments -950 (XhoI)/+75 et -461 (KpnI)/+75 des régions génomiques localisées en 5' de l'Exon 1 de type IV, respectivement. Un plasmide dit de référence est également utilisé à titre de contrôle : il s'agit d'un plasmide renfermant un gène codant pour la β globine de lapin présentant une délétion de 40 bases qui est transcrit sous le contrôle d'un promoteur constitutif de poulet (pGAβcβGID, Sperisen et al., 1992). L'expression du gène reporteur est mesurée par RT-PCR quantitative comme décrit dans Sperisen et al., 1992 avec les modifications suivantes. 5.10⁶ de cellules Raji et 2.5 10⁶ de cellules Me67.8 ont été transfectées par électroporation à 250V, 960µF (GenePulser, BioRad) avec 20 µg d'une préparation plasmidique constituée d'un rapport défini de plasmide tel que décrit précédemment et de plasmide de référence et 400 µg d'ARNt de E. Coli comme molécule porteuse dans 750 µl de tampon RPMI. Pour l'étape d'induction par l'interféron γ, les cultures cellulaires sont placées après transformation en présence (500U/ml) ou en absence de l'inducteur. Les cellules sont cultivées à 37°C pendant 48 heures. Les ARN totaux sont extraits avec le réactif au Trizol et soumis à une digestion par la RNAse free-DNAseI (Boehringer). 1 µg d'ARN total sont utilisés pour la transcription inverse en présence d'une (dT)₂₀ et de transcriptase inverse RNAse free Superscript (50U, GIBCO BRL) et de 10U d'inhibiteur de RNAse. Dans un second temps, 1/10 de l'ADNc obtenu est amplifié avec les amorces βGP5'(5'-TCCCCCAAAACAGACAGAATGG-3')(40pmol) et βGP3' (5'-GTCACAGTGCAGTTCACTCAG-3') (40pmol) dans un volume de 50µl contenant 5µl de tampon 10x Vent en présence de 2 µCi (α³² P)dCTP (Amersham). Après une préincubation de 3 minutes à 95°C, 2U de Vent ADN polymérase (NEB) sont ajoutées. L'amplification est réalisée en 30 cycles de 40 secondes à 94°C, 30 secondes à 59°C et 60secondes à 72°C. Les produits de PCR sont dénaturés et déposés sur gel de polyacrylamide dénaturant (6%, 8M urée). Les signaux sont quantifiés par le Phospholmager.

Les résultats obtenus montrent que la transfection de lymphocytes B avec les plasmides pIII-974 et pIII-322 s'accompagne d'une forte activité du promoteur PIII, tandis que les mêmes promoteurs sont inactifs dans les cellules Me67.8 avant ou après induction. On constate en outre dans les lymphocytes B que le PIII-322 est mieux exprimé que le plasmide pIII-974

Par contre, avec les plasmides pIV-950 et pIV-461 on n'observe qu'une expression de base dans les lymphocytes B mais une très forte expression dans les cellules Me67.8 induites, et dans d'autres types cellulaires induits (Hela, 2FTGH). Par ailleurs, les signaux d'expression de ces deux plasmides pIV-950 et pIV-461 ont des valeurs de 0.13 et 0.18, respectivement, avant induction et de 7.9 et 29.6, respectivement, après induction par l'interféron.

Tableau 1 : Pourcentages des différents types de ARNm CIITA observés dans différents tissus et lignées cellulaires.

| | **TYPE I** | **TYPE III** | **TYPE IV** |
|---|---|---|---|
| Rate | 3,5 % | 67 % | 33 % |
| Amygdale | 0% | 96 % | 17 % |
| Thymus | 6% | 60 % | 33% |
| Raji | 0% | 86 % | 2,5% |
| Mann | 0% | 72 % | 17 % |
| Dendr. | 74 % | 39 % | 2,7 % |
| Me67.1 + IFN-γ | 0% | 2% | 88 % |
| THP1 + IFN-γ | 0% | 14 % | 62 % |
| HUVEC + IFN-γ | n.d. | 10 % | 68 % |
| PP2 + IFN-γ | n.d. | 16 % | 66 % |

### Exemple 6:

Comme l'indique la figure 4, la SEQ ID N° 6, correspondant plus particulièrement au promoteur CIITA de type IV inductible par une cytokine, renferme au moins trois régions pouvant être impliquées dans la régulation *en cis* de l'expression du gène localisé en amont de ladite séquence. Il s'agit des boîtes GAS, boîte E et IRF-1.

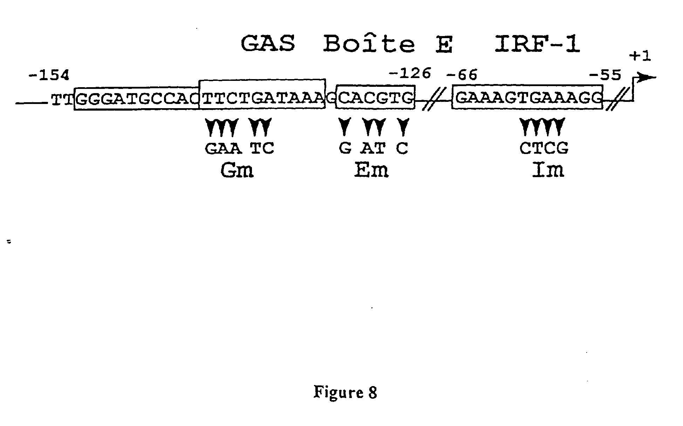

Afin d'étudier l'importance fonctionnelle de ces éléments, des expériences de mutagénèses dirigées ont été conduites. Pour cela, un gène reporteur a été construit qui renferme en amont du gène codant pour la béta globine de lapin du plasmide pGβG(+) un fragment de 308 paires de bases correspondant à la séquence -308/+75 de SEQ ID N° 6. Par mutagénèse dirigée, plusieurs mutations ponctuelles ont été introduites dans le plasmide ainsi construit, au niveau des régions à étudier (voir Figure 8), donnant lieu à trois mutants notés Gm, Em et Im correspondant respectivement à la mutation des boîtes GAS, boîtes E et IRF-1.

L'activité transcriptionnelle du plasmide sauvage (PIV-308 wt) et de chacun des mutants (Gm, Em et Im) est analysée dans la lignée cellulaire Me67.8 (mélanome) par amplification quantitative par PCR réverse des ARN exprimés (Sperisen et al., 1992, PCR Meth. Appli., 1, 164-170) après activation par l'interféron gamma. La transfection des cellules, l'induction, la préparation des ARN et l'analyse par PCR réverse sont réalisées selon les conditions expérimentales précédemment décrites dans Muhlethaler-Mottet et al., 1997, EMBO J., 16, 2851-2860.

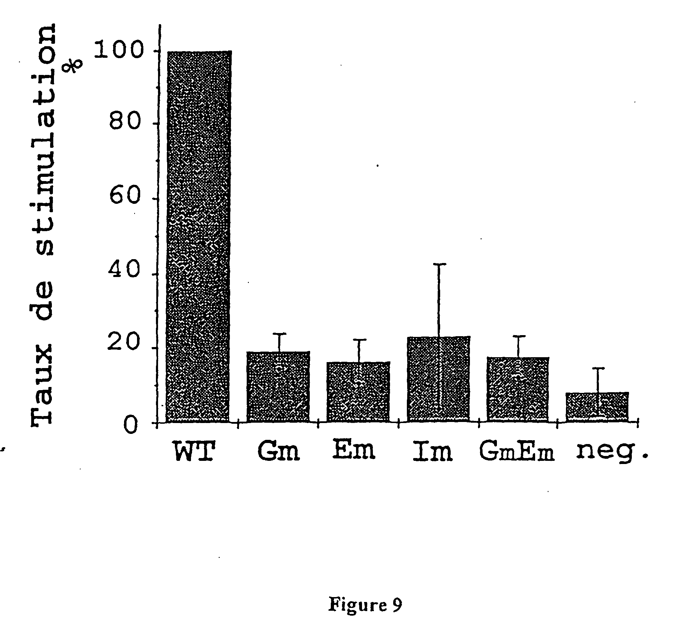

La transfection du plasmide sauvage à l'intérieur des cellules Me67.8, en l'absence d'activation par l'interféron gamma, conduit à un niveau très faible d'expression du gène reporteur codant pour la β globine (non montré). Après traitement de ces mêmes cellules transfectées avec l'interféron gamma, on observe (figure 9) une très forte expression du gène β globine qui traduit une forte activité du promoteur IV (la valeur obtenue figure le taux de stimulation 100 %).

De manière identique, on a analysé l'expression du gène β globine placé sous le contrôle des séquences IV mutées (Gm, Em et Im). Les résultats observés (figure 9) montrent que les mutations apportées individuellement aux différentes régions conduisent à une abolition quasi totale de l'activation par l'interféron gamma (19 % de stimulation pour Gm, 16 % pour Em et 23 % pour Im). Par ailleurs, un double mutant a été construit pour lequel les mutations des boîtes GAS et E ont été combinées. L'analyse de l'expression du gène β globine placé sous le contrôle d'un tel promoteur IV muté montre que l'on observe également dans ce cas une expression du gène reporteur correspondant à 17 % de stimulation obtenue avec le promoteur sauvage.

Par conséquent, ces résultats indiquent clairement que chacune des régions GAS, Boîte É et IRF-1 jouent un rôle fonctionnel dans l'induction par l'interféron gamma de l'expression des gènes placés sous le contrôle du promoteur IV.

### Exemple 7 :

Ayant montré l'importance fonctionnelle du site de fixation IRF-1 pour la fonction du promoteur IV et étant donné le rôle joué par le facteur IRF-1 dans l'induction de plusieurs gènes activables par l'interféron gamma, tel que par exemple le gène GBP (Briken et al., 1995, Mol. Cell. Biol., 15,975-982), nous avons étudié le rôle du facteur IRF-1 dans l'induction par l'interféron gamma du gène CIITA. Pour cela, l'expression des ARN messagers de CIITA a été analysée, après stimulation par l'interféron gamma, dans des fibroblastes embryonnaires dérivés de souris de phénotype sauvage ou de souris IRF-1, c'est à dire qui n'expriment pas le facteur IRF-1. Les expériences de protection à la RNAse ont permis de mettre en évidence que, contrairement à ce qui est observé chez les souris sauvages, l'expression des ARN messagers de CIITA obtenus après stimulation par l'interféron gamma est fortement réduite dans la lignée IRF-1'. De manière identique, il a été montré que la stimulation du gène GBP est également inhibée dans les souris mutantes. Ces résultats montrent que la séquence IRF-1 est un facteur essentiel pour l'efficacité de l'induction par l'interféron gamma.

## Revendications

1. Séquence d'acide nucléique comprenant tout ou partie de la séquence d'acide nucléique SEQ ID N°3 d'un gène CIITA ou de sa séquence complémentaire, telle que ou ladite partie ou sa séquence complémentaire
a) présente une activité de promoteur transcriptionnel induite par une cytokine, ou bien
b) comprend la partie non-traduite de la séquence SEQ ID N°3.

2. Séquence d'acide nucléique mutée par rapport à une séquence selon la revendication 1, contenant moins de 20 mutations ponctuelles au total et présentant une activité de promoteur transcriptionnel induite par une cytokine.

3. Séquence d'acide nucléique selon la revendication 1 **caractérisée en ce que** la séquence comprend la séquence SEQ ID N°6 ou sa séquence complémentaire.

4. Séquence d'acide nucléique selon la revendication 1 **caractérisée en ce que** ladite activité de promoteur est spécifiquement exprimée dans un type cellulaire.

5. Séquence d'acide nucléique selon la revendication 1 **caractérisée en ce que** ladite cytokine est choisie parmi le groupe consistant en l'interféron γ et l'interleukine 4.

6. Séquence d'acide nucléique selon la revendication 5 **caractérisée en ce que** ladite séquence comprend tout ou partie de la séquence SEQ ID N°6, ou de sa séquence complémentaire.

7. Séquence d'acide nucléique selon la revendication 1 **caractérisée en ce qu'**elle comprend, tout ou partie de la séquence SEQ ID N°10 ou de sa séquence complémentaire.

8. Séquence d'acide nucléique **caractérisée en ce qu'**elle comprend au moins une séquence selon la revendication 1a) ou selon l'une quelconque des revendications 2 à 6 dans leur dépendance à la revendication 1a), localisée en amont d'au moins l'une quelconque des séquences selon la revendication 7.

9. Séquence d'acide nucléique **caractérisée en ce qu'**elle comprend au moins une séquence selon la revendication 1a) ou selon l'une quelconque des revendications 2 à 6 dans leur dépendance à la revendication 1a), localisée en amont de tout ou partie d'au moins l'une quelconque des séquences SEQ ID N°7, SEQ ID N°8, SEQ ID N° 9, SEQ ID N° 11, SEQ ID N° 12, SEQ ID N° 13, SEQ ID N° 14 et SEQ 10 N° 15 et leur séquence complémentaire.

10. Séquence d'acide nucléique codant pour un polypeptide CIITA consistant en les amino-acides définis selon la SEQ ID N°17.

11. Séquence d'acide nucléique selon la revendication 1, comportant au moins 10 bases, utilisable comme amorce pour l'amplification enzymatique ou comme sonde de détection.

12. Séquence d'acide nucléique comprenant tout ou partie de la séquence SEQ ID N°3 ou de sa séquence complémentaire, comportant de 5 à 50 nucléotides, capable d'hybridation avec une séquence d'acide nucléique selon l'une quelconque des revendications 1 à 6 dans des conditions assurant au moins 95% d'homologie, et capable de bloquer l'activité promotrice de ladite séquence.

13. Séquence d'acide nucléique comprenant tout ou partie de la séquence SEQ ID N°3 ou de sa séquence complémentaire, comportant de 5 à 50 nucléotides, capable d'hybridation avec une séquence d'acide nucléique selon la revendication 1 dans des conditions assurant au moins 95% d'homologie, et capable d'inhiber la synthèse du polypeptide codé par ladite séquence.

14. Vecteur de clonage ou d'expression **caractérisé en ce qu'**il comporte au moins une séquence selon l'une des revendications 1 à 10.

15. Vecteur d'expression selon la revendication 14 comprenant au moins un gène d'intérêt placé sous le contrôle d'au moins une séquence d'acide nucléique selon l'une quelconque des revendications 1 à 6.

16. Vecteur d'expression selon la revendication 15 **caractérisé en ce que** ledit gène d'intérêt est choisi parmi les gènes codant pour un facteur CIITA, les chaînes α et β des molécules HLA-DR, HLA-DQ et/ou HLA-DP.

17. Vecteur d'expression selon la revendication 15 **caractérisé en ce que** ledit gène d'intérêt est un gène reporteur.

18. Vecteur d'expression selon la revendication 17 **caractérisé en ce que** ledit gène reporteur est le gène codant pour la β globine de lapin.

19. Vecteur d'expression selon la revendication 14 comprenant au moins une séquence selon la revendication 6 placée sous le contrôle d'éléments permettant l'expression desdites séquences.

20. Vecteur d'expression selon la revendication 19 **caractérisé en ce que** lesdits éléments permettant l'expression consistent en au moins une séquence d'acide nucléique selon la revendication 1a) ou selon l'une quelconque des revendications 2 à 6 dans leur dépendance à la revendication 1a).

21. Vecteur selon l'une quelconque des revendications 14 à 20 **caractérisé en ce qu'**il s'agit d'un vecteur à réplication autonome.

22. Vecteur selon l'une quelconque des revendications 14 à 20 **caractérisé en ce qu'**il s'agit d'un vecteur d'intégration chromosomique.

23. Vecteur selon l'une quelconque des revendications 14 à 20 **caractérisé en ce qu'**il s'agit d'un vecteur viral.

24. Vecteur selon l'une des revendications 14 à 20 **caractérisé en ce que** le vecteur est réalisé sur la base d'un adénovirus, d'un rétrovirus, d'un poxvirus ou d'un virus herpétique.

25. Cellule transformée par et comprenant un vecteur selon l'une quelconque des revendications 14 à 24.

26. Cellule selon la revendication 25 **caractérisée en ce qu'**il s'agit d'une cellule procaryote.

27. Cellule selon la revendication 25 **caractérisée en ce qu'**il s'agit d'une cellule eucaryote.

28. Cellule selon la revendication 25 **caractérisée en ce que** ladite cellule est choisie dans le groupe consistant en cellule dendritique, lymphocyte B, lymphocyte T, macrophage, monocyte, cellule de l'épithélium thymique, cellule musculaire, fibroblaste, cellule endothéliale et cellule cancéreuse, notamment cellule de mélanome.

29. Procédé de production d'un polypeptide CIITA tel que défini dans la séquence SEQ ID N°17, comprenant
(i) la culture d'une cellule hôte selon l'une des revendications 25 à 28, dans des conditions de culture appropriées permettant la production dudit polypeptide et
(ii) la récupération dudit polypeptide.

30. Polypeptide CIITA susceptible d'être obtenu par la mise en oeuvre du procédé selon la revendication-29.

31. Polypeptide selon la revendication 30 **caractérisé en ce qu'**il présente la même fonction transactivatrice de l'expression des gènes codant pour des molécules MHC de classe II qu'un facteur CIITA.

32. Anticorps polyclonal ou monoclonal **caractérisé en ce qu'**il est obtenu par réaction immunologique d'un organisme humain ou animal avec un agent immunogène comprenant au moins un polypeptide selon l'une quelconque des revendications 30 à 31.

33. Molécule inhibitrice capable d'inhiber l'activité transactivatrice d'un polypeptide selon la revendication 31 consistant en un anticorps selon la revendication 32.

34. Séquence d'acide nucléique selon la revendication 1a) **caractérisée en ce qu'**elle comprend une mutation ponctuelle affectant l'activité de promoteur transcriptionnel de ladite séquence en modifiant la régulation de l'efficacité ou la spécificité cellulaire dudit promoteur.

35. Séquence d'acide nucléique selon la revendication 7, **caractérisée en ce qu'**elle comprend une mutation ponctuelle affectant la fonction ou l'expression d'un facteur CIITA.

36. Méthode de diagnostic d'une prédisposition à une affection liée à un trouble de l'expression des gènes codant pour les molécules MHC de classe II **caractérisée en ce que** :
- on détermine, sur le prélèvement biologique d'un patient, la présence d'au moins une mutation d'une séquence d'acide nucléique selon l'une des revendications 1 à 7 qui affecte la fonction ou l'expression d'un facteur CIITA, par analyse de ladite séquence d'acide nucléique et comparaison avec une séquence sauvage

37. Méthode de diagnostic selon la revendication 36 **caractérisée en ce que** la ou les mutations que l'on cherche à déterminer sont des mutations affectant l'activité de promoteur transcriptionnel d'une séquence d'acide nucléique selon l'une des revendications 1 à 6.

38. Méthode de diagnostic selon la revendication 37 **caractérisée en ce que** la ou les mutations que l'on cherche à déterminer sont des mutations qui affectent la spécificité cellulaire de ladite activité de promoteur transcriptionnel.

39. Méthode de diagnostic selon la revendication 37 **caractérisée en ce que** la ou les mutations que l'on cherche à déterminer sont des mutations qui affectent l'induction par une cytokine de ladite fonction promotrice.

40. Méthode selon l'une des revendications 36 à 39 **caractérisée en ce que** la séquence d'acide nucléique analysée est un ADN génomique.

41. Méthode selon l'une des revendications 36 à 40 **caractérisée en ce qu'**on détermine la présence d'au moins une mutation par hybridation.

42. Méthode selon la revendication 41 **caractérisée en ce que** ladite hybridation est réalisée à l'aide d'au moins une sonde oligonucléotidique spécifique de la mutation recherchée.

43. Composition pharmaceutique comprenant au moins une substance telle que définie selon l'une quelconque des revendications 1-8, 12-24 ou 30-35.

44. Composition pharmaceutique selon la revendication 43 **caractérisée en ce qu'**elle comprend au moins une substance selon l'une quelconque des revendications 1-6, 14-16, 19, 20, 30 pour le traitement d'affecfions pour lesquelles une augmentation de l'expression des gènes codant pour les molécules MHC de classe ll est désirée.

45. Composition pharmaceutique selon la revendication 44 **caractérisée en ce que** ladite augmentation de l'expression des gènes codant pour les molécules MHC de classe Il est spécifiquement désirée dans un type cellulaire.

46. Composition pharmaceutique selon la revendication 45 **caractérisée en ce que** ladite augmentation de l'expression des gènes codant pour les molécules MHC de classe Il est désirée après induction par une cytokine, et plus particulièrement par l'interféron a ou l'interleukine 4.

47. Composition pharmaceutique selon la revendication 46 **caractérisée en ce que** ladite substance consiste en la séquence d'acide nucléique selon la revendication 5.

48. Composition pharmaceutique selon la revendication 44 **caractérisée en ce qu'**elle comprend au moins une substance selon l'une quelconque des revendications 10, 11, 30-33 pour le traitement d'affections pour lesquelles une réduction de l'expression des gènes codant pour les molécules MHC de classe II est désirée.

## Claims

1. Nucleic acid sequence which comprises all or part of the nucleic acid sequence SEQ ID No. 3 of a CIITA gene or its complementary sequence, wherein either said part or its complementary sequence
a) exhibits a transcriptional promoter activity induced by a cytokine, or
b) comprises the non-translated part of the sequence SEQ ID No. 3.

2. Nucleic acid sequence which is mutated with respect to a sequence according to claim 1, comprising less than 20 point mutations in all and exhibiting a transcriptional promoter activity induced by a cytokine.

3. Nucleic acid sequence according to claim 1, **characterized in that** the sequence comprises the sequence SEQ ID No. 6 or its complementary sequence.

4. Nucleic acid sequence according to claim 1, **characterized in that** said promoter activity is specifically expressed in one cell type.

5. Nucleic acid sequence according to claim 1, **characterized in that** said cytokine is selected from the group consisting of interferon γ and interleukin 4.

6. Nucleic acid sequence according to claim 5, **characterized in that** said sequence comprises all or part of the sequence SEQ ID No. 6, or of its complementary sequence.

7. Nucleic acid sequence according to claim 1, **characterized in that** said sequence comprises all or part of the sequence SEQ ID No. 10, or of its complementary sequence.

8. Nucleic acid sequence, **characterized in that** it comprises at least one sequence according to claim 1a) or according to any one of claims 2 to 6 in their dependency on claim 1a), which sequence is located upstream of at least any one of the sequences according to claim 7.

9. Nucleic acid sequence, **characterized in that** it comprises at least one sequence according to claim 1a) or according to any one of claims 2 to 6 in their dependency on claim 1a), which sequence is located upstream of all or part of at least any one of the sequences SEQ ID No. 7, SEQ ID No. 8, SEQ ID No. 9, SEQ ID No. 11, SEQ ID No. 12, SEQ ID No. 13, SEQ ID No. 14 and SEQ ID No. 15, and their complementary sequences.

10. Nucleic acid sequence encoding a CIITA polypeptide which consists of the amino acids defined in accordance with SEQ ID No. 17.

11. Nucleic acid sequence according to claim 1, which includes at least 10 bases and which can be used as a primer for enzymatic amplification or as a detection probe.

12. Nucleic acid sequence comprising all or part of the sequence SEQ ID NO.3 or its complementary sequence, including from 5 to 50 nucleotides, which is able to hybridize with a nucleic acid sequence according to any one of claims 1 to 6 under conditions ensuring at least 95% homology, and which is able to block the promoter activity of said sequence.

13. Nucleic acid sequence comprising all or part of the sequence SEQ ID No.3 or its complementary sequence, including from 5 to 50 nucleotides, which is able to hybridize with a nucleic acid sequence according to claim 1 under conditions ensuring at least 95% homology and which is able to inhibit the synthesis of the polypeptide which is encoded by said sequence.

14. Cloning or expression vector, **characterized in that** it includes at least one sequence according to any one of claims 1 to 10.

15. Expression vector according to claim 14, which comprises at least one gene of interest which is placed under the control of at least one nucleic acid sequence according to any one of claims 1 to 6.

16. Expression vector according to claim 15, **characterized in that** said gene of interest is selected from the genes which encode a CIITA factor and the α and β chains of the HLA-DR, HLA-DQ and/or HLA-DP molecules.

17. Expression vector according to claim 15, **characterized in that** said gene of interest is a reporter gene.

18. Expression vector according to claim 17, **characterized in that** said reporter gene is the gene which encodes rabbit β globin.

19. Expression vector according to claim 14 which comprises at least one sequence according to claim 6, which sequence is placed under the control of elements which enable said sequences to be expressed.

20. Expression vector according to claim 19, **characterized in that** said elements which enable expression to take place consist of at least one nucleic acid sequence according to claim 1a) or according to any one of claims 2 to 6 in their dependency to claim 1a).

21. Vector according to any one of claims 14 to 20, **characterized in that** it is an autonomously replicating vector.

22. Vector according to any one of claims 14 to 20, **characterized in that** it is a vector which integrates into the chromosome.

23. Vector according to any one of claims 14 to 20, **characterized in that** it is a viral vector.

24. Vector according to any one of claims 14 to 20, **characterized in that** the vector is constructed on the basis of an adenovirus, a retrovirus, a poxvirus or a herpesvirus.

25. Cell which is transformed with a vector according to any one of claims 14 to 24 and which comprises said vector.

26. Cell according to claim 25, **characterized in that** it is a prokaryotic cell.

27. Cell according to claim 25, **characterized in that** it is a eukaryotic cell.

28. Cell according to claim 25, **characterized in that** said cell is selected from the group consisting of dendritic cells, B lymphocytes, T lymphocytes, macrophages, monocytes, thymic epithelium cells, muscle cells, fibroblasts, endothelial cells and cancer cells, in particular melanoma cells.

29. Process for producing a CIITA polypeptide, as defined in the sequence SEQ ID No. 17, which comprises
(i) culturing a host cell according to any one of claims 25 to 28 under culture conditions which are appropriate for producing said polypeptide, and
(ii) recovering said polypeptide.

30. CIITA polypeptide which can be obtained by implementing the process according to claim 29.

31. Polypeptide according to claim 30, **characterized in that** it exhibits the same function of transactivating the expression of the genes encoding MHC class II molecules as a CIITA factor.

32. Polyclonal or monoclonal antibody, **characterized in that** it is obtained by the immunological reaction of a human or animal organism with an immunogenic agent which comprises at least one polypeptide according to any one of claims 30 to 31.

33. Inhibitory molecule which is able to inhibit the transactivating activity of a polypeptide according to claim 31, which consists of an antibody according to claim 32.

34. Nucleic acid sequence according to claim 1a), **characterized in that** it contains one point mutation which affects the transcriptional promoter activity of said sequence by changing the regulation of the efficacy or of the cellular specificity of said promoter.

35. Nucleic acid sequence according to claim 7, **characterized in that** it contains one point mutation which affects the function or the expression of a CIITA factor.

36. Method for diagnosing a predisposition to a disorder which is linked to a disturbance in the expression of the genes encoding MHC class II molecules, **characterized in that**:
- in a biological sample from a patient, the presence in a nucleic acid sequence according to any one of claims 1 to 7 of at least one mutation which affects the function or the expression of a CIITA factor is determined by analysing said nucleic acid sequence and comparing with a wild-type sequence.

37. Diagnostic method according to claim 36, **characterized in that** the mutation(s) which it is sought to determine is/are (a) mutation(s) which affect(s) the transcriptional promoter activity of a nucleic acid sequence according to any one of claims 1 to 6.

38. Diagnostic method according to claim 37, **characterized in that** the mutation(s) which it is sought to determine is/are (a) mutation(s) which affect(s) the cell specificity of said transcriptional promoter activity.

39. Diagnostic method according to claim 37, **characterized in that** the mutation(s) which it is sought to determine is/are (a) mutation(s) which affect(s) the induction of said promoter function by a cytokine.

40. Method according to any one of claims 36 to 39, **characterized in that** the analysed nucleic acid sequence is a genomic DNA.

41. Method according to any one of claims 36 to 40, **characterized in that** the presence of at least one mutation is determined by means of hybridization.

42. Method according to claim 41, **characterized in that** said hybridization is carried out using at least one oligonucleotide probe which is specific for the sought-after mutation.

43. Pharmaceutical composition which comprises at least one substance as defined according to any one of claims 1-8, 12-24 or 30-35.

44. Pharmaceutical composition according to claim 43, **characterized in that** it comprises at least one substance according to any one of claims 1-6, 14-16, 19, 20, 30 for treating disorders in which it is desired to increase the expression of the genes encoding MHC class II molecules.

45. Pharmaceutical composition according to claim 44, **characterized in that** said increase in the expression of the genes which encode MHC class II molecules is specifically desired in one cell type.

46. Pharmaceutical composition according to claim 45, **characterized in that** said increase in the expression of the genes which encode MHC class II molecules is desired following induction by a cytokine, more specifically by interferon γ or interleukin 4.

47. Pharmaceutical composition according to claim 46, **characterized in that** said substance consists of the nucleic acid sequence according to claim 5.

48. Pharmaceutical composition according to claim 44, **characterized in that** it comprises at least one substance according to any one of claims 10, 11, 30-33 for treating disorders in which it is desired to reduce the expression of the genes encoding MHC class II molecules.

## Patentansprüche

1. Nukleinsäuresequenz umfassend die ganze oder einen Teil der Nukleinsäuresequenz SEQ ID Nr. 3 eines CIITA-Gens oder ihrer komplementären Sequenz, wobei dieser Teil oder ihre komplementäre Sequenz
a) eine Aktivität als Transkriptionspromotor aufweist, die durch ein Cytokin induziert wird, oder
b) den nicht translierten Teil der Sequenz SEQ ID Nr. 3 umfasst.

2. Nukleinsäuresequenz, die bezüglich einer Sequenz nach Anspruch 1 mutiert ist, die insgesamt weniger als 20 punktuelle Mutationen enthält und eine Aktivität als Transkriptionspromotor aufweist, die durch ein Cytokin induziert wird.

3. Nukleinsäuresequenz nach Anspruch 1, **dadurch gekennzeichnet, dass** die Sequenz die Sequenz SEQ ID Nr. 6 oder ihre komplementäre Sequenz umfasst.

4. Nukleinsäuresequenz nach Anspruch 1, **dadurch gekennzeichnet, dass** die Aktivität als Promotor in einem Zelltyp spezifisch exprimiert wird.

5. Nukleinsäuresequenz nach Anspruch 1, **dadurch gekennzeichnet, dass** das Cytokin ausgewählt ist aus der Gruppe bestehend aus γ-Interferon und Interleukin-4.

6. Nukleinsäuresequenz nach Anspruch 5, **dadurch gekennzeichnet, dass** die Sequenz die ganze oder einen Teil der Sequenz SEQ ID Nr. 6 oder ihrer komplementären Sequenz umfasst.

7. Nukleinsäuresequenz nach Anspruch 1, **dadurch gekennzeichnet, dass** sie die ganze oder einen Teil der Sequenz SEQ ID Nr. 10 oder ihrer komplementären Sequenz umfasst.

8. Nukleinsäuresequenz, **dadurch gekennzeichnet, dass** sie mindestens eine Sequenz nach Anspruch 1a) oder nach einem der Ansprüche 2 bis 6 in Abhängigkeit von Anspruch 1a) umfasst, die vor mindestens einer der Sequenzen nach Anspruch 7 liegt.

9. Nukleinsäuresequenz, **dadurch gekennzeichnet, dass** sie mindestens eine Sequenz nach Anspruch 1a) oder nach einem der Ansprüche 2 bis 6 in Abhängigkeit von Anspruch 1a) umfasst, die vor der ganzen oder einem Teil mindestens einer der Sequenzen SEQ ID Nr. 7, SEQ ID Nr. 8, SEQ ID Nr. 9, SEQ ID Nr. 11, SEQ ID Nr. 12, SEQ ID Nr. 13, SEQ ID Nr. 14 und SEQ ID Nr. 15 und ihrer komplementären Sequenz liegt.

10. Nukleinsäuresequenz, die für ein CIITA-Polypeptid kodiert, das aus den in SEQ ID Nr. 17 definierten Aminosäuren gebildet ist.

11. Nukleinsäuresequenz nach Anspruch 1, umfassend mindestens 10 Basen, die als Initiator zur enzymatischen Amplifizierung oder als Detektionssonde geeignet ist.

12. Nukleinsäuresequenz umfassend die ganze oder einen Teil der Sequenz SEQ ID Nr. 3 oder ihrer komplementären Sequenz mit 5 bis 50 Nukleotiden, geeignet zur Hybridisierung mit einer Nukleinsäuresequenz nach einem der Ansprüche 1 bis 6 unter Bedingungen, die mindestens 95 % Homologie gewährleisten, und geeignet zum Blockieren der Promotoraktivität der Sequenz.

13. Nukleinsäuresequenz umfassend die ganze oder einen Teil der Sequenz SEQ ID Nr. 3 oder ihrer komplementären Sequenz mit 5 bis 50 Nukleotiden, geeignet zur Hybridisierung mit einer Nukleinsäuresequenz nach Anspruch 1 unter Bedingungen, die mindestens 95 % Homologie gewährleisten, und geeignet zum Inhibieren der Synthese des von der Sequenz kodierten Polypeptids.

14. Klonierungsvektor oder Expressionsvektor, **dadurch gekennzeichnet, dass** er mindestens eine Sequenz nach einem der Ansprüche 1 bis 10 umfasst.

15. Expressionsvektor nach Anspruch 14, umfassend mindestens ein interessierendes Gen, das unter der Kontrolle mindestens einer Nukleinsäuresequenz nach einem der Ansprüche 1 bis 6 steht.

16. Expressionsvektor nach Anspruch 15, **dadurch gekennzeichnet, dass** das interessierende Gen ausgewählt ist aus den Genen, die für einen CIITA-Faktor, die α- oder β-Ketten von Molekülen HLA-DR, HLA-DQ und/oder HLA-DP kodieren.

17. Expressionsvektor nach Anspruch 15, **dadurch gekennzeichnet, dass** das interessierende Gen ein Reportergen ist.

18. Expressionsvektor nach Anspruch 17, **dadurch gekennzeichnet, dass** das Reportergen das Gen ist, das für das β-Globin des Kaninchens kodiert.

19. Expressionsvektor nach Anspruch 14, umfassend mindestens eine Sequenz nach Anspruch 6, die unter der Kontrolle von Elementen steht, die die Expression dieser Sequenzen ermöglichen.

20. Expressionsvektor nach Anspruch 19, **dadurch gekennzeichnet, dass** die Elemente, die die Expression ermöglichen, aus mindestens einer Nukleinsäuresequenz nach Anspruch 1a) oder nach einem der Ansprüche 2 bis 6 in Abhängigkeit von Anspruch 1a) gebildet sind.

21. Vektor nach einem der Ansprüche 14 bis 20, **dadurch gekennzeichnet, dass** es sich um einen Vektor der autonomen Replikation handelt.

22. Vektor nach einem der Ansprüche 14 bis 20, **dadurch gekennzeichnet, dass** es sich um einen Vektor der Chromosomenintegration handelt.

23. Vektor nach einem der Ansprüche 14 bis 20, **dadurch gekennzeichnet, dass** es sich um einen viralen Vektor handelt.

24. Vektor nach einem der Ansprüche 14 bis 20, **dadurch gekennzeichnet, dass** der Vektor auf Basis eines Adenovirus, eines Retrovirus, eines Poxvirus oder eines Herpesvirus realisiert ist.

25. Zelle, die von einem Vektor nach einem der Ansprüche 14 bis 24 transformiert ist und diesen enthält.

26. Zelle nach Anspruch 25, **dadurch gekennzeichnet, dass** es sich um eine prokaryotische Zelle handelt.

27. Zelle nach Anspruch 25, **dadurch gekennzeichnet, dass** es sich um eine eukaryotische Zelle handelt.

28. Zelle nach Anspruch 25, **dadurch gekennzeichnet, dass** die Zelle ausgewählt ist aus der Gruppe bestehend aus dendritischen Zellen, B-Lymphozyten, T-Lymphozyten, Makrophagen, Monozyten, Thymusepithelzellen, Muskelzellen, Fibroblasten, Endothelzellen und Krebszellen, insbesondere Melanomzellen.

29. Verfahren zur Herstellung eines CIITA-Polypeptids wie es in der Sequenz SEQ ID Nr. 17 definiert ist, umfassend:
(i) Kultur einer Wirtszelle nach einem der Ansprüche 25 bis 28, unter geeigneten Kulturbedingungen, die die Bildung des Polypeptids ermöglichen, und
(ii) Gewinnung des Polypeptids.

30. CIITA-Polypeptid, das durch Ausführung des Verfahrens nach Anspruch 29 erhalten werden kann.

31. Polypeptid nach Anspruch 30, **dadurch gekennzeichnet, dass** es die selbe Transaktivatorfunktion der Expression von Genen, die für MHC-Moleküle der Klasse II kodieren, wie ein CIITA-Faktor aufweist.

32. Polyklonaler oder monoklonaler Antikörper, **dadurch gekennzeichnet, dass** er durch immunologische Reaktion eines menschlichen oder tierischen Organismus mit einer immunogenen Substanz erhalten ist, der mindestens ein Polypeptid nach einem der Ansprüche 30 bis 31 aufweist.

33. Inhibitormolekül, das in der Lage ist, die Transaktivatoraktivität eines Polypeptids nach Anspruch 31 zu inhibieren, das aus einem Antikörper nach Anspruch 32 gebildet ist.

34. Nukleinsäuresequenz nach Anspruch 1a), **dadurch gekennzeichnet, dass** sie eine punktuelle Mutation aufweist, die die Aktivität als Transkriptionspromotor der Sequenz beeinflusst, indem die Regulation der Wirksamkeit oder der Zellspezifität des Promotors modifiziert wird.

35. Nukleinsäuresequenz nach Anspruch 7, **dadurch gekennzeichnet, dass** sie eine punktuelle Mutation aufweist, die die Funktion oder Expression eines CIITA-Faktors beeinflusst.

36. Verfahren zur Diagnose einer Prädisposition für ein Leiden, das mit einer Störung der Expression von Genen verbunden ist, die für die MHC-Moleküle der Klasse II kodieren, **dadurch gekennzeichnet, dass**:
bei einer biologischen Probe von einem Patienten das Vorhandensein mindestens einer Mutation einer Nukleinsäuresequenz nach einem der Ansprüche 1 bis 7, die die Funktion oder Expression eines CIITA-Faktors beeinflusst, durch Analyse der Nukleinsäuresequenz und Vergleich mit einer Sequenz der Wildform bestimmt wird.

37. Verfahren zur Diagnose nach Anspruch 36, **dadurch gekennzeichnet, dass** die eine oder mehreren Mutationen, die man zu bestimmen sucht, Mutationen sind, die die Aktivität als Transkriptionspromotor einer Nukleinsäuresequenz nach einem der Ansprüche 1 bis 6 beeinflussen.

38. Verfahren zur Diagnose nach Anspruch 37, **dadurch gekennzeichnet, dass** die eine oder mehreren Mutationen, die man zu bestimmen sucht, Mutationen sind, die die Zellspezifität der Aktivität als Transkriptionspromotor beeinflussen.

39. Verfahren zur Diagnose nach Anspruch 37, **dadurch gekennzeichnet, dass** die eine oder mehreren Mutationen, die man zu bestimmen sucht, Mutationen sind, die die Induktion der Promotorfunktion durch ein Cytokin beeinflussen.

40. Verfahren nach einem der Ansprüche 36 bis 39, **dadurch gekennzeichnet, dass** die analysierte Nukleinsäuresequenz eine genomische DNA ist.

41. Verfahren nach einem der Ansprüche 36 bis 40, **dadurch gekennzeichnet, dass** das Vorhandensein mindestens einer Mutation durch Hybridisierung bestimmt wird.

42. Verfahren nach Anspruch 41, **dadurch gekennzeichnet, dass** die Hybridisierung mit Hilfe mindestens einer Oligonukleotidsonde vorgenommen wird, die für die gesuchte Mutation spezifisch ist.

43. Pharmazeutische Zusammensetzung umfassend mindestens eine Substanz wie sie in einem der Ansprüche 1-8, 12-24 oder 30-35 definiert ist.

44. Pharmazeutische Zusammensetzung nach Anspruch 43, **dadurch gekennzeichnet, dass** sie mindestens eine Substanz nach einem der Ansprüche 1-6, 14-16, 19, 20, 30 für die Behandlung von Leiden umfasst, für die eine Steigerung der Expression von Genen, die für die MHC-Moleküle der Klasse II kodieren, gewünscht ist.

45. Pharmazeutische Zusammensetzung nach Anspruch 44, **dadurch gekennzeichnet, dass** die Steigerung der Expression von Genen, die für die MHC-Moleküle der Klasse II kodieren, spezifisch in einem Zelltyp gewünscht ist.

46. Pharmazeutische Zusammensetzung nach Anspruch 45, **dadurch gekennzeichnet, dass** die Steigerung der Expression von Genen, die für die MHC-Moleküle der Klasse 11 kodieren, nach Induktion durch ein Cytokin, und insbesondere durch α-Interferon oder Interleukin-4 gewünscht ist.

47. Pharmazeutische Zusammensetzung nach Anspruch 46, **dadurch gekennzeichnet, dass** die Substanz aus der Nukleinsäuresequenz nach Anspruch 5 gebildet ist.

48. Pharmazeutische Zusammensetzung nach Anspruch 44, **dadurch gekennzeichnet, dass** sie mindestens eine Substanz nach einem der Ansprüche 10, 11, 30-33 für die Behandlung von Leiden umfasst, für die eine Verringerung der Expression von Genen, die für die MHC-Moleküle der Klasse II kodieren, gewünscht ist.
